# EUROPEAN PATENT APPLICATION

(11) **EP 4 606 823 A1**
(43) Date of publication of application: **27.08.2025**
(21) Application number: 23910633.9
(22) Date of filing: 26.12.2023
(51) Int. Cl.: C07K 19/00, C07K 16/28, C12N 15/62, C12N 15/13, C12N 15/63, C12N 5/10, A61K 38/17, A61P 35/00

(54) **SYNTHETIC T CELL RECEPTOR ANTIGEN RECEPTOR SPECIFICALLY BINDING TO LILRB4 AND USE THEREOF**

(30) Priority: 26.12.2022 CN 202211678944
(71) Applicant: Bristar Immunotech Limited, Beijing 102206 (CN)
(72) Inventor: RUI, Wei, Beijing 102206 (CN); PAN, Guohua, Beijing 102206 (CN); WU, Chunyan, Beijing 102206 (CN); LEI, Lei, Beijing 102206 (CN)
(74) Representative: HGF
(86) International application number: PCT/CN2023/142032
(87) International publication number: WO 2024/140710

(57) **Abstract**

Disclosed in the present invention are a synthetic T cell receptor antigen receptor specifically binding to LILRB4 and the use thereof. In the present invention, TCR and CAR are modified by means of targeting the antigen binding fragment of LILRB4, so that a better effect on tumor treatment is obtained.

## Description

### TECHNICAL FIELD

The present disclosure relates to the field of biomedicine, in particular to a synthetic T cell receptor and antigen receptor (STAR) specifically binding to LILRB4, a STAR complex, an immune cell comprising the STAR or the STAR complex, and use thereof in the field of biomedicine.

### BACKGROUND

LILRB4 is a member of the leukocyte immunoglobulin receptor family, and has a single-pass transmembrane structure, two extracellular C-type Ig-like domains, and three intracellular immunoreceptor tyrosine-based inhibitory motifs (ITIMs). In normal humans, LILRB4 is primarily expressed in myeloid immune cells, such as macrophages, DCs, and monocytes, etc., and plays an immunosuppressive role. LILRB4 is highly expressed in acute myelomonocytic leukemia and acute monocytic leukemia, with expression levels higher than those in normal monocytes, and the expression of LILRB4 is also detected in leukemia tumor stem cells. In addition, LILRB4 exhibits relatively high expression in various immunosuppressive cells, such as TAMs, M-MDSCs, tolerogenic DCs, and Tregs, in other tumor microenvironments. Studies have shown that LILRB4 expressed in AML promotes the migration and infiltration of tumor cells and inhibits the proliferation of T cells through Arginase-1 and uPAR. Moreover, the expression of LILRB4 in other myeloid immune cells, such as macrophages, DCs, and monocytes, can significantly promote the differentiation of immune cells towards an immune tolerance direction, thereby exerting the immunosuppressive effect. Based on the specific expression and immunosuppressive function of LILRB4 in leukemia cells and myeloid immune cells, LILRB4 has the potential to become an ideal therapeutic target for acute myelomonocytic leukemia and acute monocytic leukemia through its ability to eliminate tumor cells and improve the immunosuppressive environment.

Chimeric antigen receptor T cell (CAR-T) therapy is an anti-cancer immunotherapy that has achieved good efficacy in recent years. Unlike the way in which natural T cells recognize tumor cells, CAR-T cells recognize tumor cells independently of MHC molecules. The CAR molecule consists of three main components: an extracellular domain, which is an antigen recognition domain derived from an antibody and responsible for recognizing a target antigen; a transmembrane region; and an intracellular region, which is a signaling molecule and a co-stimulatory signaling molecule derived from a T cell receptor and responsible for the transduction of T cell activation signals upon stimulation. The working principle is as follows: when CAR molecules bind to their corresponding antigens, the CAR molecules are aggregated, thereby increasing the local phosphorylation level, activating downstream signals, and finally, starting the effector function of T cells and killing target tumor cells.

The T cell receptor (TCR) complex molecule contains multiple chains, in which the TCRα chain and the TCRβ chain are responsible for recognizing MHC-polypeptide molecules, and the other 6 CD3 subunits bind to the TCRα/β chains to exert the signaling function. The natural TCR complex contains a total of 10 ITAM signal sequences, theoretically enabling it to transduce stronger signals than the CAR. Previous studies have shown that although TCR signaling is slower than CAR signaling, the TCR signaling is more persistent. Therefore, a novel receptor can be constructed to relieve the incapacity of T cells by utilizing the signaling function of the natural TCR, so that the T cells can better play a role in combating solid tumors.

The extracellular domain of the TCR is very similar to the Fab domain of an antibody, and thus, the TCR variable region sequences can be replaced by antibody variable region sequences to obtain a synthetic T cell receptor and antigen receptor (STAR), which has both the specificity of the antibody and the superior signaling function of the natural TCR, and can mediate complete T cell activation.

However, a STAR derived from the natural TCR still has the defects of poor membrane stability, relatively low α/β chain pairing ability, mismatches with endogenous TCRs, difficulty in introducing it into T cells, etc. Therefore, there is still a need in the art for an improved STAR.

### SUMMARY

In order to solve the defects in the prior art, the present disclosure provides a synthetic T cell receptor and antigen receptor (STAR) and use thereof. The synthetic T cell receptor and antigen receptor can specifically bind to LILRB4, and a TCR and a CAR are modified by targeting an antigen-binding fragment of LILRB4. Based on the existing results, the STAR-T cell in the present disclosure has higher potential in the treatment of recurrent or refractory AML FAB M4/M5, and the treatment effectiveness and safety are better than those of the prior art such as CAR-T. Based on the high expression of LILRB4 in AML FAB M4/M5 and myeloid immunosuppressive cells, the development of LILRB4 dual-epitope STAR-T can provide a new immune cell therapeutic strategy for patients with R/R AML FAB M4/M5 by killing tumor cells and improving the tumor microenvironment.

Specifically, in a first aspect of the present disclosure, provided is a synthetic T cell receptor and antigen receptor (STAR),
i) the synthetic T cell receptor and antigen receptor comprises an α chain and a β chain, wherein the α chain comprises a first target-binding region and a first constant region, and the β chain comprises a second target-binding region and a second constant region; or the α chain comprises a first target-binding region, and the β chain comprises a second target-binding region and a second constant region; or
ii) the synthetic T cell receptor and antigen receptor comprises a γ chain and a δ chain, wherein the γ chain comprises a first target-binding region and a first constant region, and the δ chain comprises a second target-binding region and a second constant region; or the γ chain comprises a first target-binding region and a first constant region, and the δ chain comprises a second target-binding region and a second constant region.

Preferably, i) the α chain and/or the β chain is linked at its C-terminus to at least one functional domain; or ii) the γ chain and/or the δ chain is linked at its C-terminus to at least one functional domain.

Further preferably, i) the at least one functional domain is linked, directly or via a linker, to the C-terminus of the α chain and/or the β chain, or ii) the at least one functional domain is linked, directly or via a linker, to the C-terminus of the γ chain and/or the δ chain.

Preferably, i) an intracellular region of the α chain and/or the β chain in the synthetic T cell receptor and antigen receptor is deleted; or ii) an intracellular region of the γ chain and/or the δ chain in the synthetic T cell receptor and antigen receptor is deleted.

Still further preferably, i) the functional domain is linked, directly or via a linker, to the C-terminus of the α chain and/or the β chain with the intracellular region deleted; or ii) the functional domain is linked, directly or via a linker, to the C-terminus of the γ chain and/or the δ chain with the intracellular region deleted.

Preferably, i) the C-terminus of the α chain in the synthetic T cell receptor and antigen receptor is linked to 1, 2, 3, 4, 5, 6, 7, 8, 9, 10, or more functional domains, and/or the C-terminus of the β chain in the synthetic T cell receptor and antigen receptor is linked to 1, 2, 3, 4, 5, 6, 7, 8, 9, 10, or more functional domains; or
ii) the C-terminus of the γ chain in the synthetic T cell receptor and antigen receptor is linked to 1, 2, 3, 4, 5, 6, 7, 8, 9, 10, or more functional domains, and/or the C-terminus of the δ chain in the STAR is linked to 1, 2, 3, 4, 5, 6, 7, 8, 9, 10, or more functional domains.

In one specific embodiment of the present disclosure, the plurality of (including two or more) functional domains are linked directly or via a linker.

In one specific embodiment of the present disclosure, the C-terminus of the α chain in the synthetic T cell receptor and antigen receptor is linked to at least one functional domain. The intracellular region of the α chain is deleted. The functional domain is linked to the C-terminus of the α chain with the intracellular region deleted via a linker.

In one specific embodiment of the present disclosure, the C-terminus of the β chain in the synthetic T cell receptor and antigen receptor is linked to at least one functional domain. The intracellular region of the β chain is deleted. The functional domain is linked to the C-terminus of the (3 chain with the intracellular region deleted via a linker.

In one specific embodiment of the present disclosure, the C-terminus of the γ chain in the synthetic T cell receptor and antigen receptor is linked to at least one functional domain. The intracellular region of the γ chain is deleted. The functional domain is linked to the C-terminus of the γ chain with the intracellular region deleted via a linker.

In one specific embodiment of the present disclosure, the C-terminus of the δ chain in the synthetic T cell receptor and antigen receptor is linked to at least one functional domain. The intracellular region of the δ chain is deleted. The functional domain is linked to the C-terminus of the δ chain with the intracellular region deleted via a linker.

Preferably, i) the functional domains linked to the C-terminus of the α chain and/or the β chain in the synthetic T cell receptor and antigen receptor are the same or different;
or ii) the functional domains linked to the C-terminus of the γ chain and/or the δ chain in the synthetic T cell receptor and antigen receptor are the same or different.

In one specific embodiment of the present disclosure, the plurality of functional domains linked to the α chain in the synthetic T cell receptor and antigen receptor may be the same or different. In one specific embodiment of the present disclosure, the plurality of functional domains linked to the β chain in the synthetic T cell receptor and antigen receptor may be the same or different. In one specific embodiment of the present disclosure, the plurality of functional domains linked to the γ chain in the synthetic T cell receptor and antigen receptor may be the same or different. In one specific embodiment of the present disclosure, the plurality of functional domains linked to the δ chain in the synthetic T cell receptor and antigen receptor may be the same or different. In one specific embodiment of the present disclosure, in the synthetic T cell receptor and antigen receptor, the functional domain linked to the α chain and the functional domain linked to the β chain may be the same or different.

In one specific embodiment of the present disclosure, in the synthetic T cell receptor and antigen receptor, the functional domain linked to the γ chain and the functional domain linked to the δ chain may be the same or different.

Preferably, the functional domain is a co-stimulatory molecule or a fragment thereof, a co-inhibitory molecule or a fragment thereof, a cytokine receptor or a fragment thereof, or an intracellular protein or a fragment thereof. Further preferably, the functional domain is an intracellular domain of a co-stimulatory molecule, an intracellular domain of a co-inhibitory molecule, an intracellular domain of a cytokine receptor, or an intracellular protein. The functional domain may also be a fusion of the intracellular domain of the cytokine receptor, directly or via a linker, to the human STAT5 activation module (with the amino acid sequence set forth in SEQ ID NO: 25).

The co-stimulatory molecule is selected from CD40, OX40, ICOS, CD28, 4-1BB (CD137), or CD27.

The co-inhibitory molecule is selected from TIM3, PD1, CTLA4, or LAG3.

The cytokine receptor is selected from an interleukin receptor (e.g., an IL-2 receptor), an interferon receptor, a tumor necrosis factor superfamily receptor, a colony-stimulating factor receptor, a chemokine receptor, a growth factor receptor, and other membrane proteins.

The intracellular protein is a domain of a T-cell regulatory factor, e.g., a domain of NIK.

In one specific embodiment of the present disclosure, the co-stimulatory molecule is CD40, the intracellular domain of which comprises the amino acid sequence set forth in SEQ ID NO: 10. In one specific embodiment of the present disclosure, the co-stimulatory molecule is OX40, the intracellular domain of which comprises the amino acid sequence set forth in SEQ ID NO: 11. In one specific embodiment of the present disclosure, the co-stimulatory molecule is ICOS, the intracellular domain of which comprises the amino acid sequence set forth in SEQ ID NO: 12. In one specific embodiment of the present disclosure, the co-stimulatory molecule is CD28, the intracellular domain of which comprises the amino acid sequence set forth in SEQ ID NO: 13. In one specific embodiment of the present disclosure, the co-stimulatory molecule is 4-1BB, the intracellular domain of which comprises the amino acid sequence set forth in SEQ ID NO: 14. In one specific embodiment of the present disclosure, the co-stimulatory molecule is CD27, the intracellular domain of which comprises the amino acid sequence set forth in SEQ ID NO: 15. In one specific embodiment of the present disclosure, the cytokine receptor is IL-2β, the intracellular domain of which comprises the amino acid sequence set forth in SEQ ID NO: 22. In one specific embodiment of the present disclosure, the cytokine receptor is IL-7α, the intracellular domain of which comprises the amino acid sequence set forth in SEQ ID NO: 23.

In one specific embodiment of the present disclosure, the cytokine receptor is IL-21, the intracellular domain of which comprises the amino acid sequence set forth in SEQ ID NO: 24. In one specific embodiment of the present disclosure, the functional domain is a fusion of the intracellular domain of IL-2β to the human STAT5 activation module, which comprises the amino acid sequence set forth in SEQ ID NO: 26.

In one specific embodiment of the present disclosure, the functional domain is a fusion of the intracellular domain of IL-7α to the human STAT5 activation module, which comprises the amino acid sequence set forth in SEQ ID NO: 27.

Preferably, the first constant region is a TCRα chain constant region or a TCRγ chain constant region, preferably a modified TCRα chain constant region or a modified TCRγ chain constant region.

The TCRα chain constant region is selected from a human TCRα chain constant region or a rodent (preferably murine, and further preferably mouse) TCRα chain constant region. The TCRγ chain constant region is selected from a human TCRγ chain constant region or a rodent (preferably murine, and further preferably mouse) TCRγ chain constant region.

In one specific embodiment of the present disclosure, the amino acid sequence of the human TCRα chain constant region is set forth in SEQ ID NO: 1, and the amino acid sequence of the rodent (preferably murine, and further preferably mouse) TCRα chain constant region is set forth in SEQ ID NO: 3.

In one specific embodiment of the present disclosure, the amino acid sequence of the human TCRγ chain constant region is set forth in SEQ ID NO: 45, and the amino acid sequence of the rodent (preferably murine, and further preferably mouse) TCRγ chain constant region is set forth in SEQ ID NO: 46.

The modified TCRα chain constant region is derived from a human TCRα chain constant region comprising one or more modifications at position 48, 116, or 119 relative to a wild-type human TCRα chain constant region, the modification being a mutation or a deletion.

The modified TCRα chain constant region is derived from a human TCRα chain constant region comprising a mutation of threonine T at position 48 to cysteine C, relative to the wild-type human TCRα chain constant region.

The modified TCRα chain constant region is derived from a human TCRα chain constant region comprising a mutation of serine S at position 116 to leucine L and a mutation of glycine G at position 119 to valine V, relative to the wild-type human TCRα chain constant region.

The modified TCRα chain constant region is derived from a human TCRα chain constant region comprising a mutation of threonine T at position 48 to cysteine C, a mutation of serine S at position 116 to leucine L, and a mutation of glycine G at position 119 to valine V, relative to the wild-type human TCRα chain constant region.

The modified TCRα chain constant region is derived from a rodent (preferably murine, and further preferably mouse) TCRα chain constant region comprising one or more modifications at position 6, 13, 15-18, 48, 112, 114, or 115 relative to a wild-type rodent (preferably murine, and further preferably mouse) TCRα chain constant region, the modification being a mutation or a deletion.

The modified TCRα chain constant region is derived from a rodent (preferably murine, and further preferably mouse) TCRα chain constant region comprising one or more modifications at position 13, 36, 47, 53, 58, 78, 98, or 122 relative to the wild-type rodent (preferably murine, and further preferably mouse) TCRα chain constant region, the modification being a mutation or a deletion.

Preferably, the modified TCRα chain constant region is derived from a rodent (preferably murine, and further preferably mouse) TCRα chain constant region comprising cysteine introduction, relative to the wild-type rodent (preferably murine, and further preferably mouse) TCRα chain constant region.

Further preferably, the modified TCRα chain constant region is derived from a rodent (preferably murine, and further preferably mouse) TCRα chain constant region comprising a mutation of the amino acid at position 48, e.g., threonine T, to cysteine C, relative to the wild-type rodent (preferably murine, and further preferably mouse) TCRα chain constant region. In one specific embodiment of the present disclosure, the rodent (preferably murine, and further preferably mouse) TCRα chain constant region comprising cysteine introduction comprises the amino acid sequence set forth in SEQ ID NO: 5.

Preferably, the modified TCRα chain constant region is derived from a rodent (preferably murine, and further preferably mouse) TCRα chain constant region comprising a hydrophobic amino acid mutation, relative to the wild-type rodent (preferably murine, and further preferably mouse) TCRα chain constant region.

Further preferably, the modified TCRα chain constant region is derived from a rodent (preferably murine, and further preferably mouse) TCRα chain constant region comprising a mutation of the amino acid at position 112, e.g., serine S, to leucine L, a mutation of the amino acid at position 114, e.g., methionine M, to isoleucine I, and/or a mutation of the amino acid at position 115, e.g., glycine G, to valine V, relative to the wild-type rodent (preferably murine, and further preferably mouse) TCRα chain constant region.

In one specific embodiment of the present disclosure, the rodent (preferably murine, and further preferably mouse) TCRα chain constant region comprising a hydrophobic amino acid mutation comprises the amino acid sequence set forth in SEQ ID NO: 7.

Preferably, the modified TCRα chain constant region is derived from a rodent (preferably murine, and further preferably mouse) TCRα chain constant region comprising an N-terminal modification, relative to the wild-type rodent (preferably murine, and further preferably mouse) TCRα chain constant region.

Further preferably, the modified TCRα chain constant region is derived from a rodent (preferably murine, and further preferably mouse) TCRα chain constant region comprising a substitution of the amino acid at position 6, e.g., E, with D, a substitution of K at position 13 with R, and deletions of the amino acids at positions 15-18, relative to the wild-type rodent (preferably murine, and further preferably mouse) TCRα chain constant region.

Preferably, the modified TCRα chain constant region is derived from a rodent (preferably murine, and further preferably mouse) TCRα chain constant region comprising a mutation of lysine to arginine in the transmembrane region.

Further preferably, the modified TCRα chain constant region is derived from a rodent (preferably murine, and further preferably mouse) TCRα chain constant region comprising a substitution of the amino acid K at position 122 with R, relative to the wild-type rodent (preferably murine, and further preferably mouse) TCRα chain constant region.

In one specific embodiment of the present disclosure, the rodent (preferably murine, and further preferably mouse) TCRα chain constant region comprising a mutation of lysine to arginine in the transmembrane region comprises the amino acid sequence set forth in SEQ ID NO: 8. Preferably, the modified TCRα chain constant region is derived from a rodent (preferably murine, and further preferably mouse) TCRα chain constant region comprising cysteine introduction and a hydrophobic amino acid mutation, relative to the wild-type rodent (preferably murine, and further preferably mouse) TCRα chain constant region.

Further preferably, the modified TCRα chain constant region is derived from a rodent (preferably murine, and further preferably mouse) TCRα chain constant region comprising a mutation of the amino acid at position 48, e.g., threonine T, to cysteine C, a mutation of the amino acid at position 112, e.g., serine S, to leucine L, a mutation of the amino acid at position 114, e.g., methionine M, to isoleucine I, and a mutation of the amino acid at position 115, e.g., glycine G, to valine V, relative to the wild-type rodent (preferably murine, and further preferably mouse) TCRα chain constant region.

In one specific embodiment of the present disclosure, the rodent (preferably murine, and further preferably mouse) TCRα chain constant region comprising cysteine introduction and a hydrophobic amino acid mutation comprises the amino acid sequence set forth in SEQ ID NO: 30.

The modified TCRα chain constant region is derived from a rodent (preferably murine, and further preferably mouse) TCRα chain constant region comprising a mutation of the amino acid at position 48, e.g., threonine T, to cysteine C, and a substitution of the amino acid K at position 122 with R, relative to the wild-type rodent (preferably murine, and further preferably mouse) TCRα chain constant region.

The modified TCRα chain constant region is derived from a rodent (preferably murine, and further preferably mouse) TCRα chain constant region comprising a substitution of the amino acid at position 6, e.g., E, with D, a substitution of K at position 13 with R, deletions of the amino acids at positions 15-18, and a mutation of the amino acid at position 48, e.g., threonine T, to cysteine C, relative to the wild-type rodent (preferably murine, and further preferably mouse) TCRα chain constant region.

The modified TCRα chain constant region is derived from a rodent (preferably murine, and further preferably mouse) TCRα chain constant region comprising a mutation of the amino acid at position 48, e.g., threonine T, to cysteine C, a mutation of the amino acid at position 112, e.g., serine S, to leucine L, a mutation of the amino acid at position 114, e.g., methionine M, to isoleucine I, a mutation of the amino acid at position 115, e.g., glycine G, to valine V, and a substitution of the amino acid K at position 122 with R, relative to the wild-type rodent (preferably murine, and further preferably mouse) TCRα chain constant region.

The modified TCRα chain constant region is derived from a rodent (preferably murine, and further preferably mouse) TCRα chain constant region comprising a substitution of the amino acid at position 6, e.g., E, with D, a substitution of K at position 13 with R, deletions of the amino acids at positions 15-18, a mutation of the amino acid at position 48, e.g., threonine T, to cysteine C, a mutation of the amino acid at position 112, e.g., serine S, to leucine L, a mutation of the amino acid at position 114, e.g., methionine M, to isoleucine I, a mutation of the amino acid at position 115, e.g., glycine G, to valine V, and a substitution of the amino acid K at position 122 with R, relative to the wild-type rodent (preferably murine, and further preferably mouse) TCRα chain constant region.

The modified TCRα chain constant region is derived from a rodent (preferably murine, and further preferably mouse) TCRα chain constant region comprising a substitution of the amino acid at position 6, e.g., E, with D, a substitution of K at position 13 with R, deletions of the amino acids at positions 15-18, a mutation of the amino acid at position 48, e.g., threonine T, to cysteine C, and a substitution of the amino acid K at position 122 with R, relative to the wild-type rodent (preferably murine, and further preferably mouse) TCRα chain constant region. The modified TCRα chain constant region is derived from a rodent (preferably murine, and further preferably mouse) TCRα chain constant region comprising a substitution of the amino acid at position 6, e.g., E, with D, a substitution of K at position 13 with R, deletions of the amino acids at positions 15-18, a mutation of the amino acid at position 112, e.g., serine S, to leucine L, a mutation of the amino acid at position 114, e.g., methionine M, to isoleucine I, and a mutation of the amino acid at position 115, e.g., glycine G, to valine V, relative to the wild-type rodent (preferably murine, and further preferably mouse) TCRα chain constant region. The modified TCRα chain constant region is derived from a rodent (preferably murine, and further preferably mouse) TCRα chain constant region comprising a substitution of the amino acid at position 6, e.g., E, with D, a substitution of K at position 13 with R, deletions of the amino acids at positions 15-18, and a substitution of the amino acid K at position 122 with R, relative to the wild-type rodent (preferably murine, and further preferably mouse) TCRα chain constant region.

The modified TCRα chain constant region is derived from a rodent (preferably murine, and further preferably mouse) TCRα chain constant region comprising a substitution of the amino acid at position 6, e.g., E, with D, a substitution of K at position 13 with R, deletions of the amino acids at positions 15-18, a mutation of the amino acid at position 112, e.g., serine S, to leucine L, a mutation of the amino acid at position 114, e.g., methionine M, to isoleucine I, a mutation of the amino acid at position 115, e.g., glycine G, to valine V, and a substitution of the amino acid K at position 122 with R, relative to the wild-type rodent (preferably murine, and further preferably mouse) TCRα chain constant region.

Preferably, the modified TCRα chain constant region is derived from a rodent (preferably murine, and further preferably mouse) TCRα chain constant region comprising cysteine introduction, a hydrophobic amino acid mutation, and an N-terminal modification, relative to the wild-type rodent (preferably murine, and further preferably mouse) TCRα chain constant region.

Further preferably, the modified TCRα chain constant region is derived from a rodent (preferably murine, and further preferably mouse) TCRα chain constant region comprising a substitution of the amino acid at position 6, e.g., E, with D, a substitution of K at position 13 with R, deletions of the amino acids at positions 15-18, a mutation of the amino acid at position 48, e.g., threonine T, to cysteine C, a mutation of the amino acid at position 112, e.g., serine S, to leucine L, a mutation of the amino acid at position 114, e.g., methionine M, to isoleucine I, and a mutation of the amino acid at position 115, e.g., glycine G, to valine V, relative to the wild-type rodent (preferably murine, and further preferably mouse) TCRα chain constant region. In one specific embodiment of the present disclosure, the rodent (preferably murine, and further preferably mouse) TCRα chain constant region comprising cysteine introduction, a hydrophobic amino acid mutation, and intracellular region deletion comprises the amino acid sequence set forth in SEQ ID NO: 16.

Preferably, the modified TCRα chain constant region is derived from a rodent (preferably murine, and further preferably mouse) TCRα chain constant region comprising an N-terminal modification, cysteine introduction, and a hydrophobic amino acid mutation.

Further preferably, the modified TCRα chain constant region is derived from a rodent (preferably murine, and further preferably mouse) TCRα chain constant region comprising a substitution of the amino acid at position 6, e.g., E, with D, a substitution of K at position 13 with R, deletions of the amino acids at positions 15-18, a mutation of the amino acid at position 48, e.g., threonine T, to cysteine C, a mutation of the amino acid at position 112, e.g., serine S, to leucine L, a mutation of the amino acid at position 114, e.g., methionine M, to isoleucine I, and a mutation of the amino acid at position 115, e.g., glycine G, to valine V.

In one specific embodiment of the present disclosure, the rodent (preferably murine, and further preferably mouse) TCRα chain constant region comprising an N-terminal modification, cysteine introduction, and a hydrophobic amino acid mutation comprises the amino acid sequence set forth in SEQ ID NO: 31.

Preferably, the modified TCRα chain constant region is derived from a rodent (preferably murine, and further preferably mouse) TCRα chain constant region comprising an intracellular deletion, an N-terminal modification, cysteine introduction, and a hydrophobic amino acid mutation.

Further preferably, the modified TCRα chain constant region is derived from a rodent (preferably murine, and further preferably mouse) TCRα chain constant region comprising an intracellular deletion, a mutation of the amino acid at position 48, e.g., threonine T, to cysteine C, a mutation of the amino acid at position 112, e.g., serine S, to leucine L, a mutation of the amino acid at position 114, e.g., methionine M, to isoleucine I, a mutation of the amino acid at position 115, e.g., glycine G, to valine V, a substitution of the amino acid at position 6, e.g., E, with D, a substitution of K at position 13 with R, and deletions of the amino acids at positions 15-18.

In one specific embodiment of the present disclosure, the rodent (preferably murine, and further preferably mouse) TCRα chain constant region comprising an intracellular deletion, an N-terminal modification, cysteine introduction, and a hydrophobic amino acid mutation comprises the amino acid sequence set forth in SEQ ID NO: 43.

In one specific embodiment of the present disclosure, the first constant region comprises the amino acid sequence set forth in any one of SEQ ID NOs: 1, 3, 5, 7, 8, 16, 30, 31, or 43. Preferably, the second constant region is a TCRβ chain constant region or a TCRδ chain constant region, preferably a modified TCRβ chain constant region or a modified TCRδ chain constant region.

Further preferably, the TCRβ chain constant region is selected from a human TCRβ chain constant region or a rodent (preferably murine, and further preferably mouse) TCRβ chain constant region, and the TCRδ chain constant region is selected from a human TCRδ chain constant region or a rodent (preferably murine, and further preferably mouse) TCRδ chain constant region.

In one specific embodiment of the present disclosure, the human TCRβ chain constant region comprises the amino acid sequence set forth in SEQ ID NO: 2.

In one specific embodiment of the present disclosure, the rodent (preferably murine, and further preferably mouse) TCRβ chain constant region comprises the amino acid sequence set forth in SEQ ID NO: 4.

In one specific embodiment of the present disclosure, the human TCRδ chain constant region comprises the amino acid sequence set forth in SEQ ID NO: 47.

In one specific embodiment of the present disclosure, the rodent (preferably murine, and further preferably mouse) TCRδ chain constant region comprises the amino acid sequence set forth in SEQ ID NO: 48.

The modified TCRβ chain constant region is derived from a human TCRβ chain constant region comprising one or more modifications at position 57, 173, or 175 relative to a wild-type human TCRβ chain constant region, the modification being a mutation or a deletion.

The modified TCRβ chain constant region is derived from a human TCRβ chain constant region comprising a mutation of serine S at position 57 to cysteine C, relative to the wild-type human TCRβ chain constant region.

The modified TCRβ chain constant region is derived from a human TCRβ chain constant region comprising mutations of lysine K at positions 173 and 175 to arginine, relative to the wild-type human TCRβ chain constant region.

The modified TCRβ chain constant region is derived from a human TCRβ chain constant region comprising a mutation of serine S at position 57 to cysteine C and mutations of lysine K at positions 173 and 175 to arginine, relative to the wild-type human TCRβ chain constant region. The modified TCRβ chain constant region is derived from a rodent (preferably murine, and further preferably mouse) TCRβ chain constant region comprising one or more modifications at position 3, 6, 9, 11, 12, 17, 21-25, 56, 150, 168, or 170 relative to a wild-type rodent (preferably murine, and further preferably mouse) TCRβ chain constant region, the modification being a mutation or a deletion.

The modified TCRβ chain constant region is derived from a rodent (preferably murine, and further preferably mouse) TCRβ chain constant region comprising one or more modifications at position 9, 17, 23, 25, 49, 63, 103, 110, 150, 168, or 170 relative to the wild-type rodent (preferably murine, and further preferably mouse) TCRβ chain constant region, the modification being a mutation or a deletion.

Preferably, the modified TCRβ chain constant region is derived from a rodent (preferably murine, and further preferably mouse) TCRβ chain constant region comprising cysteine introduction, relative to the wild-type rodent (preferably murine, and further preferably mouse) TCRβ chain constant region.

Further preferably, the modified TCRβ chain constant region is derived from a rodent (preferably murine, and further preferably mouse) TCRβ chain constant region comprising a mutation of the amino acid at position 56, e.g., serine S, to cysteine C, relative to the wild-type rodent (preferably murine, and further preferably mouse) TCRβ chain constant region.

In one specific embodiment of the present disclosure, the rodent (preferably murine, and further preferably mouse) TCRβ chain constant region comprising cysteine introduction comprises the amino acid sequence set forth in SEQ ID NO: 6.

Preferably, the modified TCRβ chain constant region is derived from a rodent (preferably murine, and further preferably mouse) TCRβ chain constant region comprising a substitution of lysine with arginine in the intracellular region.

Further preferably, the modified TCRβ chain constant region is derived from a rodent (preferably murine, and further preferably mouse) TCRβ chain constant region comprising a substitution of lysine at position 150, 168, or 170 with arginine.

In one specific embodiment of the present disclosure, the rodent (preferably murine, and further preferably mouse) TCRβ chain constant region comprising a substitution of lysine with arginine in the intracellular region comprises the amino acid sequence set forth in SEQ ID NO: 9. Preferably, the modified TCRβ chain constant region is derived from a rodent (preferably murine, and further preferably mouse) TCRβ chain constant region comprising an N-terminal modification, relative to the wild-type rodent (preferably murine, and further preferably mouse) TCRβ chain constant region.

Further preferably, the modified TCRβ chain constant region is derived from a rodent (preferably murine, and further preferably mouse) TCRβ chain constant region comprising a substitution of the amino acid at position 3, e.g., R, with K, a substitution of the amino acid at position 6, e.g., T, with F, a substitution of K at position 9 with E, a substitution of S at position 11 with A, a substitution of L at position 12 with V, and deletions of the amino acids at positions 17 and 21-25, relative to the wild-type rodent (preferably murine, and further preferably mouse) TCRβ chain constant region.

Preferably, the modified TCRβ chain constant region is derived from a rodent (preferably murine, and further preferably mouse) TCRβ chain constant region comprising cysteine introduction and an intracellular region deletion, relative to the wild-type rodent (preferably murine, and further preferably mouse) TCRβ chain constant region.

In one specific embodiment of the present disclosure, the rodent (preferably murine, and further preferably mouse) TCRβ chain constant region comprising cysteine introduction and an intracellular region deletion comprises the amino acid sequence set forth in SEQ ID NO: 17. The modified TCRβ chain constant region is derived from a rodent (preferably murine, and further preferably mouse) TCRβ chain constant region comprising a mutation of the amino acid at position 56, e.g., serine S, to cysteine C, and a substitution of lysine at position 150, 168, or 170 with arginine, relative to the wild-type rodent (preferably murine, and further preferably mouse) TCRβ chain constant region.

The modified TCRβ chain constant region is derived from a rodent (preferably murine, and further preferably mouse) TCRβ chain constant region comprising a substitution of the amino acid at position 3, e.g., R, with K, a substitution of the amino acid at position 6, e.g., T, with F, a substitution of K at position 9 with E, a substitution of S at position 11 with A, a substitution of L at position 12 with V, deletions of the amino acids at positions 17 and 21-25, a mutation of the amino acid at position 56, e.g., serine S, to cysteine C, and a substitution of lysine at position 150, 168, or 170 with arginine, relative to the wild-type rodent (preferably murine, and further preferably mouse) TCRβ chain constant region.

The modified TCRβ chain constant region is derived from a rodent (preferably murine, and further preferably mouse) TCRβ chain constant region comprising a substitution of the amino acid at position 3, e.g., R, with K, a substitution of the amino acid at position 6, e.g., T, with F, a substitution of K at position 9 with E, a substitution of S at position 11 with A, a substitution of L at position 12 with V, deletions of the amino acids at positions 17 and 21-25, and a substitution of lysine at position 150, 168, or 170 with arginine, relative to the wild-type rodent (preferably murine, and further preferably mouse) TCRβ chain constant region.

Preferably, the modified TCRβ chain constant region is derived from a rodent (preferably murine, and further preferably mouse) TCRβ chain constant region comprising an N-terminal modification and cysteine introduction, relative to the wild-type rodent (preferably murine, and further preferably mouse) TCRβ chain constant region.

The modified TCRβ chain constant region is derived from a rodent (preferably murine, and further preferably mouse) TCRβ chain constant region comprising a substitution of the amino acid at position 3, e.g., R, with K, a substitution of the amino acid at position 6, e.g., T, with F, a substitution of K at position 9 with E, a substitution of S at position 11 with A, a substitution of L at position 12 with V, deletions of the amino acids at positions 17 and 21-25, and a mutation of the amino acid at position 56, e.g., serine S, to cysteine C, relative to the wild-type rodent (preferably murine, and further preferably mouse) TCRβ chain constant region.

In one specific embodiment of the present disclosure, the rodent (preferably murine, and further preferably mouse) TCRβ chain constant region comprising an N-terminal modification and cysteine introduction comprises the amino acid sequence set forth in SEQ ID NO: 32. Preferably, the modified TCRβ chain constant region is derived from a rodent (preferably murine, and further preferably mouse) TCRβ chain constant region comprising an intracellular deletion, an N-terminal modification, and cysteine introduction, relative to the wild-type rodent (preferably murine, and further preferably mouse) TCRβ chain constant region.

Further preferably, the modified TCRβ chain constant region is derived from a rodent (preferably murine, and further preferably mouse) TCRβ chain constant region comprising an intracellular deletion, a mutation of the amino acid at position 56, e.g., serine S, to cysteine C, a substitution of the amino acid at position 3, e.g., R, with K, a substitution of the amino acid at position 6, e.g., T, with F, a substitution of K at position 9 with E, a substitution of S at position 11 with A, a substitution of L at position 12 with V, and deletions of the amino acids at positions 17 and 21-25, relative to the wild-type rodent (preferably murine, and further preferably mouse) TCRβ chain constant region.

In one specific embodiment of the present disclosure, the rodent (preferably murine, and further preferably mouse) TCRβ chain constant region comprising an intracellular deletion, an N-terminal modification, and cysteine introduction comprises the amino acid sequence set forth in SEQ ID NO: 44.

In one specific embodiment of the present disclosure, the second constant region comprises the amino acid sequence set forth in any one of SEQ ID NOs: 2, 4, 6, 9, 17, 32, or 44.

The target-binding region is located at the N-terminus of the constant region. The target-binding region and the constant region may be linked directly or via a linker.

The first target-binding region may comprise one or more, the same or different, binding regions. The binding region is an antigen-binding region or a fragment thereof, a non-immunoglobulin antigen-binding domain or a fragment thereof, an antibody-binding region or a fragment thereof, a receptor or a fragment thereof, or a ligand or a fragment thereof; preferably, the receptor is a natural T cell receptor.

The antigen-binding region is derived from an antibody.

The STAR comprises one or a plurality of antigen-binding regions;
preferably, the plurality of antigen-binding regions are the same or different;
and further preferably, the plurality of antigen-binding regions are linked directly or via a linker.

The antibody may be a monoclonal antibody or a polyclonal antibody.

The antibody may further comprise a fragment such as an F_{ab}, an F_{ab}', an F_{ab}'-SH, an Fv, an scFv, an (F_{ab}')₂, a single domain antibody, a diabody (dAb), or a linear antibody.

The antibody may be a monospecific antibody or a multispecific antibody (e.g., bispecific antibody).

Preferably, the antibody may be a fully human antibody, a humanized antibody, or an animal-derived antibody, wherein the animal may be a mouse, a rabbit, a cow, a monkey, etc. Preferably, the first target-binding region is linked, directly or via a linker, to the first constant region, and/or the second target-binding region is linked, directly or via a linker, to the second constant region.

Preferably, the first target-binding region and the second target-binding region, each independently or in combination, specifically bind to a target antigen.

Preferably, the target antigen is a disease-related antigen, preferably a cancer-related antigen, such as a cancer-related antigen selected from the following antigens: LILRB4, GPC3, CD16, CD64, CD78, CD96, CLL1, CD116, CD117, CD71, CD45, CD71, CD123, CD138, ErbB2 (HER2/neu), carcinoembryonic antigen (CEA), epithelial cell adhesion molecule (EpCAM), epidermal growth factor receptor (EGFR), EGFR variant III (EGFRvIII), CD19, CD20, CD30, CD40, disialoganglioside GD2, ductal epithelial mucin, gp36, TAG-72, glycosphingolipid, neuroglioma-related antigens, β-human chorionic gonadotropin, α-fetoprotein (AFP), lectin-reactive AFP, thyroglobulin, RAGE-1, MN-CA IX, human telomerase reverse transcriptase, RU1, RU2 (AS), intestinal carboxylesterase, mut hsp70-2, M-CSF, prostase, prostase-specific antigen (PSA), PAP, NY-ESO-1, LAGA-1a, p53, Prostein, PSMA, survival and telomerase, prostate cancer tumor antigen-1 (PCTA-1), MAGE, ELF2M, neutrophil elastase, ephrin B2, CD22, insulin growth factor (IGF1)-I, IGF-II, IGFI receptor, mesothelin, major histocompatibility complex (MHC) molecules presenting tumor-specific peptide epitopes, 5T4, ROR1, Nkp30, NKG2D, tumor stromal antigen, fibronectin extra domain A (EDA) and extra domain B (EDB), tenascin-C A1 domain (TnC A1), fibroblast-associated protein (fap), CD3, CD4, CD8, CD24, CD25, CD33, CD34, CD133, CD138, Foxp3, B7-1 (CD80), B7-2 (CD86), GM-CSF, cytokine receptors, endothelial factors, major histocompatibility complex (MHC) molecules, BCMA (CD269, TNFRSF17), TNFRSF17 (UNIPROTQ02223), SLAMF7 (LTNIPROT Q9NQ25), GPRC5D (UNIPROT Q9NZD1), FKBP11 (UNIPROT Q9NYL4), KAMP3, ITGA8 (UNIPROT P53708), or FCRL5 (UNIPROT Q68SN8).

Preferably, the first target-binding region comprises one or a plurality of antibodies or antibody fragments specifically binding to the target antigen, and the second target-binding region comprises one or a plurality of antibodies or antibody fragments specifically binding to the target antigen.

The plurality of antibodies or antibody fragments are linked directly or via a linker.

Preferably, the first target-binding region comprises one or a plurality of single chain antibodies or one or a plurality of single domain antibodies specifically binding to the target antigen; and/or the second target-binding region comprises one or a plurality of single chain antibodies or one or a plurality of single domain antibodies specifically binding to the target antigen.

The plurality of single chain antibodies are linked directly or via a linker. The plurality of single domain antibodies are linked directly or via a linker.

Preferably, the single chain antibody comprises a heavy chain variable region and a light chain variable region, which are linked directly or via a linker.

Preferably, the plurality of antigen-binding regions in the first target-binding region and/or the second target-binding region bind to the same or different target antigens.

Preferably, the plurality of antigen-binding regions in the first target-binding region and/or the second target-binding region bind to different regions of the same target antigen, such as different epitopes.

In one specific embodiment of the present disclosure, the target antigen is LILRB4.

In one specific embodiment of the present disclosure, the antigen-binding region in the first target-binding region comprises one or a plurality of single domain antibodies, and/or the antigen-binding region in the second target-binding region comprises one or a plurality of single domain antibodies;
preferably, the plurality of single domain antibodies comprised in the antigen-binding region in the first target-binding region are the same or different;
preferably, the plurality of single domain antibodies comprised in the antigen-binding region in the second target-binding region are the same or different;
further preferably, the plurality of single domain antibodies are linked directly or via a linker.

Preferably, the single domain antibody comprised in the antigen-binding region in the first target-binding region and the single domain antibody comprised in the antigen-binding region in the second target-binding region are the same or different.

In one specific embodiment of the present disclosure, the single domain antibody comprises a heavy chain variable region, and the heavy chain variable region comprises CDR1-3, wherein
i) the CDR1 comprises the amino acid sequence set forth in SEQ ID NO: 33, the CDR2 comprises the amino acid sequence set forth in SEQ ID NO: 34, and the CDR3 comprises the amino acid sequence set forth in SEQ ID NO: 35;
   or,
ii) the CDR1 comprises the amino acid sequence set forth in SEQ ID NO: 36, the CDR2 comprises the amino acid sequence set forth in SEQ ID NO: 37, and the CDR3 comprises the amino acid sequence set forth in SEQ ID NO: 38.

In one specific embodiment of the present disclosure, the single domain antibody comprises the amino acid sequence set forth in SEQ ID NO: 28 or 29.

In a second aspect of the present disclosure, provided is a STAR complex, wherein
i) the STAR complex comprises an α chain, a β chain, CD3ε, CD3γ, CD3δ, and CD3ζ, wherein the α chain comprises a first target-binding region and a first constant region, and the β chain comprises a second target-binding region and a second constant region; or the α chain comprises a first target-binding region, and the β chain comprises a second target-binding region and a second constant region; or
ii) the STAR complex comprises a γ chain, a δ chain, CD3ε, CD3γ, CD3δ, and CD3ζ, wherein the γ chain comprises a first target-binding region and a first constant region, and the δ chain comprises a second target-binding region and a second constant region; or the γ chain comprises a first target-binding region and a first constant region, and the δ chain comprises a second target-binding region and a second constant region.

Preferably, i) at least one of the α chain, β chain, CD3ε, CD3γ, CD3δ, and CD3ζ is linked at its C-terminus to at least one functional domain; or ii) at least one of the γ chain, δ chain, CD3ε, CD3γ, CD3δ, and CD3ζ is linked at its C-terminus to at least one functional domain.

Preferably, i) the at least one functional domain is linked, directly or via a linker, to the C-terminus of at least one of the α chain, β chain, CD3ε, CD3γ, CD3δ, and CD3ζ; or ii) the at least one functional domain is linked, directly or via a linker, to the C-terminus of at least one of the γ chain, δ chain, CD3ε, CD3γ, CD3δ, and CD3ζ.

Preferably, i) an intracellular region of at least one of the α chain, β chain, CD3ε, CD3γ, CD3δ, and CD3ζ in the STAR complex is deleted; or ii) an intracellular region of at least one of the γ chain, δ chain, CD3ε, CD3γ, CD3δ, and CD3ζ in the STAR complex is deleted.

Further preferably, i) the at least one functional domain is linked, directly or via a linker, to the C-terminus of at least one of the α chain, β chain, CD3ε, CD3γ, CD3δ, and CD3ζ with the intracellular region deleted; or ii) the at least one functional domain is linked, directly or via a linker, to the C-terminus of at least one of the γ chain, δ chain, CD3ε, CD3γ, CD3δ, and CD3ζ with the intracellular region deleted.

In one specific embodiment of the present disclosure, i) the C-terminus of at least one of the α chain, β chain, CD3ε, CD3γ, CD3δ, and CD3ζ in the STAR complex is linked to 1, 2, 3, 4, 5, 6, 7, 8, 9, 10, or more functional domains; or ii) the C-terminus of at least one of the γ chain, δ chain, CD3ε, CD3γ, CD3δ, and CD3ζ in the STAR complex is linked to 1, 2, 3, 4, 5, 6, 7, 8, 9, 10, or more functional domains.

Preferably, the plurality of functional domains may be linked directly or via a linker.

Preferably, i) the functional domains linked to the C-terminus of at least one of the α chain, β chain, CD3ε, CD3γ, CD3δ, and CD3ζ in the STAR complex are the same or different; or ii) the functional domains linked to the C-terminus of at least one of the γ chain, δ chain, CD3ε, CD3γ, CD3δ, and CD3ζ in the STAR complex are the same or different.

In one specific embodiment of the present disclosure, the plurality of functional domains linked to the α chain may be the same or different. The plurality of functional domains linked to the β chain may be the same or different. The plurality of functional domains linked to the γ chain may be the same or different. The plurality of functional domains linked to the δ chain may be the same or different. The plurality of functional domains linked to the CD3ε may be the same or different. The plurality of functional domains linked to the CD3γ may be the same or different. The plurality of functional domains linked to the CD3δ may be the same or different. The plurality of functional domains linked to the CD3ζ may be the same or different.

In one specific embodiment of the present disclosure, the functional domains separately linked to the α chain, β chain, CD3ε, CD3γ, CD3δ, and CD3ζ may be the same or different.

In one specific embodiment of the present disclosure, the functional domains separately linked to the γ chain, δ chain, CD3ε, CD3γ, CD3δ, and CD3ζ may be the same or different.

Preferably, the functional domain is a co-stimulatory molecule or a fragment thereof, a co-inhibitory molecule or a fragment thereof, a cytokine receptor or a fragment thereof, or an intracellular protein or a fragment thereof. Further preferably, the functional domain is an intracellular domain of a co-stimulatory molecule, an intracellular domain of a co-inhibitory molecule, an intracellular domain of a cytokine receptor, or an intracellular protein;
preferably, the co-stimulatory molecule is selected from CD40, OX40, ICOS, CD28, 4-1BB (CD137), or CD27;
preferably, the co-inhibitory molecule is selected from TIM3, PD1, CTLA4, or LAG3;
preferably, the cytokine receptor is selected from an interleukin receptor (e.g., an IL-2 receptor), an interferon receptor, a tumor necrosis factor superfamily receptor, a colony-stimulating factor receptor, a chemokine receptor, a growth factor receptor, and other membrane proteins;
preferably, the intracellular protein is a domain of a T-cell regulatory factor, e.g., a domain of NIK.

In one specific embodiment of the present disclosure, the co-stimulatory molecule is CD40, the intracellular domain of which comprises the amino acid sequence set forth in SEQ ID NO: 10. In one specific embodiment of the present disclosure, the co-stimulatory molecule is OX40, the intracellular domain of which comprises the amino acid sequence set forth in SEQ ID NO: 11. In one specific embodiment of the present disclosure, the co-stimulatory molecule is ICOS, the intracellular domain of which comprises the amino acid sequence set forth in SEQ ID NO: 12. In one specific embodiment of the present disclosure, the co-stimulatory molecule is CD28, the intracellular domain of which comprises the amino acid sequence set forth in SEQ ID NO: 13. In one specific embodiment of the present disclosure, the co-stimulatory molecule is 4-1BB, the intracellular domain of which comprises the amino acid sequence set forth in SEQ ID NO: 14. In one specific embodiment of the present disclosure, the co-stimulatory molecule is CD27, the intracellular domain of which comprises the amino acid sequence set forth in SEQ ID NO: 15. In one specific embodiment of the present disclosure, the STAR complex comprises the STAR described above, as well as CD3ε, CD3γ, CD3δ, and CD3ζ.

Preferably, the CD3ε, CD3γ, CD3δ, and/or CD3ζ is of human origin.

In one specific embodiment of the present disclosure, the CD3ε comprises the amino acid sequence set forth in SEQ ID NO: 20.

In one specific embodiment of the present disclosure, the CD3γ comprises the amino acid sequence set forth in SEQ ID NO: 18.

In one specific embodiment of the present disclosure, the CD3δ comprises the amino acid sequence set forth in SEQ ID NO: 19.

In one specific embodiment of the present disclosure, the CD3ζ comprises the amino acid sequence set forth in SEQ ID NO: 21.

In a third aspect of the present disclosure, provided is an antibody or an antigen-binding fragment, comprising a heavy chain variable region and/or a light chain variable region.

The heavy chain variable region comprises CDR1-3, wherein
i) the CDR1 comprises the amino acid sequence set forth in SEQ ID NO: 33, the CDR2 comprises the amino acid sequence set forth in SEQ ID NO: 34, and the CDR3 comprises the amino acid sequence set forth in SEQ ID NO: 35;
   or,
ii) the CDR1 comprises the amino acid sequence set forth in SEQ ID NO: 36, the CDR2 comprises the amino acid sequence set forth in SEQ ID NO: 37, and the CDR3 comprises the amino acid sequence set forth in SEQ ID NO: 38.

The antibody may further comprise a fragment such as an F_{ab}, an F_{ab}', an F_{ab}'-SH, an Fv, an scFv, an (F_{ab}')₂, a single domain antibody, a diabody (dAb), or a linear antibody.

The antibody may be a monospecific antibody or a multispecific antibody (e.g., bispecific antibody).

Preferably, the antibody may be a fully human antibody, a humanized antibody, or an animal-derived antibody, wherein the animal may be a mouse, a rabbit, a cow, a monkey, etc.

In one specific embodiment of the present disclosure, the antibody or the antigen-binding fragment is a single chain antibody or a single domain antibody.

In one specific embodiment of the present disclosure, the antibody or the antigen-binding fragment comprises the amino acid sequence set forth in SEQ ID NO: 28 or 29.

In a fourth aspect of the present disclosure, provided is a single chain antibody comprising a heavy chain variable region and/or a light chain variable region.

The heavy chain variable region comprises CDR1-3, wherein
i) the CDR1 comprises the amino acid sequence set forth in SEQ ID NO: 33, the CDR2 comprises the amino acid sequence set forth in SEQ ID NO: 34, and the CDR3 comprises the amino acid sequence set forth in SEQ ID NO: 35;
   or,
ii) the CDR1 comprises the amino acid sequence set forth in SEQ ID NO: 36, the CDR2 comprises the amino acid sequence set forth in SEQ ID NO: 37, and the CDR3 comprises the amino acid sequence set forth in SEQ ID NO: 38.

In one specific embodiment of the present disclosure, the antibody or the antigen-binding fragment comprises the amino acid sequence set forth in SEQ ID NO: 28 or 29.

In a fifth aspect of the present disclosure, provided is a single domain antibody comprising a heavy chain variable region, and the heavy chain variable region comprises CDR1-3, wherein i) the CDR1 comprises the amino acid sequence set forth in SEQ ID NO: 33, the CDR2 comprises the amino acid sequence set forth in SEQ ID NO: 34, and the CDR3 comprises the amino acid sequence set forth in SEQ ID NO: 35; or ii) the CDR1 comprises the amino acid sequence set forth in SEQ ID NO: 36, the CDR2 comprises the amino acid sequence set forth in SEQ ID NO: 37, and the CDR3 comprises the amino acid sequence set forth in SEQ ID NO: 38.

In one specific embodiment of the present disclosure, the single domain antibody comprises the amino acid sequence set forth in SEQ ID NO: 28 or 29.

In a sixth aspect of the present disclosure, provided is a preparation method for the antibody or the antigen-binding fragment described above or the single domain antibody described above, the method comprising: preparing a phage display library, and screening the phage display library to obtain the antibody or the antigen-binding fragment or the single domain antibody.

In a seventh aspect of the present disclosure, provided is an antigen receptor comprising a transmembrane region, an intracellular region, and one or more, the same or different, extracellular binding domains.

The antigen receptor is a TCR or a CAR.

In one specific embodiment of the present disclosure, the antigen receptor is a CAR.

The extracellular binding domain is an extracellular antigen-binding domain, an extracellular antibody-binding domain, a receptor, or a ligand; preferably, the receptor is a natural T cell receptor.

In one specific embodiment of the present disclosure, the extracellular binding domain is an extracellular antigen-binding domain.

The extracellular antigen-binding domain is derived from an antibody.

Preferably, the transmembrane region is linked, directly or via a linker, to one or more extracellular antigen-binding domains.

Preferably, the antigen is selected from cancer-related antigens, such as a cancer-related antigen selected from the following antigens: LILRB4, GPC3, CD16, CD64, CD78, CD96, CLL1, CD116, CD117, CD71, CD45, CD71, CD123, CD138, ErbB2 (HER2/neu), carcinoembryonic antigen (CEA), epithelial cell adhesion molecule (EpCAM), epidermal growth factor receptor (EGFR), EGFR variant III (EGFRvIII), CD19, CD20, CD30, CD40, disialoganglioside GD2, ductal epithelial mucin, gp36, TAG-72, glycosphingolipid, neuroglioma-related antigens, β-human chorionic gonadotropin, α-fetoprotein (AFP), lectin-reactive AFP, thyroglobulin, RAGE-1, MN-CA IX, human telomerase reverse transcriptase, RU1, RU2 (AS), intestinal carboxylesterase, mut hsp70-2, M-CSF, prostase, prostase-specific antigen (PSA), PAP, NY-ESO-1, LAGA-1a, p53, Prostein, PSMA, survival and telomerase, prostate cancer tumor antigen-1 (PCTA-1), MAGE, ELF2M, neutrophil elastase, ephrin B2, CD22, insulin growth factor (IGF1)-I, IGF-II, IGFI receptor, mesothelin, major histocompatibility complex (MHC) molecules presenting tumor-specific peptide epitopes, 5T4, ROR1, Nkp30, NKG2D, tumor stromal antigen, fibronectin extra domain A (EDA) and extra domain B (EDB), tenascin-C A1 domain (TnC A1), fibroblast-associated protein (fap), CD3, CD4, CD8, CD24, CD25, CD33, CD34, CD133, CD138, Foxp3, B7-1 (CD80), B7-2 (CD86), GM-CSF, cytokine receptors, endothelial factors, major histocompatibility complex (MHC) molecules, BCMA (CD269, TNFRSF17), TNFRSF17 (UNIPROTQ02223), SLAMF7 (UNIPROT Q9NQ25), GPRC5D (UNIPROT Q9NZD1), FKBP11 (UNIPROT Q9NYL4), KAMP3, ITGA8 (UNIPROT P53708), or FCRL5 (UNIPROT Q68SN8);
preferably, the antigen is LILRB4.

Preferably, the extracellular antigen-binding domain comprises CDR1-3, wherein i) the CDR1 comprises the amino acid sequence set forth in SEQ ID NO: 33, the CDR2 comprises the amino acid sequence set forth in SEQ ID NO: 34, and the CDR3 comprises the amino acid sequence set forth in SEQ ID NO: 35; or ii) the CDR1 comprises the amino acid sequence set forth in SEQ ID NO: 36, the CDR2 comprises the amino acid sequence set forth in SEQ ID NO: 37, and the CDR3 comprises the amino acid sequence set forth in SEQ ID NO: 38.

In one specific embodiment of the present disclosure, the extracellular antigen-binding domain comprises the antibody or the antigen-binding fragment described above, the single chain antibody described above, or the single domain antibody described above.

Preferably, the transmembrane region is derived from human CD8.

Preferably, the intracellular region is derived from 4-1BB, CD28, or CD3ζ.

In an eighth aspect of the present disclosure, provided is a nucleic acid encoding the STAR described above, the STAR complex described above, the antibody or the antigen-binding fragment described above, the single chain antibody described above, the single domain antibody described above, or the antigen receptor described above.

In a ninth aspect of the present disclosure, provided is a vector comprising the nucleic acid described above.

The vector is capable of expression *in vivo* or *in vitro* or *ex vivo,* and is preferably an expression vector. Preferably, the expression vector is a prokaryotic expression vector, a viral expression vector, a plasmid, a cosmid, a phage, a virus, etc.

Preferably, the prokaryotic expression vector is of the *Escherichia coli* series, such as pET-26b or pET28a+.

Preferably, the virus may be Rous sarcoma virus (RSV), lentivirus, human immunodeficiency virus (HIV), murine leukemia virus (MLV), equine infectious anemia virus (EIAV), mouse mammary tumor virus (MMTV), Fujinami sarcoma virus (FuSV), FBR murine sarcoma virus (FBR MSV), Moloney murine leukemia virus (Mo-MLV), Moloney murine sarcoma virus (Mo-MSV), Abelson murine leukemia virus (A-MLV), avian myelocytomatosis virus 29 (MC29), or avian erythroblastosis virus (AEV), etc. Still further preferably, the expression vector is a lentiviral expression vector, such as pHAGE-IRES-RFP.

In a tenth aspect of the present disclosure, provided is a host cell comprising the nucleic acid described above or the vector described above.

Preferably, the host cell may be eukaryotic or prokaryotic. More preferably, the host cell is a yeast cell, a 293 cell, a CHO cell, *Escherichia coli,* etc.

In an eleventh aspect of the present disclosure, provided is an immune cell expressing the STAR described above, the STAR complex described above, the antibody or the antigen-binding fragment described above, the single domain antibody described above, or the antigen receptor described above.

Preferably, the immune cell comprises one or more of the nucleic acids described above.

Preferably, the immune cell is selected from a T cell, a Treg cell, a macrophage, an NK cell, an NKT cell, a peripheral blood mononuclear cell, a TIL cell, and a dendritic cell (DC). Preferably, the immune cell is isolated from a T cell derived from a subject.

In one specific embodiment of the present disclosure, the immune cell is selected from a T cell, an NK cell, a CTL, a human embryonic stem cell, a lymphoid progenitor cell, and/or a T-cell precursor cell.

In a twelfth aspect of the present disclosure, provided is a CAR-T cell comprising the antibody or the antigen fragment described above, the single chain antibody described above, or the single domain antibody described above.

In a thirteenth aspect of the present disclosure, provided is a preparation method for an immune cell, the method comprises transfecting an immune cell with the nucleic acid sequence described above for expression.

In a fourteenth aspect of the present disclosure, provided is a preparation method for a recombinant T cell, which comprises the following steps:
1) obtaining the nucleic acid described above from a positive T cell clone;
2) isolating and culturing a primary T cell; and
3) delivering the nucleic acid obtained in step 1) to the primary T cell in step 2) to obtain a recombinant T cell expressing the STAR described above.

In a fifteenth aspect of the present disclosure, provided is a preparation method for a STAR or a STAR complex, which comprises the following steps:
(1) obtaining the nucleic acid described above from a positive T cell clone;
(2) ligating the nucleic acid obtained in step (1) to a vector backbone to obtain an expression vector;
(3) transforming the expression vector obtained in step (2) into a host cell, and subsequently, inducing the expression thereof; and
(4) obtaining a STAR.

In a sixteenth aspect of the present disclosure, provided is a preparation method for an antibody or an antigen-binding fragment, a single chain antibody, or a single domain antibody, the method comprises protein immunization and/or DNA immunization.

In a seventeenth aspect of the present disclosure, provided is a preparation method for an antibody or an antigen-binding fragment, a single chain antibody, or a single domain antibody, the method comprises:
A) obtaining the encoded nucleic acid sequence; and
B) transforming the nucleic acid sequence obtained in step A) into a host cell, subsequently inducing the expression thereof, and purifying the host cell.

In an eighteenth aspect of the present disclosure, provided is use of the STAR described above, the STAR complex described above, the antibody or the antigen-binding fragment described above, the single domain antibody described above, the antigen receptor described above, the nucleic acid described above, or the immune cell described above in preparing a product for the diagnosis or treatment of a tumor.

Preferably, the tumor includes, but is not limited to, lymphoma, non-small cell lung cancer, leukemia, ovarian cancer, nasopharyngeal cancer, breast cancer, endometrial cancer, colon cancer, rectal cancer, stomach cancer, bladder cancer, lung cancer, bronchial cancer, bone cancer, prostate cancer, pancreatic cancer, liver and bile duct cancer, esophageal cancer, kidney cancer, thyroid cancer, head and neck cancer, testicular cancer, glioblastoma, astrocytoma, melanoma, myelodysplastic syndrome, and sarcoma;
preferably, the leukemia is selected from acute lymphocytic (lymphoblastic) leukemia, acute myeloid leukemia, myeloid leukemia, chronic lymphocytic leukemia, multiple myeloma, plasma cell leukemia, and chronic myeloid leukemia;
preferably, the lymphoma is selected from Hodgkin's lymphoma and non-Hodgkin's lymphoma, comprising B-cell lymphoma, diffuse large B-cell lymphoma, follicular lymphoma, mantle cell lymphoma, marginal zone B-cell lymphoma, T-cell lymphoma, and Waldenstrom macroglobulinemia;
preferably, the sarcoma is selected from osteosarcoma, Ewing's sarcoma, leiomyosarcoma, synovial sarcoma, soft tissue sarcoma, angiosarcoma, liposarcoma, fibrosarcoma, rhabdomyosarcoma, and chondrosarcoma;
preferably, the acute myeloid leukemia is M4 or M5 acute myeloid leukemia;
preferably, the chronic myeloid leukemia is chronic myelomonocytic leukemia.

In a nineteenth aspect of the present disclosure, provided is an immunoconjugate or an antibody-drug conjugate, comprising the antibody or the antigen-binding fragment described above, the single chain antibody described above, or the single domain antibody described above of the present disclosure conjugated to a therapeutic agent or a diagnostic agent.

In a twentieth aspect of the present disclosure, provided is a pharmaceutical composition comprising the STAR described above, the STAR complex described above, the antibody or the antigen-binding fragment described above, the single domain antibody described above, the antigen receptor described above, the nucleic acid described above, or the immune cell described above.

Preferably, the medicament further comprises a pharmaceutically acceptable auxiliary material. Further preferably, the pharmaceutically acceptable auxiliary material includes, but is not limited to, a diluent, a binder, a humectant, a surfactant, a lubricant, a disintegrant, etc.

In a twenty-first aspect of the present disclosure, provided is a kit comprising the STAR described above, the STAR complex described above, the antibody or the antigen-binding fragment described above, the single domain antibody described above, the antigen receptor described above, the nucleic acid described above, or the immune cell described above.

In a twenty-second aspect of the present disclosure, provided is a method for treating a tumor, the method comprises administering to a subject an effective amount of the STAR, the STAR complex, the CAR, the antibody or the antigen-binding fragment thereof, the single chain antibody, the single domain antibody, the immune cell, the CAR-T cell, or the pharmaceutical composition described herein.

The term "linker" described herein includes, but is not limited to, a rigid linker, a flexible linker, a cleavable linker, or a non-essential amino acid. Preferably, the amino acid sequence of the rigid linker is selected from one or a combination of two or more of SEQ ID NOs: 49-59. Preferably, the flexible linker is selected from a peptide fragment rich in glycine and/or serine; preferably, the flexible linker is selected from one or a combination of two or more of SEQ ID NOs: 60-112. preferably, the cleavable linker is selected from one or a combination of two or more of SEQ ID NOs: 113-117.

The term "antibody" described herein may be of any class (e.g., IgA, IgD, IgE, IgG, and IgM) or subclass (e.g., IgG1, IgG2, IgG3, IgG4, IgA1, or IgA2).

The term "antigen-binding fragment" described herein includes, but is not limited to: a Fab fragment, having VL, CL, VH and CH1 domains; a Fab' fragment, which is a Fab fragment having one or more cysteine residues at the C-terminus of the CH1 domain; an Fd fragment, having VH and CH1 domains; an Fd' fragment, having VH and CH1 domains and one or more cysteine residues at the C-terminus of the CH1 domain; an Fv fragment, having VL and VH domains of a single arm of an antibody; a dAb fragment, consisting of a VH domain or a VL domain; an isolated CDR region; an F(ab')2 fragment, which is a bivalent fragment comprising two Fab' fragments connected by a disulfide bridge at the hinge region; a single chain antibody molecule (e.g., single chain Fv; scFv); a "diabody" with two antigen-binding sites, comprising a heavy chain variable domain (VH) linked to a light chain variable domain (VL) in the same polypeptide chain; a "linear antibody", comprising a pair of tandem Fd segments (VH-CH1-VH-CH1) that, together with a complementary light chain polypeptide, form a pair of antigen-binding regions; and a modified form of any of the foregoing, which retains antigen-binding activity.

The term "CDR" described herein refers to a complementarity determining region within an antibody variable sequence. For each variable region, there are three CDRs in each variable region of the heavy and light chains, which are referred to as CDR1, CDR2, and CDR3. The exact boundaries of these CDRs are defined differently according to different systems. The system described by Kabat et al. (Kabat et al, Sequences of Proteins of Immunological Interest (National Institutes of Health, Bethesda, Md. (1987) and (1991)) provides not only a clear residue numbering system applicable to antibody variable regions, but also residue boundaries defining the three CDRs. Those CDRs may be referred to as Kabat CDRs. Each complementarity determining region may comprise amino acid residues from a "complementarity determining region" as defined by Kabat. Chothia et al. (Chothia & Lesk, J. Mol. Biol, 196: 901-917 (1987) and Chothia et al., Nature 342: 877-883 (-1989)) have found that certain sub-portions within the Kabat CDRs adopt almost identical peptide backbone conformation, although with large diversity at the amino acid sequence level. Those sub-portions are referred to as L1, L2 and L3, or H1, H2 and H3, respectively, where "L" and "H" represent the light and heavy chain regions, respectively. Those regions may be referred to as Chothia CDRs, which have boundaries that overlap with those of Kabat CDRs. There are some other CDRs whose boundaries may not be defined strictly following one of the above systems, but will still overlap with those of the Kabat CDRs. CDRs defined according to any of these systems may be used in the methods used herein, although CDRs defined by Kabat or Chothia are used in preferred embodiments. "Antibody variable region" refers to the portion of the light and heavy chains of an antibody molecule that includes the amino acid sequences of the complementarity determining regions (CDRs, i.e., CDR1, CDR2 and CDR3) and the framework regions (FRs). VH refers to the variable domain of the heavy chain. VL refers to the variable domain of the light chain.

The term "diagnosis" or "diagnosing" described herein refers to the determination of whether a patient has suffered from, is suffering from, or will suffer from a disease or condition in the past, at the time of diagnosis, or in the future, or the determination of the progression or likely progression in the future of a disease, or the assessment of a patient's response to a therapy. The term "treatment" or "treating" described herein refers to slowing, interrupting, arresting, controlling, stopping, alleviating, or reversing the progression or severity of a sign, symptom, disorder, condition or disease, but does not necessarily involve the complete elimination of all disease-related signs, symptoms, conditions or disorders, and refers to therapeutic intervention that ameliorates the signs, symptoms, and the like of a disease or pathological state after the disease has begun to progress.

The term "effective amount" described herein refers to an amount or dose of the STAR, the STAR complex, the CAR, the CAR-T, the STAR-T, the immune cell, the pharmaceutical composition, etc., described herein which provides the desired treatment or prevention after administration to a patient or organ in a single dose or multiple doses.

The term "product" described herein may be a kit, a chip, an antibody conjugate, a multifunctional antibody, a pharmaceutical composition, etc.

The term "individual" or "subject" described herein may be a human or a non-human animal, and the non-human animal may be a non-human mammal such as a mouse, a cow, a sheep, a rabbit, a pig, or a monkey, etc.

The term "and/or" described herein encompasses all combinations of the items connected by the term, and each combination should be deemed to have been individually listed herein. For example, "A and/or B" encompasses "A", "A and B", and "B". For another example, "A, B, and/or C" encompasses "A", "B", "C", "A and B", "A and C", "B and C", and "A and B and C". The term "comprises" or "comprising" described herein is an open-ended expression. When the term is used to describe a sequence of a protein or a nucleic acid, the protein or the nucleic acid may consist of the sequence, or may have additional amino acids or nucleotides at one or both ends of the protein or the nucleic acid, but still possess the activity described herein. Furthermore, it is clear to those skilled in the art that methionine encoded by a start codon at the N-terminus of a polypeptide may be retained in certain practical cases (e.g., upon expression in a particular expression system), but does not substantially affect the function of the polypeptide. Therefore, when specific polypeptide amino acid sequences are described in the specification and claims of the present application, although these sequences may not include methionine encoded by the start codon at the N-terminus, sequences including such methionine are also encompassed, and accordingly, the encoding nucleotide sequences may include the start codon; and vice versa.

### BRIEF DESCRIPTION OF THE DRAWINGS

Hereinafter, examples of the present disclosure will be described in detail with reference to the accompanying drawings, in which:
FIG. 1A- FIG. 1B shows expression level detection results of LILRB4 in different AML cell lines;
FIG. 2 shows a schematic structure of a STAR targeting LILRB4;
FIG. 3 shows killing effects of different antibodies on target cells;
FIG. 4 A- FIG. 4B shows affinity results of NLB4/NLB 14 nanobodies detected by SPR;
FIG. 5 A- FIG. 5B shows competitive binding results of NLB4/NLB14 nanobodies detected by SPR;
FIG. 6 shows a schematic structure of the LILRB4 protein;
FIG. 7 shows epitopes of LILRB4 recognized by NLB4/NLB14 nanobodies;
FIG. 8 shows flow cytometry graphs for detecting whether NLB4/NLB14 nanobodies non-specifically bind to various proteins of the LILRB4 family;
FIG. 9 A- FIG. 9B shows flow cytometry graphs for detecting whether NLB4/NLB14 nanobodies non-specifically bind to different tissues of a human body;
FIG. 10 shows flow cytometry graphs for detecting the binding of NLB4/NLB 14 nanobodies to human LILRB4 and mouse LILRB4;
FIG. 11 shows schematic structures of single-epitope and dual-epitope STARs targeting LILRB4;
FIG. 12 shows flow cytometry graphs for verifying expression-on-membrane of single-epitope and dual-epitope LILRB4 STARs;
FIG. 13 shows recognition and killing effects of LILRB4 STAR-T cells on the target cell line THP1;
FIG. 14 A- FIG. 14B shows the killing effect of LILRB4 STAR-T cells on target cells;
FIG. 15 shows cytokine secretion levels of LILRB4 STAR-T;
FIG. 16 A- FIG. 16D shows the killing effect of LILRB4 STAR-T in a mouse tumor model;
FIG. 17 shows HE staining results of different tissues in mice after LILRB4 STAR-T reinfusion;
FIG. 18 shows results of *in vitro* tumorigenicity detection of LILRB4 STAR-T cells;
FIG. 19 A- FIG. 19C shows the relationship between NLB4/NLB 14 and LILRB4 ligand ApoE.

### DETAILED DESCRIPTION

The technical solutions in the examples of the present disclosure will be described clearly and completely below with reference to the drawings. It is apparent that the described examples are only a part of the examples of the present disclosure, but not all of them. Based on the examples of the present disclosure, all other examples obtained by those of ordinary skill in the art without creative work shall fall within the protection scope of the present disclosure.

### Experimental materials and methods

### Vector construction

The lentiviral vector and lentiviral packaging plasmid used in the examples of the present application were purchased from commercial companies or synthesized by commercial companies. The gene fragments used in the examples of the present application, including signal peptides, antibody-binding regions, hinge regions, TCR constant regions, tag proteins, etc., were all synthesized by commercial companies. One or more target fragments were linked by PCR with synthetic primers to obtain corresponding functional sequences. The lentiviral vector used in the present disclosure is pHAGE-hEF1α-RFP. The pHAGE-hEF1A-WPRE-AMP vector was obtained using restriction endonuclease SpeI/SalI, the gene fragments were obtained by synthesis and PCR method, and the complete vector was obtained by the homologous recombination method under the action of a recombinase.

The α chain constant region was derived from mice, and the wild type was named TRAC. The β chain constant region was derived from mice, and the wild type was named TRBC. The co-stimulatory molecule is OX40.

### Lentiviral packaging

Lentix-293T cells were inoculated into 10-cm culture dishes at 5 × 10⁵ cells/mL, cultured in a 5% CO₂ incubator at 37 °C, and transfected when the cell density reached about 80% (observed under a microscope). The four plasmids were mixed well with 500 µL of serum-free DMEM at a ratio of PMD2.G:REV:PMDLG:transfer plamid = 1:1:2:4. 54 µL of PEI-max was mixed well with 500 µL of serum-free DMEM, and the mixture was left to stand at room temperature for 5 min (the volume-to-massratio of PEI-Max to plasmid was 3:1). The PEI-max mixed solution was slowly added to the plasmid mixed solution, gently pipetted, mixed well, and left to stand at room temperature for 15 min. The final mixed solution was slowly added to the culture medium, mixed well, placed back into the incubator for culture for 12-16 h, and changed to 6% FBS DMEM culture medium for further culture. The virus solution was collected at 48 h and 72 h.

### Culture and infection of T cells

### 1) Culture method for Jurkat T cell line

The Jurkat T cell line was cultured in an RPMI 1640 culture medium containing 10% FBS. The culture density was 3 × 10⁵ cells/mL, and the maximum density was not more than 3 × 10⁶ cells/mL. The cells were passaged every 1-2 days. The required amount of cells was taken after the cells were counted, the culture medium was supplemented to adjust the density to the above density, and the cells were cultured in a CO₂ incubator.

### 2) Infection method for Jurkat T cell line

The cells were counted, and the cells at 1 × 10⁶ cells/mL were taken, subjected to buffer exchange by centrifugation, resuspended in 1 mL of an RPMI 1640 culture medium containing 10% FBS, and added to a 24-well plate. The virus solution (MOI = 1) was added, and the mixture was centrifuged at 1500 rpm for 90 min and cultured in a CO₂ incubator. After 12 h of infection, the culture medium was completely exchanged to a fresh RPMI 1640 culture medium containing 10% FBS, and the positive rate was detected at 72 h.

### 3) Culture method for human primary T cells

After the primary T cells were obtained by the Ficoll isolation method, they were cultured in an X-VIVO culture medium containing 10% FBS and 100 IU/mL IL-2 at an initial culture density of 1 × 10⁶ cell/mL and added to a well plate pre-coated with CD3 and RetroNectin (with final concentrations of 5 µg/mL). During the later stage, the culture density was 5 × 10⁵ cells/mL, and the maximum density was not more than 3 × 10⁶ cells/mL. The cells were passaged every 1-2 days.

### 4) Infection method for human primary T cells

After the primary T cells were cultured for 48 h, the virus solution (MOI = 20) was added, and the mixture was centrifuged at 1500 rpm for 90 min, and cultured in a CO₂ incubator. After 24 h of infection, an X-VIVO culture medium containing 10% FBS and 100 IU/mL IL-2 was supplemented, and the cells were transferred to another well. The infection efficiency was detected using tag proteins or antibodies at 72 h.

### 5) Detection method for infection efficiency

After 72 h of infection, the cells were pipetted and mixed well, and counted. The cells at 5 × 10⁵ cells/mL were taken and centrifuged, and the supernatant was discarded. The staining solution was PBS + 2% FBS + 2 mM EDTA, and the corresponding antibody was added and incubated for 30 min. The mixture was washed twice with PBS and detected on a machine.

### Assay for in vitro function of STAR-T cells

Positive T cells were co-cultured with target cells expressing Luciferase (Raji cells, CD19-KO Raji cells, or CD22-KO Raji cells) at a specified effector-to-target ratio.

After 24 h of co-culture, the cell suspension was gently pipetted and mixed well. The cell suspension was added to a white 96-well plate at 150 µL/well, each with 2 replicate wells, and the luciferase substrate was added. The mixture was shaken (at a low speed) and co-incubated for 10 min, and the chemiluminescence value was measured by using a multifunctional microplate reader. The cell killing was calculated as follows: killing efficiency = 100% - (well value of effector cell - well value of target cell/well value of control cell - well value of target cell). Alternatively, after 24 h of co-culture, the cell culture supernatant was collected, and the cytokine content was measured by a commercial kit.

### Assay for in vivo function of STAR-T cells

The model was constructed using NSG immunodeficient mice. The mouse genotype is NOD-Prkdcem26Il2rgem26/Nju, lacks T cells, B cells, and NK cells, and is also deficient in macrophages and dendritic cells. In this experiment, female NSG mice aged 6-8 weeks were used, and the weight difference of the mice in each batch of experiments was controlled within 2 g. The mice were housed in specific pathogen-free (SPF) separated ventilated cages and provided with a normal diet and drinking water with an acidic pH to prevent pathogen contamination.

Human Burkitt's lymphoma Raji cells were used to construct a tumor model by xenografting. The Raji cell is a cell strain expressing the luciferase gene through lentiviral vectors, and the development and changes of Raji tumors are monitored in real time in mice by fluorescein chemiluminescence and *in vivo* imaging. In this model, 1 × 10⁶ to 3 × 10⁶ Raji-luciferase cells were inoculated to female NSG mice aged 6-8 weeks through tail vein reinfusion. A fluorescein potassium salt solution was intraperitoneally injected into mice, and the fluorescence signals of the tumor cells *in vivo* were detected by *in vivo* imaging.

### Example 1. Construction of Target Cells

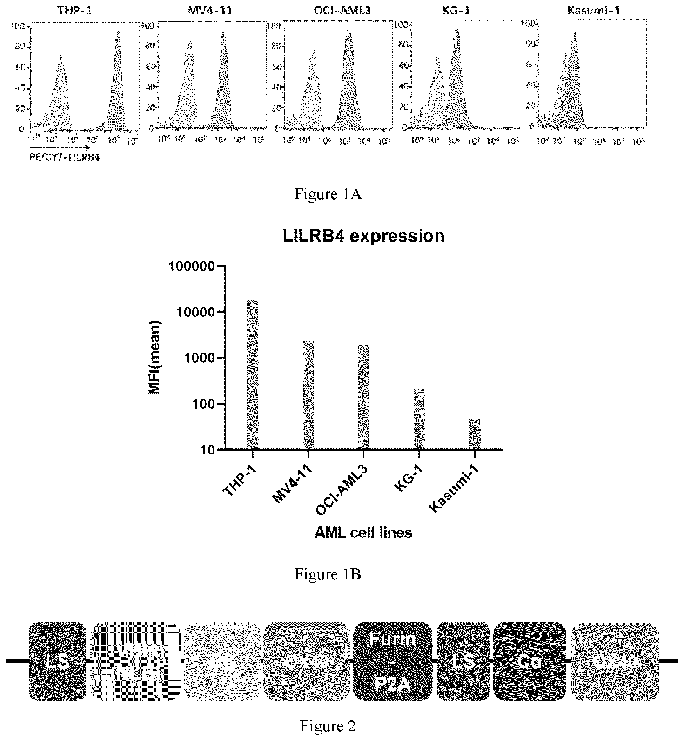

In order to screen nanobodies targeting LILRB4 and to detect the killing effect of STAR-T on AML tumor cells, the inventors detected LILRB4 antigen expression in various AML cells by flow cytometry. The results are shown in FIGs. 1A and 1B. The LILRB4 antigen expression was relatively high in THP1, MV4-11, and OCI-AML3, while the LILRB4 antigen expression was relatively low in KASUMI-1 cells with a myeloblast morphology.

### Example 2. Screening of Nanobodies Targeting LILRB4

### 1. Alpaca immunization by human LILRB4 protein

Healthy alpacas were immunized with the extracellular domain of the commercially available human LILRB4 protein (100 µg). The adjuvants include complete Freund's adjuvant (CFA, Sigma) and incomplete Freund's adjuvant (IFA, Sigma). The extracellular domain of the expressed and purified human LILRB4 protein was diluted with PBS, and subsequently, mixed with the corresponding adjuvant at a ratio of 1:1. The antigen and the adjuvant were completely mixed to form a stable emulsifier, and the antigen mixture was extracted by a syringe and injected subcutaneously at multiple points under the skin of the alpaca neck, with 100-200 µL of the mixture injected at each point. The specific procedures for immunizing animals are as follows:
1) Day 1 (1st immunization): the alpaca was immunized by subcutaneous injection with an LILRB4 antigen (100 µg) mixed with complete Freund's adjuvant (CFA);
2) Day 14 (2nd immunization): the alpaca was immunized by subcutaneous injection with an LILRB4 antigen (100 µg) mixed with incomplete Freund's adjuvant (IFA);
3) Day 28 (3rd immunization): the alpaca was immunized again by subcutaneous injection with the LILRB4 antigen (100 µg) mixed with incomplete Freund's adjuvant (IFA);
4) Day 42 (1st serum collection): an alpaca blood sample was collected from the alpaca ear vein, the serum was extracted, and antibody titer detection was performed, with the PIN value of the 200,000-fold diluted serum being more than 2;
5) Day 42 (4th immunization): the alpaca was immunized again by subcutaneous injection with the LILRB4 antigen (100 µg) mixed with incomplete Freund's adjuvant (IFA) to enhance the immune effect;
6) Day 53 (2nd serum collection): an alpaca blood sample was collected from the alpaca ear vein, the serum was extracted, and antibody titer detection was performed, with the P/N value of the 200,000-fold diluted serum being more than 2; and
7) Days 54, 57, and 60: 30-40 mL of alpaca blood samples were collected from the hind leg vein of the alpaca, and the PBMC isolation was performed.

### 2. PBMC isolation

1) PBMCs were isolated in a biosafety cabinet; the blood in the anticoagulation tube was combined into a 50 mL centrifuge tube (30 mL); PBS was added to a total volume of 50 mL; and the mixture was gently mixed.
2) A Ficoll isolation solution was added to another new 50 mL centrifuge tube at 15 mL/tube; the blood sample was layered on top of the Ficol solution at 25 mL/tube; the operation process should be stable; and the Ficoll and the blood were layered to prevent mixing.
3) The centrifuge acceleration and deceleration rates were set to be 0 at RT, with a centrifugal force of 800 g, and the centrifugation was performed for 30 min; after the centrifugation was completed, the sample was taken out of the centrifuge and layered as follows: upper aqueous phase-buffy coat layer-Ficoll layer-erythrocyte layer, with PBMCs in the buffy coat layer; and the buffy coat layer was pipetted and transferred to a new 50 mL centrifuge tube.
4) PBS was added to the sample tube to 50 mL; the mixture was mixed well and centrifuged at 2000 rpm at RT for 5 min; the supernatant was discarded; and 5 mL of PBS was added to resuspend the cell pellet.
5) Step 4 was repeated, and the mixture was cooled to 4 °C.
6) 5 mL of PBS was added to resuspend the cell pellet; PBS was supplemented to 40 mL; and the cells were counted.
7) The mixture was centrifuged at 1500 rpm at 4 °C for 5 min, and the supernatant was discarded; 1 mL of PBS was added, and the cells were resuspended and pipetted and mixed well; 20 mL of Trizol was added, and the mixture was mixed well and left to stand at room temperature for 5 min; the cells were lysed and aliquoted into 1.5 mL RNase-free EP tubes at 1 mL/tube; and the cells were cryopreserved at -80 °C for RNA extraction.

### 3. RNA extraction and reverse transcription

1) The sample was taken out of the -80 °C refrigerator and thawed at room temperature; 200 µL of chloroform was added; the mixture was left to stand at room temperature for 3 min; the sample was centrifuged at 12000 g at 4 °C for 15 min, and the sample was separated into an upper aqueous phase, a middle layer, and an organic layer; the upper layer was transferred to a new RNase-free tube; isopropanol was added at a ratio of 1 µL glycogen to 500 µL isopropanol; and the mixture was left to stand at 4 °C overnight.
2) The sample was centrifuged at 12000 g at 4 °C for 20 min, and the supernatant was discarded; 1 mL of pre-cooled 75% alcohol was added to wash the precipitate; the mixture was centrifuged again to remove alcohol and dried; and 15 µL of RNase-free water was added to each tube to dissolve the RNA precipitate, and reverse transcription was performed.
3) cDNA (20 µL system) was synthesized using a Promega reverse transcription kit.

Step 1: a certain template RNA was taken, and Oligo (dT) was added, as shown in Table 1;

**Table 1**

| Component | Volume (µL) |
|---|---|
| RNA(<5µg/reaction) | 11 |
| Oligo(dT) 15 Primer | 1 |
| Total volume | 12 |

Step 2: the mixture of the template RNA and Oligo (dT) was pre-denatured at 65 °C for 5 min and placed back on ice after the pre-denaturation was completed.

Step 3: during the pre-denaturation, RT-Mix could be prepared in advance at 8 µL/tube, and the components and the volumes are shown in Table 2.

**Table 2**

| Component | Volume (µL) |
|---|---|
| 5 ×Reaction Buffer | 4 µL |
| RiboLockRnaseInhibitor(20 U/µL) | 1 µL |
| 10mM dNTP Mix | 2 µL |
| RevertAid M-MuLVRT(200 U/µL) | 1 µL |
| Total volume | 8 µL |

Step 4: a reverse transcription program, including extension and reverse transcriptase inactivation, was set up; and after the program was completed, cDNA was obtained.

### 4. Construction of phage library

### 1) VHH sequence obtained by PCR

The VHH sequence was obtained by two rounds of PCR, and homology arms of the vector were added at both ends of the sequence.

### 2) First round of PCR

Step 1: the reaction system was prepared as shown in Table 3.

**Table 3**

| Component | Volume (µL) |
|---|---|
| cDNA | 1-2 |
| Primer-F | 1 |
| Primer-R | 1 |
| PrimeSTAR Max DNA polymerase | 15 |
| H₂O | 11-12 |
| Total volume | 30 |

Step 2: the PCR conditions are shown in Table 4.

**Table 4**

| Temperature (°C) | Time | Action | Cycle |
|---|---|---|---|
| 94 °C | 5 min | Pre-denaturation | 1 |
| 98 °C | 15 s | Denaturation | 32× |
| 56 °C | 20 s | Annealing | |
| 72 °C | 45 s | Extension | |
| 72 °C | 5 min | Final extension | 1 |
| 4 °C | 10 min | | 1 |

The PCR product was subjected to gel electrophoresis, and the target band at 0.7 kb was cut out for product recovery.

### 3) Second round of PCR

Step 1: the reaction system was prepared as shown in Table 5.

**Table 5**

| Component | Volume (µL) |
|---|---|
| Template, a product from the first round of PCR | 1-3(60 ng) |
| VHH-F | 1 |
| VHH-R | 1 |
| PrimeSTAR Max DNA polymerase | 15 |
| H₂O | 10-12 |
| Total volume | 30 |

Step 2: the PCR conditions are shown in Table 6.

**Table 6**

| Temperature (°C) | Time | Action | Cycle |
|---|---|---|---|
| 94 °C | 5 min | Pre-denaturation | 1 |
| 98 °C | 15 s | Denaturation | 17× |
| 56 °C | 20 s | Annealing | |
| 72 °C | 30 s | Extension | |
| 72 °C | 5 min | Final extension | 1 |
| 4 °C | 10 min | | 1 |

The PCR product was subjected to gel electrophoresis, and the target band at 400 bp was cut out for product recovery.

### 4) Vector PCR

A vector region of the phagemid was obtained by PCR for the expression of the VHH sequence. Step 1: the reaction system was prepared as shown in Table 7.

**Table 7**

| Component | Volume (µL) |
|---|---|
| pADL plasmid | 1(10 ng) |
| pADL-Vector-F | 1 |
| pADL-Vector-R | 1 |
| PrimeSTAR Max DNA polymerase | 15 |
| H₂O | 10-12 |
| Total volume | 30 |

Step 2: the PCR conditions are shown in Table 8.

**Table 8**

| Temperature (°C) | Time | Action | Cycle |
|---|---|---|---|
| 94 °C | 5 min | Pre-denaturation | 1 |
| 98 °C | 15 s | Denaturation | 34× |
| 56 °C | 20 s | Annealing | |
| 72 °C | 3 min | Extension | |
| 72 °C | 5 min | Final extension | 1 |
| 4 °C | 10 min | | 1 |

The PCR product was subjected to gel electrophoresis, and the target band at 4000 bp was cut out for product recovery.

### 5) Ligation as well as purification and concentration of ligation product

The VHH fragment was ligated to the vector of the phagemid, and subsequently, the ligation product was concentrated.

Step 1: the reaction system was prepared as shown in Table 9.

**Table 9**

| Component | Amount |
|---|---|
| NEBuilder HiFi DNA Assembly Master Mix | 10 µL |
| VHH fragment | 0.133 pmols |
| Vector fragment | 0.067 pmols |
| H₂O | Balance |
| Total volume | 20 |

Step 2: the above mixture was incubated at 50 °C for 2 h and cooled on ice.

Step 3: the ligation product was purified to remove components such as salt ions and proteins from the ligation system, and the volume was decreased to 1/10 of the original volume.

### 5. Electroporation and library construction

1) One tube of *E. coli* competent cells was thawed on ice.
2) 2 µL of a ligation product or a positive control was added to the above competent cells and gently pipetted and mixed well; and the mixture was left to stand on ice for 1-2 min, transferred to a pre-cooled electroporation cuvette, and electroporation was performed.
3) After the electroporation, 1 mL of 2YT-G was immediately added at 37 °C, and the electroporation cuvette was washed by using a pipette tip; the electroporated bacterial solution was transferred to a 15 mL centrifuge tube or a 2 mL EP tube and thawed in a water bath at 37 °C until all the samples were electroporated (2YT-G: 2× YT culture medium containing 2% glucose); and the tube was transferred to a shaker at 220 rpm at 37 °C, and the bacterial solution was thawed for 1 h.
4) 5 µL of the above bacterial solution was pipetted and diluted 10^2-fold to 10^5-fold; and a 2YT-A (a 2YT plate containing 100 µg/mL ampicillin) plate was coated with the bacterial solution and incubated in an incubator at 37 °C overnight for colony counting.
5) The remaining bacterial solution was inoculated into a 2YT-AG culture medium, shaken to the logarithmic phase, and infected with a helper phage, and the mixture was shaken at 220 rpm at 30 °C for 12-16 h (2YT-AG: 2× YT culture medium containing 2% glucose and 100 µg/mL ampicillin).
6) Phages were collected and concentrated, and the titer was determined.

### 6. Phage library antibody screening

Three rounds of antibody screening were performed on the phage library obtained in step 7, each round of screening including one positive selection and one negative selection. The phages were incubated with an antigen peptide; unbound phages were discarded, and phages bound to the antigen peptide remained; subsequently, the phages were incubated with BSA for negative selection; and phages not bound to BSA remained.
1) Plate coating: the antigen was diluted with PBS to a concentration of 2 ng/µL and added to a 96-well plate at 100 µL/well; 2% BSA was prepared with PBS and added to the corresponding negative selection wells at 100 µL/well; and the plate was sealed with a preservative film and incubated at 4 °C overnight.
2) The coating solution was discarded, 200 µL of a washing solution (washing solution: 1% Tween 20/PBS, pH 7.4) was added, and the plate was washed 3 times.
3) Blocking: a 2% BSA blocking solution was added to all wells at 100 µL/well, and the plate was sealed with a preservative film and incubated at 37 °C for 1 h.
4) The supernatant was discarded, 200 µL of the washing solution was added, and the plate was washed 3 times.
5) The phages were added to positive selection wells at 1 × 10¹² phages/well and diluted to 100 µL in volume, and the plate was sealed with a preservative film and incubated at 37 °C for 1 h.
6) The supernatant was discarded, 200 µL of the washing solution was added, and the plate was washed 10 times.
7) Elution-neutralization: 200 µL of an eluent was added to the positive selection wells, and the mixtures were neutralized to pH 7-7.4.
8) Negative selection: the above eluates were added to the negative selection wells; the plate was sealed with a preservative film and incubated at 37 °C for 1 h; and the supernatant was pipetted and retained, and the titer detection was performed.
9) A small amount of phages after one round of panning were diluted and applied to a 2× YT-A plate, and the plate was incubated at 37 °C overnight; the next day, colony counting was performed, and the titer was calculated; and single clones were picked for sequencing, and sequence diversity and enrichment were analyzed.
10) The remaining phages were all used for TG1 infection.
11) M13KO7 infection: the phages were diluted, M13KO7 was added to the phage solution, and the mixture was incubated in a water bath at 37 °C for 30 min; the culture medium was replaced by a 2× YT-AK culture medium, and the mixture was shaken at 220 rpm at 30 °C for 14-16 h.
12) The phages were concentrated, the phage titer was detected, and subsequently, the next round of screening could be performed.

The coated antigen amount in the second round of screening and the third round of screening was reduced, the washing times were increased after the phages were incubated in the positive wells, and other steps were the same as the above steps.

### 7. Binding assay and sequence acquisition

TG1 was infected with the phages obtained from the three rounds of screening and M13KO7 helper phage and applied to a 2YT-AK plate; single clones were picked for phage amplification; and the phages were collected for binding assay to determine available phages/antibodies.
1) Plate coating: the antigen was diluted with a coating solution to 1 ng/µL at 100 µL/well; 2% BSA was added to control negative wells; and the plate was sealed with a preservative film and incubated at 4 °C overnight.
2) The coating solution in the plate was discarded, 200-250 µL of the washing solution was added, and the plate was washed 3 times.
3) 200 µL of 2% BSA was added to all wells, and the mixture was blocked at room temperature for 1 h.
4) The blocking solution in the plate was discarded, 200 µL of the washing solution was added, and the plate was washed once.
5) 100 µL of the phage was added to each of the positive and negative wells, and the plate was incubated at 37 °C for 1 h.
6) The phages in the plate were discarded, 200 µL of the washing solution was added, and the plate was washed 3 times.
7) An anti-M13-HRP antibody was diluted and added at 50 ng/well, and the plate was incubated at room temperature for 1 h.
8) The antibodies in the plate were discarded, 200 µL of the washing solution was added, and the plate was washed 5 times.
9) 100 µL of TMB chromogenic solution was added to each well, and the mixture was reacted at room temperature until the OD value was between 2 and 3.
10) A stopping solution was added to the color development system at 50 µL/well.
11) The absorbance value at 450 nm was measured using a spectrophotometer; and the monoclonal bacterial solution corresponding to the positive well was set for sequencing, and the VHH sequence was determined.
12) The obtained antibodies were named NLB1-NLB23.

### 8. Functional screening at cellular level

1) A STAR structure targeting LILRB4 was constructed according to the structure shown in FIG. 2. The positive antibody heavy chain (VHH) sequence obtained from the previous screening was assembled with the STAR molecule constant region and inserted into a lentiviral vector by homologous recombination to construct a complete STAR plasmid.
2) Virus packaging
   Lentix-293T cells were inoculated into 10-cm culture dishes at 5 × 10⁵ cells/mL, cultured in a 5% CO₂ incubator at 37 °C, and transfected when the cell density reached about 80% (observed under a microscope). The four plasmids were mixed well with 500 µL of serum-free DMEM at a ratio of PMD2.G:PRSV-Rev:PMDlg:transfer plamid = 1:1:2:4. 54 µL of PEI-max was mixed well with 500 µL of serum-free DMEM, and the mixture was left to stand at room temperature for 5 min (the volume-to-mass ratio of PEI-Max to plasmid was 3:1). The PEI-max mixed solution was slowly added to the plasmid mixed solution, gently pipetted, mixed well, and left to stand at room temperature for 15 min. The final mixed solution was slowly added to the culture medium, mixed well, placed back into the incubator for culture for 12-16 h, and changed to 6% FBS DMEM culture medium for further culture. The virus solution was collected at 48 h and 72 h.
3) Viral titer measurement
   TCR-knockout Jurkat-C4 cells were inoculated into a flat-bottom 96-well plate at 1.5 × 10⁵ cells/mL, 100 µL of a 1640 culture medium containing 10% FBS and 0.2 µL of 1000× polybrene was added to each well. The virus was 10-fold serially diluted with a 1640 complete culture medium. The diluted cells were added to virus wells at 100 µL/well, mixed, centrifuged at 1500 rpm at 32 °C for 90 min, and cultured in a 5% CO₂ incubator at 37 °C; after 72 h, the infection efficiency was measured by using a flow cytometer; and wells with an infection rate of 2-30% were selected when the titer was calculated, with the calculation formula being as follows: titer (TU/mL) = 1.5 × 10^4 × positive rate/virus volume (µL) × 1000. T cells were infected with the above viruses, and the STAR was expressed.
4) Isolation, activation, and infection of human primary T cells
   After the primary T cells were obtained by the Ficoil isolation method, they were cultured in an X-VIVO culture medium containing 10% FBS and 100 IU/mL IL-2 at an initial culture density of 1 × 10⁶ cells/mL and added to a well plate pre-coated with CD3, CD28, and Fibronectin for activation. After 24 h of activation, the virus solution was added, and the mixture was centrifuged at 1500 rpm for 90 min and cultured in a CO₂ incubator. After 24 h of infection, the X-VIVO culture medium containing 10% FBS and 100 IU/mL IL-2 was supplemented, and the cells were transferred to another well. During the later stage, the cells were passaged every 1-2 days.
5) Co-culture of T cells and target cells *in vitro* to determine killing efficiency
   Target cells THP1 or 293T cells overexpressing LILRB4 were plated in a 24-well plate at a density of 1E5 cells/well one day in advance and cultured overnight; the corresponding number of STAR-T cells were added to the target cells at a ratio of STAR positive T cells to target cells of 0.5:1; and the killing effect of the STAR-T cells on the target cells after 24 h or 48 h of culture was detected.

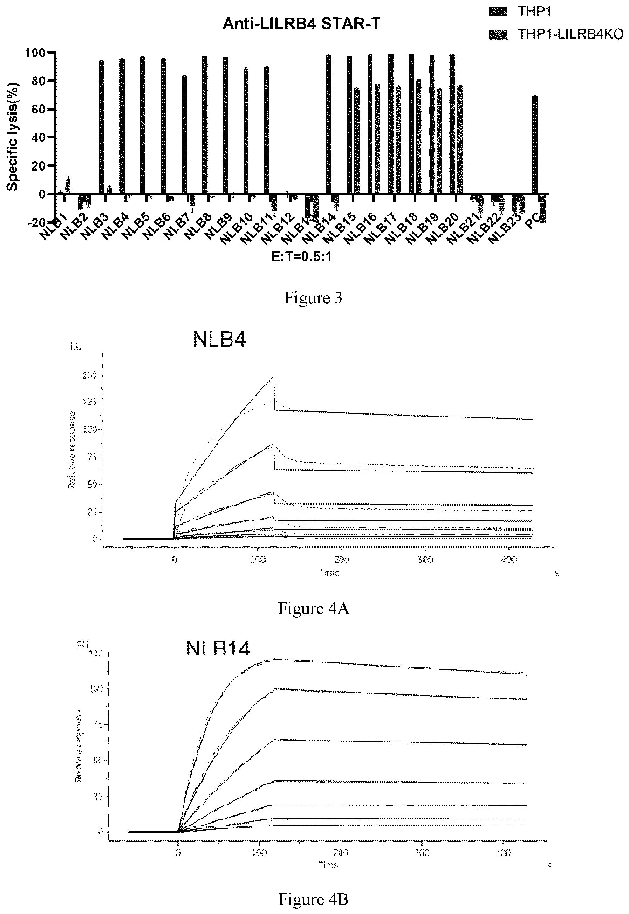

The results are shown in FIG. 3. NLB3, 4, 5, 6, 7, 8, 9, 10, 11, 14, and PC specifically recognized LILRB4 target cells; NLB1, 2, 12, 13, 21, 22, and 23 did not recognize LILRB4; and NLB15, 16, 17, 18, 19, and 20 exhibited non-specific recognition and killing effects on LILRB4 negative cells.

### Example 3. Antibody Affinity Assay (SPR)

The affinity of the antibody was detected by the surface plasmon resonance (SPR) technique. A mouse anti-human IgG (Fc) antibody was immobilized on the surface of a CM5 chip through amino coupling, a VHH antibody fused with human IgG1 (Fc) was injected into the capture experimental channels (Fc2), and the reference channels (Fc1) did not involve ligand capture. The LILRB4 protein was 2-fold serially diluted to obtain a concentration gradient of 62.5 nM, 31.25 nM, 15.625 nM, 7.813 nM, 3.906 nM, 1.953 nM, and 0.977 nM, and the diluted proteins were injected into the experimental channels and the reference channels sequentially for binding and dissociation for the corresponding period of time. The KD values of the samples were calculated using Biacore 8K analysis software, and the reference channels (Fc1) were used for background subtraction. The results are shown in Table 1 and FIG. 4. NLB4 and NLB14 had affinities of 1.32 nM and 0.493 nM, respectively, for the LILRB4 protein.

**Table 1. Affinity assay data for NLB4 and NLB14**

| Method | Ligand | Capture Level (RU) | Analyte | Analyte Conc. | ka (1/Ms) | kd(1/s) | KD(M) | Rmax (RU) | Chi² (RU²) | Fit model |
|---|---|---|---|---|---|---|---|---|---|---|
| Human IgG(Fc) Capture | NLB4 | 206.6 | Pro-LB | 0.977-62.5nM | 4.18E+05 | 5.51E-04 | 1.32E-09 | 184.7 | 17.18 | 1:1 binding |
| Human IgG(Fc) Capture | NLB14 | 244.5 | Pro-LB | 0.977-62.5nM | 6.12E+05 | 3.01E-04 | 4.93E-10 | 124.2 | 0.57 | 1:1 binding |

### Example 4. Competitive Binding Assay of Nanobodies (SPR)

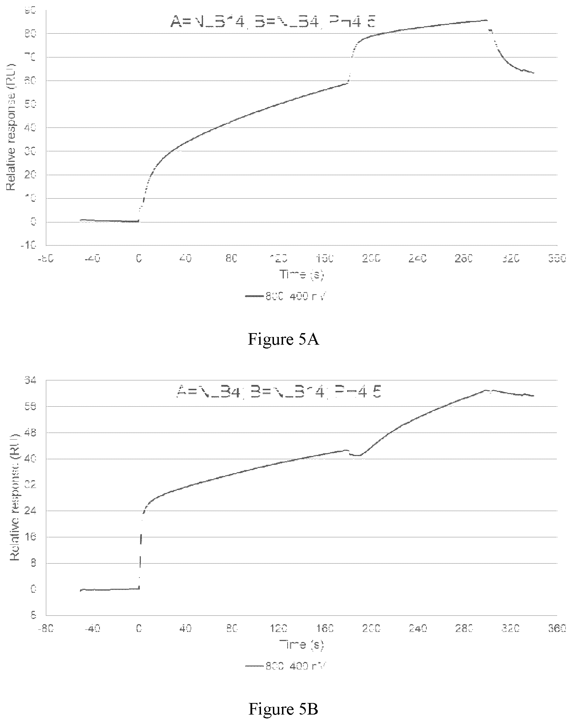

The competitive binding of the antibody was detected by the surface plasmon resonance (SPR) technique. The stationary phase was the LILRB4 protein, and the mobile phase was NLB(G4S)IGG1FC. The results are shown in FIGs. 5A and 5B. NLB4 did not compete with NLB14 for binding to LILRB4.

### Example 5. Epitopes of LILRB4 Recognized by NLB4/NLB14 Nanobodies

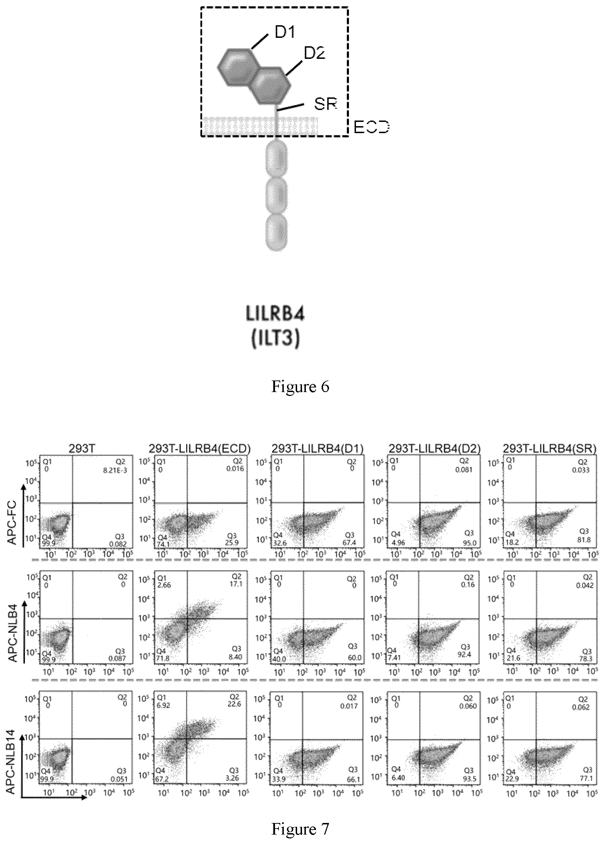

The structure of LILRB4 is shown in FIG. 6. The epitopes of LILRB4 recognized by NLB4/NLB14 nanobodies were detected by flow cytometry. The results are shown in FIG. 7. NLB4 or NLB14 was capable of recognizing the full-length extracellular domain of LILRB4, but did not bind to individual Ig domain or stock region.

### Example 6. Assay for Antibody Binding Specificity (SPR)

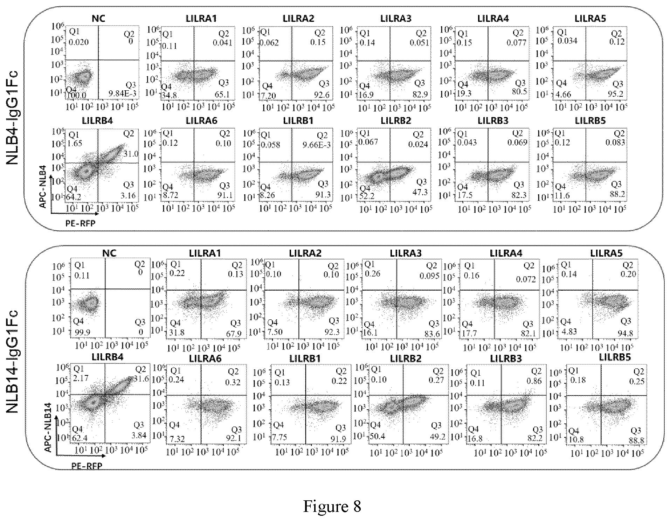

In order to detect whether the antibody had non-specific binding to other proteins of the LILRB4 family (LILRA1, LILRA2, LILRA3, LILRA4, LILRA5, LILRA6, LILRB1, LILRB2, LILRB3, LILRB4, and LILRB5), in this experiment, a 293T cell line expressing proteins of the same family was first constructed through lentivirus infection, and these proteins were co-expressed with RFP. The RFP positive rate was detected by flow cytometry to determine the infection efficiency, and LILRB4 antibody staining was used to detect whether the antibody had non-specific binding to the homologous family proteins. The results are shown in FIG. 8, which indicate that two nanobodies NLB4 and NLB14 of LILRB4 significantly bind to LILRB4 and not to other proteins of the same family.

### Example 7. LILRB4 Safety Evaluation Cell Line Binding Assay

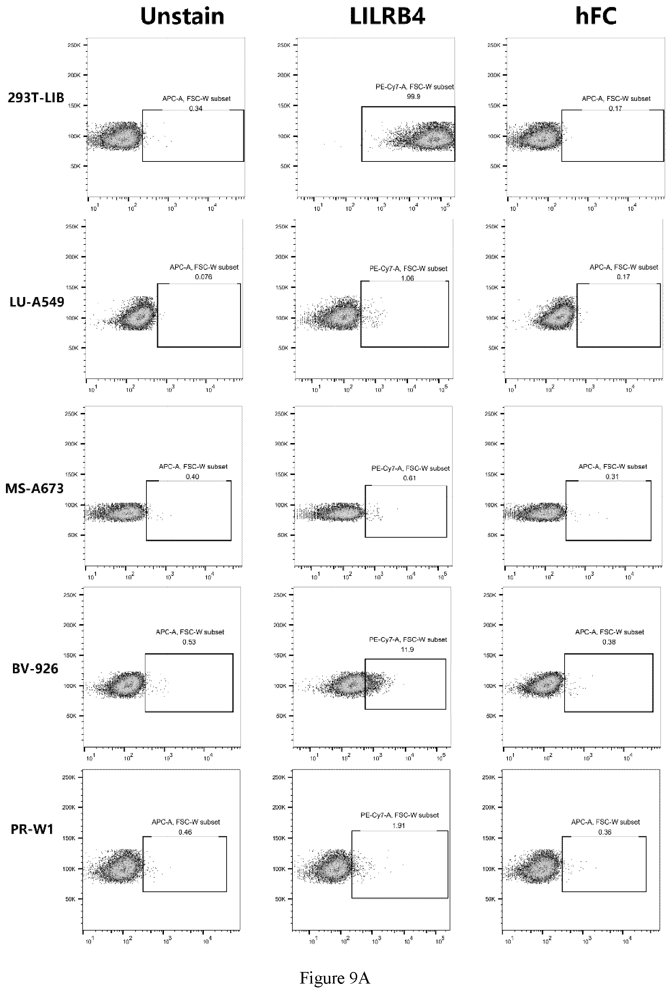

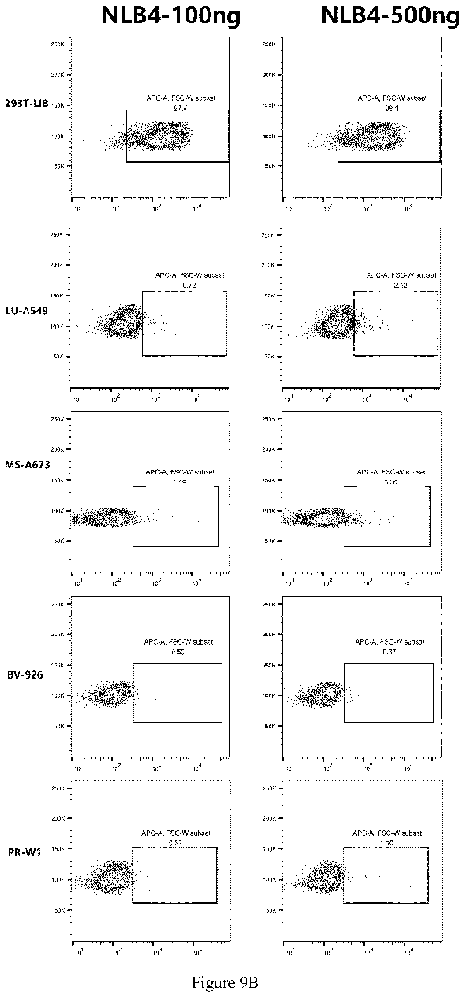

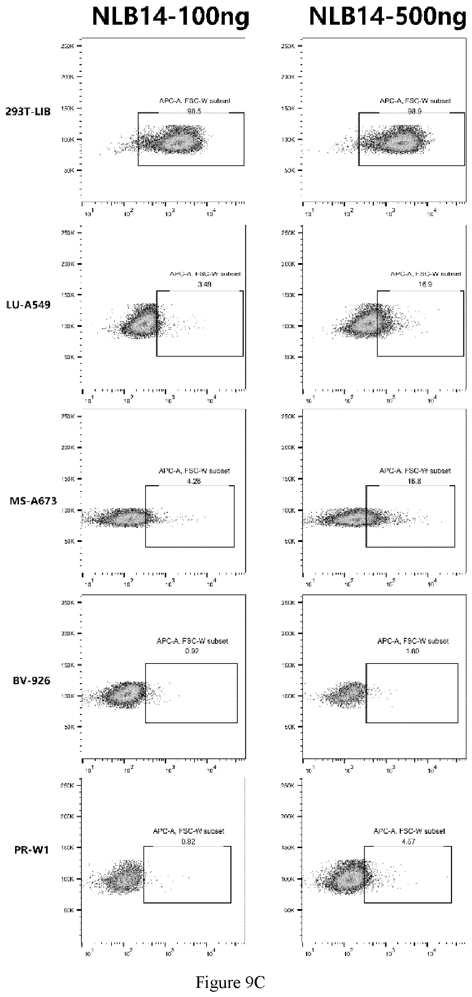

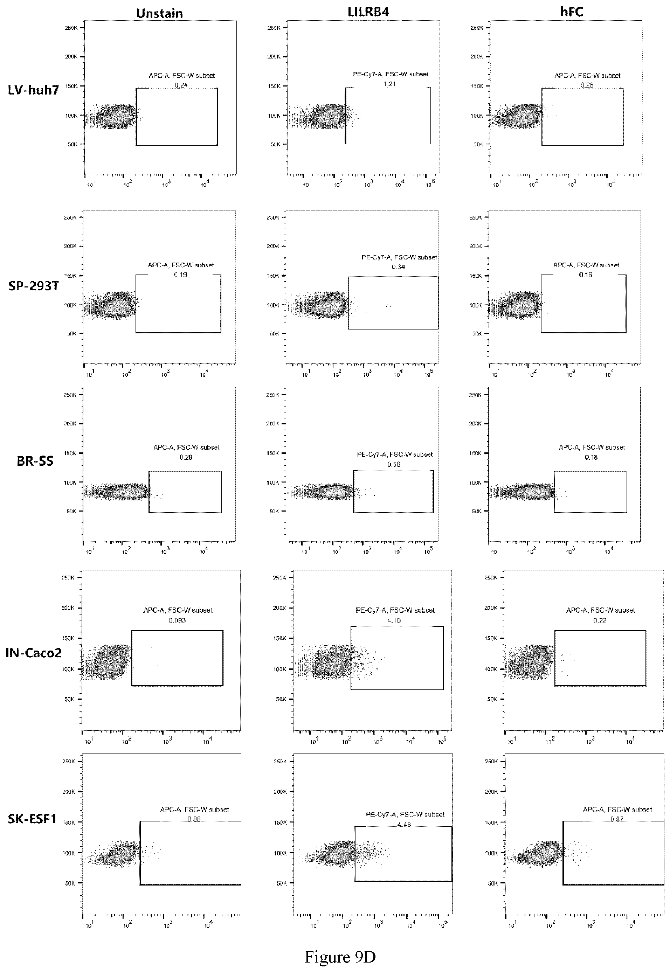

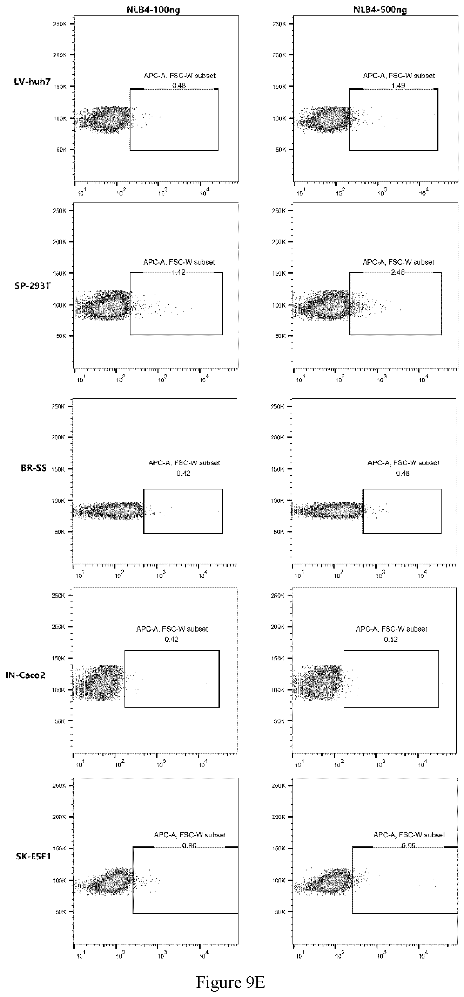

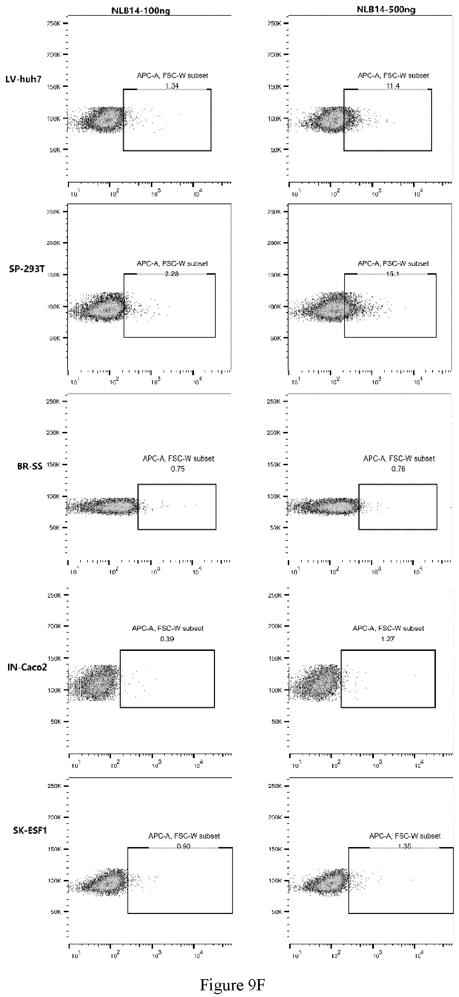

In order to detect the accuracy of target recognition by the two antibodies NLB4 and NLB14 and whether the two antibodies had non-specific binding to other components of human tissues, a safety evaluation cell bank that was derived from different tissues (lung, muscle, blood vessel, prostate, liver, kidney, brain, intestinal tract, and skin) of a human body and was composed of 9 cell lines was established in this experiment, and the safety of the antibodies was preliminarily evaluated by flow cytometry. The NLB4 and NLB14 antibodies fused with FC (Human IGG1FC) tags were incubated with the safety evaluation cell line for binding, followed by labeling with an anti-FC fluorescent secondary antibody and flow cytometry. The results are shown in FIGs. 9A and 9B. Both NLB4 and NLB14 did not have non-specific binding to 9 tissue cell lines.

### Example 8. Species Specificity Evaluation of NLB4 and NLB14 Nanobodies-Mice

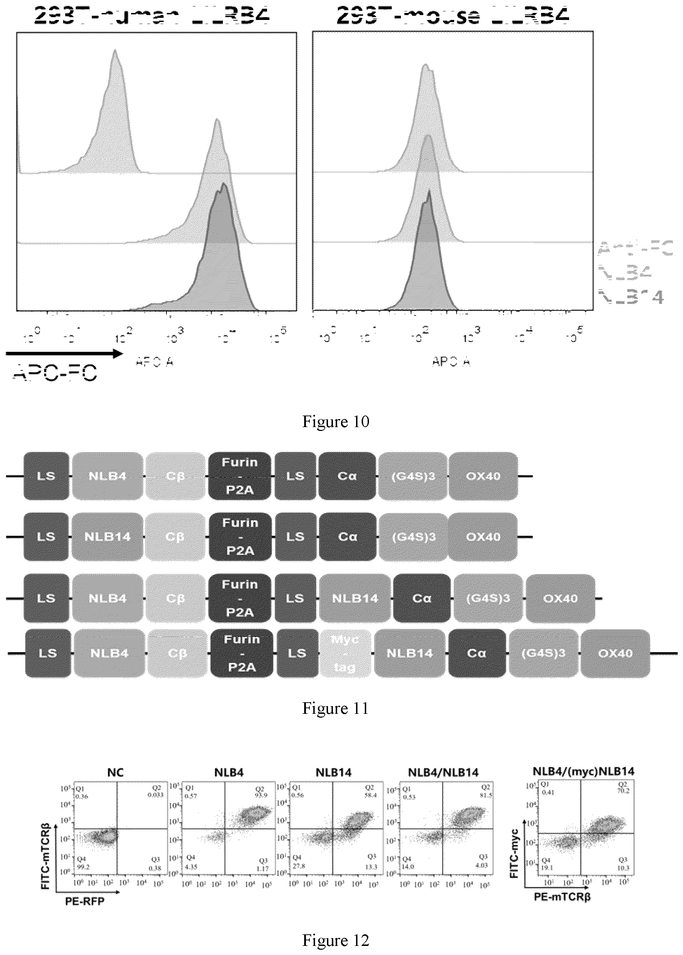

In this example, 293T-human LILRB4 and 293T-mouse LILRB4 were established, and the species specificity of the two antibodies NLB4 and NLB14 was detected by flow cytometry. The results are shown in FIG. 10. NLB4 and NLB14 antibodies recognized only the human LILRB4, and did not recognize the mouse LILRB4.

### Example 9. Single-Epitope and Dual-Epitope LILRB4 STAR Structures and Expression-on-Membrane

In order to detect the expression-on-membrane, *in vitro* killing effects, and *in vivo* killing effects of single-epitope and dual-epitope LILRB4 STARs, STAR structures targeting LILRB4 were constructed as shown in FIG. 11, in which the amino acid sequence of NLB4 STAR is set forth in SEQ ID NO: 39, the amino acid sequence of NLB14 STAR is set forth in SEQ ID NO: 40, the amino acid sequence of NLB4/NLB14 STAR is set forth in SEQ ID NO: 41, and the amino acid sequence of NLB4/(myc)NLB14 STAR is set forth in SEQ ID NO: 42.

For STAR-T cells, the primary T cells were infected with lentiviruses and detected for their expression-on-membrane by flow staining technique. The RFP fluorescent protein was co-expressed at the terminus of the STAR by IRES, and the expression-on-membrane of the dual-epitope STARs was determined through the expression efficiencies of the anti-mouse TCRβ chain antibody and RFP. The experimental results are shown in FIG. 12. The STAR had high infection efficiency, and the mouse TCRβ chain and RFP exhibited double-positive expression, indicating that the STAR is capable of expressing on the membrane. After verification of expression-on-membrane, the IRES-RFP sequence in the vector was removed.

### Example 10. Killing Effect and Killing Specificity of LILRB4 STAR-T Cells on Target Cell Line THP1

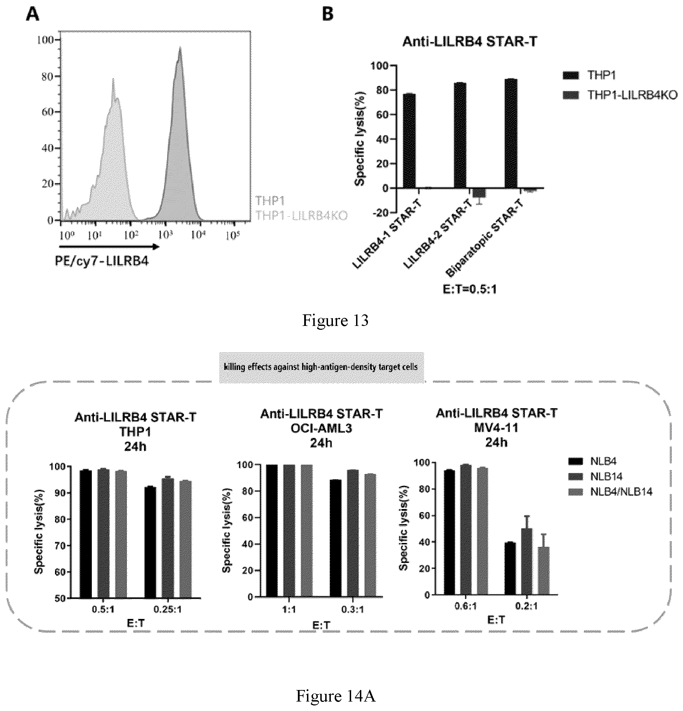

In order to verify the specific killing of LILRB4 target cells by STAR-T, the firefly luciferase was stably overexpressed in the AML cell line, THP1, based on which the LILRB4 gene in THP1 cells was knocked out by CRISPR (FIG. 13A). Nanobodies NLB4 and NLB14 capable of recognizing LILRB4 were screened through alpaca, and NLB4, NLB14, and NLB4/NLB14 were simultaneously constructed into STAR vectors to obtain LILRB4-1 STAR, LILRB4-2 STAR, and dual-epitope STAR (biparatopic STAR). The above STARs were introduced into T cells through the above lentiviral vectors to obtain LILRB4-1 STAR-T, LILRB4-2 STAR-T, and LILRB4 dual-epitope STAR-T, and the above STAR-T systems were functionally evaluated. After STAR-T was co-cultured with target cells for 24 h, a luciferase substrate was added, and the killing effect of STAR-T on the target cells was detected by using a chemiluminescence method. The experimental results are shown in FIG. 13. LILRB4-1 STAR-T, LILRB4-2 STAR-T, and dual-epitope STAR-T all had strong killing effects on THP1, but did not have killing effects on THP1-LILRB4KO cells, indicating that all the LILRB4-1 STAR-T, LILRB4-2 STAR-T, and dual-epitope STAR-T can specifically recognize and kill LILRB4 target cells.

### Example 11. Killing Effects of LILRB4 STAR-T Cells on Different AML Cell Lines

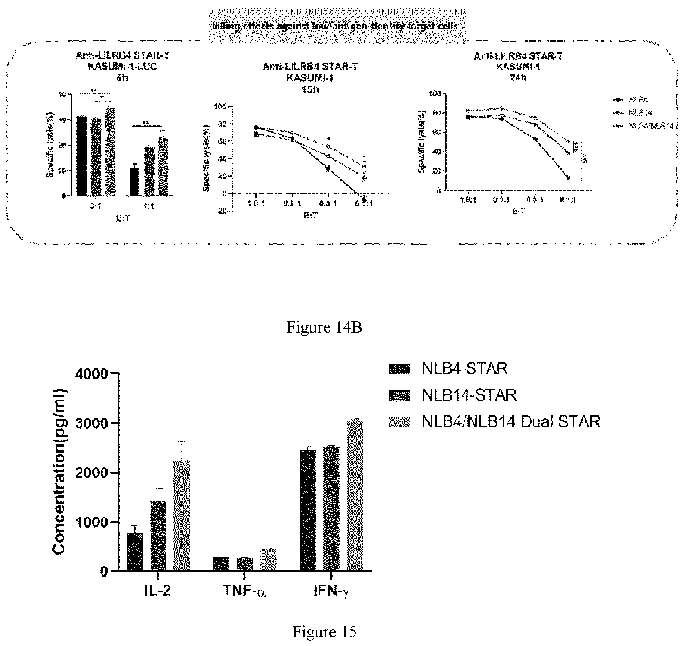

Killing assays were performed on four target cells by using two single-epitope STAR-T systems and a dual-epitope STAR-T. As shown in FIG. 14A, after co-culture for 24 h, LILRB4-1 STAR-T, LILRB4-2 STAR-T, and dual-epitope STAR-T cells all had strong killing effects against high-antigen-density AML target cell lines THP1, MV-11, and OCI-AML3; however, LILRB4 dual-epitope STAR-T had no significant advantage over the single-epitope STAR-T. As shown in FIG. 14B, after co-culture for 6 h, 15 h, and 24 h, LILRB4-1 STAR-T, LILRB4-2 STAR-T, and dual-epitope STAR-T cells also had strong killing effects on KASUMI-1 with low expression of LILRB4. In addition, the killing efficiency of the LILRB4 dual-epitope STAR-T to the low-antigen-density target cells was significantly higher than that of the single-epitope STAR-T, which indicates that the dual-epitope STAR-T is more favorable for eliminating the target cells with low expression of LILRB4. The *in vitro* killing experimental results show that the dual-epitope STAR-T can efficiently and specifically kill the LILRB4 target cells at the *in vitro* cellular level.

### Example 12. Cytokine Secretion Levels of LILRB4 STAR-T Cells

After LILRB4 STAR-T was co-cultured with MV4-11 target cells for 24 h, the culture supernatant was collected at the same time, and the cytokines IL-2, IFN-γ, and TNF-α were detected. The results are shown in FIG. 15. Whether single-epitope STAR-T or dual-epitope STAR-T, the levels of IL-2 and IFN-γ cytokines were significantly increased after co-culture with MV4-11 target cells, while the TNF-α level was relatively low. This is because TNF-α can directly stimulate macrophages, neutrophils, etc. to secrete IL-6, while IL-6 is a major cytokine of cytokine storms in cell therapy. The results suggest that the dual-epitope STAR-T may exhibit relatively good safety in clinical applications. Meanwhile, compared with LILRB4-1 STAR-T and LILRB4-2 STAR-T, the secretion levels of IL-2 and IFN-γ of the dual-epitope STAR-T were significantly increased, suggesting that the dual-epitope STAR-T may have more efficient tumor killing effect.

### Example 13. Evaluation of Killing Effect of LILRB4 STAR-T in Mouse Tumor Model

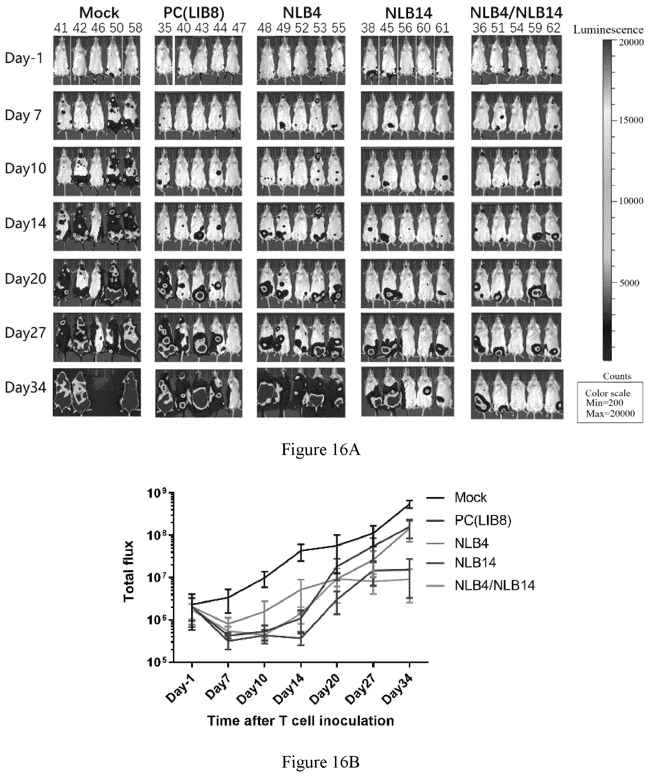

In order to verify the killing effect of STAR-T cells *in vivo* and potential safety issues, MV4-11 cells expressing luciferase were injected into NCG immunodeficient mice by intravenous infusion (tail vein infusion, 1E6 cells/mouse), and an MV4-11 cell NCG mouse tumor *in vivo* imaging model was constructed. Furthermore, in order to verify the tumor effect of dual-epitope STAR-T, the antibody scFv published on TEXAS was selected to construct a STAR-T as a positive control (PC STAR-T). On day 7 after the infusion of MV4-11 tumor cells, LILRB4-1 STAR-T cells, LILRB4-2 STAR-T cells, dual-epitope STAR-T cells, control T cells (PC STAR-T), and Mock-T cells (T cells not infected with STAR) were infused into the tail vein at a cell density of 4E6 cells/mouse, and *in vivo* imaging was performed on day 7 to detect tumor growth, twice a week during the early stage and once a week during the later stage. The results are shown in FIGs. 16A and 16B. The dual-epitope STAR-T exhibited a significant inhibitory effect on the tumor growth in the mouse tumor model, which was significantly superior to those of the PC positive control group and LILRB4-1 STAR-T and LILRB4-2 STAR-T groups.

### Example 14. Experimental Data for LILRB4 STAR-T Cell Safety

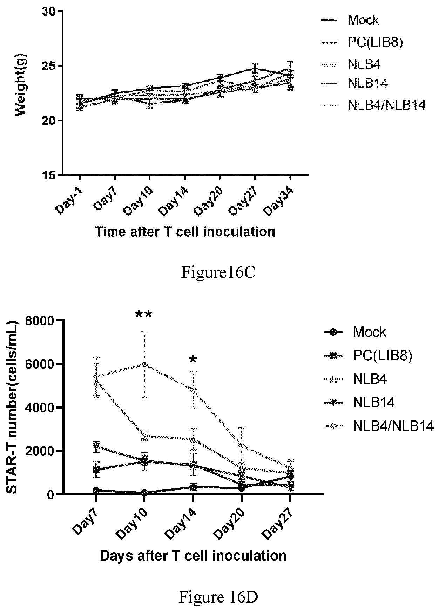

In terms of the safety of animals, before and after cell infusion, The performance (e.g., appearance, behavior, response to stimuli, excreta, etc.), death, and weight change in mice were observed. In the STAR-T cell group, the mice showed no change in appearance (e.g., hair and color), behaved normally, had no increased sensitivity to sudden stimuli, and showed no significant change in excreta (observed, not quantified) before and after STAR-T cell infusion. As shown in FIG. 16A, the results of the death data show that animals in the Mock-T group died on day 28 due to increasing tumor burden, while animals in the STAR-T groups did not die until day 34. Meanwhile, as shown in FIG. 16C, STAR-T reinfusion had no significant effect on the body weight of mice, suggesting that STAR-T has relatively good safety.

### Example 15. In Vivo Pharmacokinetic Experiment in LILRB4 STAR-T Cells

In terms of the *in vivo* metabolism of STAR-T cells, the *in vivo* amplification of STAR-T cells was first examined by collecting blood from the orbit of the mice. The results are shown in FIG. 16D. Dual-epitope STAR-T cells could exhibit rapid amplification after infusion, which was significantly higher than that of the PC control group. However, as tumor cells were killed, STAR-T cells subsequently declined and remained at a relatively low level. Furthermore, the number of the dual-epitope STAR-T cells in the peripheral blood of the mice, particularly on day 10 and day 14 after T cell reinfusion, was significantly higher than those of other groups, which indicates that the amplification effect of STAR-T in mice is better.

### Example 16. In Vivo Systemic Toxicity Experiment of LILRB4 STAR-T Cells

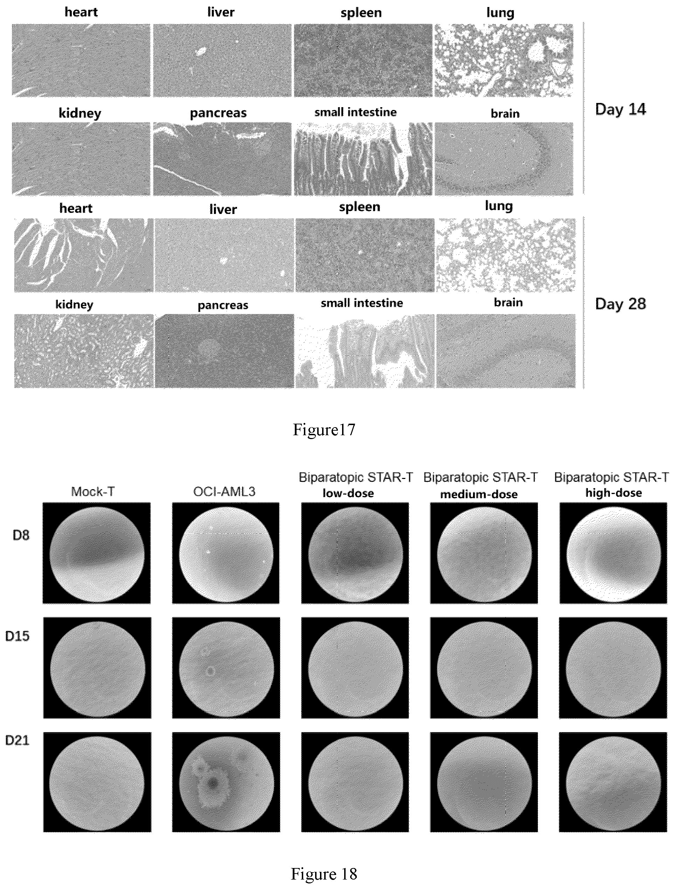

In order to further detect the safety of LILRB4 STAR-T in mice, STAR-T cells and Mock-T cells were infused in the tail vein on day 7 of reinfusion of MV4-11 cells expressing luciferase, with an effective cell amount of 4E6 cells/mouse, the heart, liver, spleen, lung, kidney, small intestine, pancreas, and brain of the mice were collected and immobilized for HE staining on day 14 and day 28 after T cell reinfusion, and the pathological changes in mouse tissues were observed. The results showed that no significant lesions were observed in mouse tissues on day 14 and day 28 after STAR-T reinfusion (FIG. 17).

### Example 17. In Vitro Tumorigenicity Detection of LILRB4 STAR-T Cells

In order to verify the tumorigenicity of LILRB4 STAR-T cells, a soft agar colony growth experiment was performed on LILRB4 STAR-T cells. LILRB4 STAR-T cells were divided into three dose groups of 0.5 × 10³ (low-dose group), 1.0 × 10³ (medium-dose group), and 2.0 × 10³ (high-dose group); AML tumor cell line OCI-AML3 with a dose of 1.0 × 10° was used as a positive control group; and Mock-T (T cells not infected with STAR, with a dose of 2.0 × 10³) was used as a negative control group. The cells of the above groups were subjected to colony growth experiments on the soft agar of 0.6% in the lower layer and 0.35% in the upper layer, and observed on day 8, day 15, and day 21 after the cells were plated. The observation results are shown in FIG. 11. OCI-AML3 tumor cells (positive control group) formed small clones on day 8 after plating and formed relatively large clones on day 21; while three doses of LILRB4 STAR-T cells and Mock-T cells did not form clones on day 21. The above results indicate that LILRB4 STAR-T failed to form clones in the soft agar colony experiment, suggesting that LILRB4 STAR-T does not have tumorigenicity *in vitro.*

### Example 18. Relationship Between NLB4/NLB14 and LILRB4 Ligand ApoE

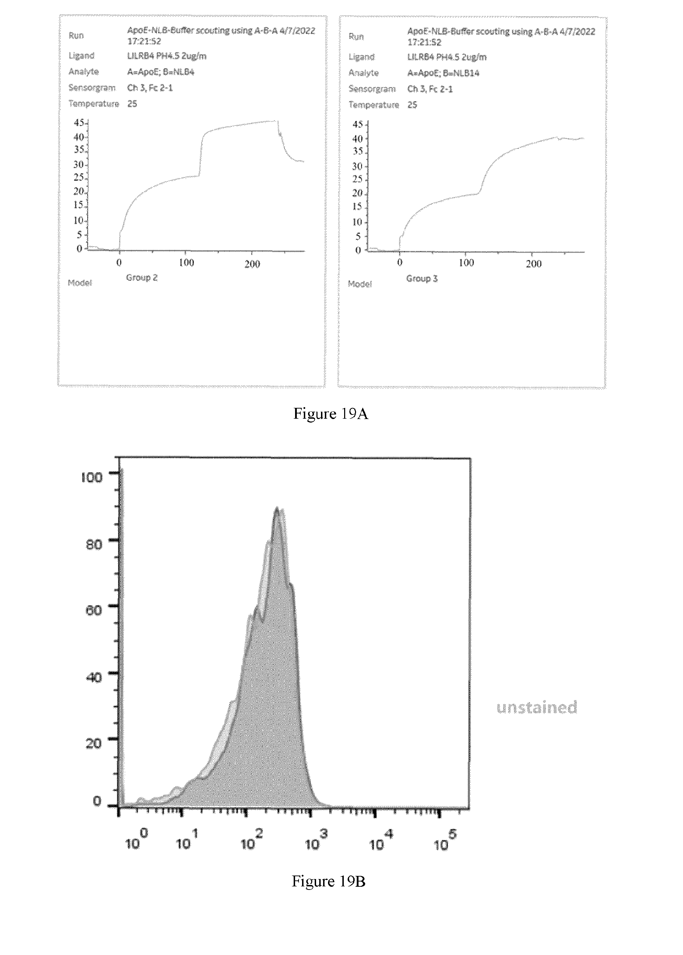

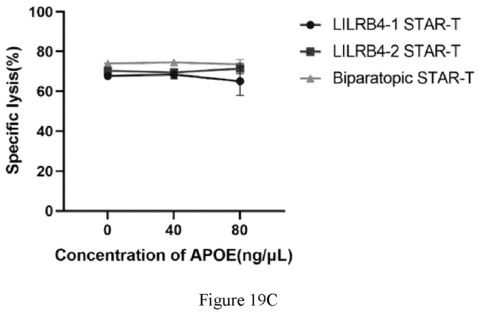

LILRB4 can bind to ApoE, support tumor cell infiltration into tissues and inhibit T cell activity in acute myeloid leukemia (AML) cells through the ApoE-LILRB4-SHP-2-uPAR-Arginase-1 signaling pathway. The competitive binding of NLB4/NLB 14 and ApoE was detected by taking LILRB4 protein as a stationary phase, ApoE as mobile phase 1, and NLB(G4S)IGG1FC as mobile phase 2, and the results are shown in FIG. 19. NLB4/NLB14 did not compete with ApoE for binding to LILRB4, and ApoE did not affect the binding of NLB4/14 to LILRB4. The target cell line KASUMI-1 was analyzed by flow cytometry, and the results are shown in FIG. 19B. The granulocyte line KASUMI-1 in the AML cell line did not express ApoE. In normal peripheral blood, the ApoE concentrations were in the range of 29 ng/µL to 70 ng/µL. Therefore, the inventors chose to detect the killing effects of single-epitope STAR-T and dual-epitope STAR-T on target cells at ApoE concentrations of 0 ng/µL, 40 ng/µL, and 80 ng/µL. The results are shown in FIG. 19C. ApoE did not inhibit the killing of target cells by LILRB4 STAR-T.

The preferred embodiments of the present disclosure are described in detail above, which, however, are not intended to limit the present disclosure. Within the scope of the technical conception of the present disclosure, various simple modifications can be made to the technical solutions of the present disclosure, all of which will fall within the protection scope of the present disclosure.

In addition, it should be noted that the various specific technical features described in the above specific embodiments can be combined in any suitable manner in which the features do not contradict each other. In order to avoid unnecessary repetition, such combinations will not be illustrated separately.

The sequences involved in the present application are as follows:
SEQ ID NO: 1, amino acid sequence of wild-type human TCRα constant region 
DIQNPDPAVYQLRDSKSSDKSVCLFTDFDSQTNVSQSKDSDVYITDKTVLDMRSMDFKS
NSAVAWSNKSDFACANAFNNSIIPEDTFFPSPESSCDVKLVEKSFETDTNLNFQNLSVIGF
RILLLKVAGFNLLMTLRLWSS*
SEQ ID NO: 2, amino acid sequence of wild-type human TCRβ constant region 
DLKNVFPPEVAVFEPSEAEISHTQKATLVCLATGFFPDHVELSWWVNGKEVHSGVSTDP
QPLKEQPALNDSRYCLSSRLRVSATFWQNPRNHFRCQVQFYGLSENDEWTQDRAKPVT
QIVSAEAWGRADCGFTSVSYQQGVLSATILYEILLGKATLYAVLVSALVLMAMVKRKD
F*
SEQ ID NO: 3, amino acid sequence of wild-type murine TCRα constant region 
DIQNPEPAVYQLKDPRSQDSTLCLFTDFDSQINVPKTMESGTFITDKTVLDMKAMDSKS
NGAIAWSNQTSFTCQDIFKETNATYPSSDVPCDATLTEKSFETDMNLNFQNLSVMGLRI
LLLKVAGFNLLMTLRLWSS
SEQ ID NO: 4, amino acid sequence of wild-type murine TCRβ constant region 
DLRNVTPPKVSLFEPSKAEIANKQKATLVCLARGFFPDHVELSWWVNGKEVHSGVSTD
PQAYKESNYSYCLSSRLRVSATFWHNPRNHFRCQVQFHGLSEEDKWPEGSPKPVTQNIS
AEAWGRADCGITSASYQQGVLSATILYEILLGKATLYAVLVSTLVVMAMVKRKNS
SEQ ID NO: 5, mouse T cell receptor α chain constant region comprising cysteine substitution (MouseTCRaC-Cys) 
DIQNPEPAVYQLKDPRSQDSTLCLFTDFDSQINVPKTMESGTFITDKCVLDMKAMDSKS
NGAIAWSNQTSFTCQDIFKETNATYPSSDVPCDATLTEKSFETDMNLNFQNLSVMGLRI
LLLKVAGFNLLMTLRLWSS
SEQ ID NO: 6, mouse T cell receptor β chain constant region comprising cysteine substitution (MouseTCRβC-Cys) 
DLRNVTPPKVSLFEPSKAEIANKQKATLVCLARGFFPDHVELSWWVNGKEVHSGVCTD
PQAYKESNYSYCLSSRLRVSATFWHNPRNHFRCQVQFHGLSEEDKWPEGSPKPVTQNIS
AEAWGRADCGITSASYQQGVLSATILYEILLGKATLYAVLVSTLVVMAMVKRKNS
SEQ ID NO: 7, mouse T cell receptor α chain constant region comprising hydrophobic amino acid substitution (mouseTCRaC-TM9) 
DIQNPEPAVYQLKDPRSQDSTLCLFTDFDSQINVPKTMESGTFITDKTVLDMKAMDSKS
NGAIAWSNQTSFTCQDIFKETNATYPSSDVPCDATLTEKSFETDMNLNFQNLLVIVLRIL
LLKVAGFNLLMTLRLWSS
SEQ ID NO: 8, mouse T cell receptor α chain constant region comprising lysine substitution in transmembrane region (mouseTCRaC-Argmut) 
DIQNPEPAVYQLKDPRSQDSTLCLFTDFDSQINVPKTMESGTFITDKTVLDMKAMDSKS
NGAIAWSNQTSFTCQDIFKETNATYPSSDVPCDATLTEKSFETDMNLNFQNLSVMGLRI
LLLRVAGFNLLMTLRLWSS
SEQ ID NO: 9, mouse T cell receptor β chain constant region comprising lysine substitution in transmembrane region and intracellular region (mouseTCRβC-Argmut), 
DLRNVTPPKVSLFEPSKAEIANKQKATLVCLARGFFPDHVELSWWVNGKEVHSGVSTD
PQAYKESNYSYCLSSRLRVSATFWHNPRNHFRCQVQFHGLSEEDKWPEGSPKPVTQNIS
AEAWGRADCGITSASYQQGVLSATILYEILLGRATLYAVLVSTLVVMAMVRRRNS
SEQ ID NO: 10, amino acid sequence of CD40 intracellular domain
   KKVAKKPTNKAPHPKQEPQEINFPDDLPGSNTAAPVQETLHGCQPVTQEDGKESRISV
SEQ ID NO: 11, amino acid sequence of OX40 intracellular domain
   RRDQRLPPDAHKPPGGGSFRTPIQEEQADAHSTLAKI
SEQ ID NO: 12, amino acid sequence of ICOS intracellular domain
   KKKYSSSVHDPNGEYMFMRAVNTAKKSRLTDVTL
SEQ ID NO: 13, amino acid sequence of CD28 intracellular domain
   RSKRSRLLHSDYMNMTPRRPGPTRKHYQPYAPPRDFAAYRS
SEQ ID NO: 14, amino acid sequence of 4-1BB intracellular domain
   KRGRKKLLYIFKQPFMRPVQTTQEEDGCSCRFPEEEEGGCEL
SEQ ID NO: 15, amino acid sequence of CD27 intracellular domain
   QRRKYRSNKGESPVEPAEPCRYSCPREEEGSTIPIQEDYRKPEPACSP
SEQ ID NO: 16, mouse T cell receptor α chain constant region comprising intracellular region deletion, cysteine substitution, and hydrophobic region engineering (mouseTCRαC-delmut) 
DIQNPEPAVYQLKDPRSQDSTLCLFTDFDSQINVPKTMESGTFITDKCVLDMKAMDSKS
NGAIAWSNQTSFTCQDIFKETNATYPSSDVPCDATLTEKSFETDMNLNFQNLLVIVLRIL
LLKVAGFNLLMTLRLW
SEQ ID NO: 17, mouse T cell receptor β chain constant region comprising intracellular region deletion and cysteine substitution (mouseTCRβC-delmut) 
DLRNVTPPKVSLFEPSKAEIANKQKATLVCLARGFFPDHVELSWWVNGKEVHSGVCTD
PQAYKESNYSYCLSSRLRVSATFWHNPRNHFRCQVQFHGLSEEDKWPEGSPKPVTQNIS
AEAWGRADCGITSASYQQGVLSATILYEILLGKATLYAVLVSTLVVMAM
SEQ ID NO: 18, amino acid sequence of human CD3y 
MEQGKGLAVLILAIILLQGTLAQSIKGNHLVKVYDYQEDGSVLLTCDAEAKNITWFKD
GKMIGFLTEDKKKWNLGSNAKDPRGMYQCKGSQNKSKPLQVYYRMCQNCIELNAATI
SGFLFAEIVSIFVLAVGVYFIAGQDGVRQSRASDKQTLLPNDQLYQPLKDREDDQYSHL
QGNQLRRN
SEQ ID NO: 19, amino acid sequence of human CD3δ 
MEHSTFLSGLVLATLLSQVSPFKIPIEELEDRVFVNCNTSITWVEGTVGTLLSDITRLDLG
KRILDPRGIYRCNGTDIYKDKESTVQVHYRMCQSCVELDPATVAGIIVTDVIATLLLALG
VFCFAGHETGRLSGAADTQALLRNDQVYQPLRDRDDAQYSHLGGNWARNK
SEQ ID NO: 20, amino acid sequence of human CD3ε 
MQSGTHWRVLGLCLLSVGVWGQDGNEEMGGITQTPYKVSISGTTVILTCPQYPGSEIL
WQHNDKNIGGDEDDKNIGSDEDHLSLKEFSELEQSGYYVCYPRGSKPEDANFYLYLRA
RVCENCMEMDVMSVATIVIVDICITGGLLLLVYYWSKNRKAKAKPVTRGAGAGGRQR
GQNKERPPPVPNPDYEPIRKGQRDLYSGLNQRRI
SEQ ID NO: 21, amino acid sequence of human CD3ζ 
MKWKALFTAAILQAQLPITEAQSFGLLDPKLCYLLDGILFIYGVILTALFLRVKFSRSAD
APAYQQGQNQLYNELNLGRREEYDVLDKRRGRDPEMGGKPRRKNPQEGLYNELQKD
KMAEAYSEIGMKGERRRGKGHDGLYQGLSTATKDTYDALHMQALPPR
SEQ ID NO: 22, amino acid sequence of human IL-2β receptor intracellular terminus 
NCRNTGPWLKKVLKCNTPDPSKFFSQLSSEHGGDVQKWLSSPFPSSSFSPGGLAPEISPL
EVLERDKVTQLLPLNTDAYLSLQELQGQDPTHLV
SEQ ID NO: 23, amino acid sequence of human IL-7α receptor intracellular terminus 
KKRIKPIVWPSLPDHKKTLEHLCKKPRKNLNVSFNPESFLDCQIHRVDDIQARDEVEGFL
QDTFPQQLEESEKQRLGGDVQSPNCPSEDVVITPESFGRDSSLTCLAGNVSACDAPILSSS
RSLDCRESGKNGPHVYQDLLLSLGTTNSTLPPPFSLQSGILTLNPVAQGQPILTSLGSNQE
EAYVTMSSFYQNQ
SEQ ID NO: 24, amino acid sequence of human IL-21 receptor intracellular terminus 
SLKTHPLWRLWKKIWAVPSPERFFMPLYKGCSGDFKKWVGAPFTGSSLELGPWSPEVP
STLEVYSCHPPRSPAKRLQLTELQEPAELVESDGVPKPSFWPTAQNSGGSAYSEERDRP
YGLVSIDTVTVLDAEGPCTWPCSCEDDGYPALDLDAGLEPSPGLEDPLLDAGTTVLSCG
 
CVSAGSPGLGGPLGSLLDRLKPPLADGEDWAGGLPWGGRSPGGVSESEAGSPLAGLDM
DTFDSGFVGSDCSSPVECDFTSPGDEGPPRSYLRQWVVIPPPLSSPGPQAS
SEQ ID NO: 25, amino acid sequence of human STAT5 activation module
   YRHQ
SEQ ID NO: 26, amino acid sequence of human IL-2β receptor intracellular terminus and human STAT5 activation module IL-2RbQ 
NCRNTGPWLKKVLKCNTPDPSKFFSQLSSEHGGDVQKWLSSPFPSSSFSPGGLAPEISPL
EVLERDKVTQLLPLNTDAYLSLQELQGQDPTHLVGGGGSYRHQ
SEQ ID NO: 27, amino acid sequence of human IL-7α receptor intracellular terminus and human STAT5 activation module IL-7RbQ 
KKRIKPIVWPSLPDHKKTLEHLCKKPRKNLNVSFNPESFLDCQIHRVDDIQARDEVEGFL
QDTFPQQLEESEKQRLGGDVQSPNCPSEDVVITPESFGRDSSLTCLAGNVSACDAPILSSS
RSLDCRESGKNGPHVYQDLLLSLGTTNSTLPPPFSLQSGILTLNPVAQGQPILTSLGSNQE
EAYVTMSSFYQNQGGGGSYRHQ
SEQ ID NO: 28, VHH amino acid sequence of LILRB4 antibody NLB4 
DVQLQESGGGLVQSGGSLRLSCLASGTSGNVKAVGWYGWYRQAPGKQREVVATITRG
GIPNYADSVQGRFTISRDNAQDTVFLQMNSLKPADTAVYYCYARILTDDWHDLWGQG
TQVTVSS
SEQ ID NO: 29, VHH amino acid sequence of LILRB4 antibody NLB14 
DVQLQESGGGLVQPGGSLRLSCAASGFTLDYYAIGWFRQAPGKEREGVSCVSSSDGST
YYADSVKGRFTISRDNAKNTVYLQMNSLKPEDTAVYYCAADQYSSTWTIRLTRCHFGS
WGQGTQVTVSS
SEQ ID NO: 30, amino acid sequence of mouseTCRaC-Cys-TM9 (hmctSTARTCRaC) 
DIQNPEPAVYQLKDPRSQDSTLCLFTDFDSQINVPKTMESGTFITDKCVLDMKAMDSKS
NGAIAWSNQTSFTCQDIFKETNATYPSSDVPCDATLTEKSFETDMNLNFQNLLVIVLRIL
LLKVAGFNLLMTLRLWSS
SEQ ID NO: 31, amino acid sequence of mouseTCRaC-Cys-TM9-N.Rec (NrecSTARTCRaC) 
DIQNPDPAVYQLRDDSTLCLFTDFDSQINVPKTMESGTFITDKCVLDMKAMDSKSNGAI
AWSNQTSFTCQDIFKETNATYPSSDVPCDATLTEKSFETDMNLNFQNLLVIVLRILLLKV
AGFNLLMTLRLWSS
SEQ ID NO: 32, amino acid sequence of mouse T cell receptor β chain constant region comprising N-terminal modification and cysteine substitution (MouseTCRbC-Cys-N.Rec, NrecSTARTCRbC) 
DLKNVFPPEVAVFEPSAEIATLVCLARGFFPDHVELSWWVNGKEVHSGVCTDPQAYKE
SNYSYCLSSRLRVSATFWHNPRNHFRCQVQFHGLSEEDKWPEGSPKPVTQNISAEAWG
RADCGITSASYQQGVLSATILYEILLGKATLYAVLVSTLVVMAMVKRKNS
SEQ ID NO: 33, amino acid sequence of anti-LILRB4NLB4CDR1
   GTSGNVKAVG
SEQ ID NO: 34, amino acid sequence of anti-LILRB4NLB4CDR2
   TITRGGIPN
SEQ ID NO: 35, amino acid sequence of anti-LILRB4NLB4CDR3
   RILTDDWHDL
SEQ ID NO: 36, amino acid sequence of anti-LILRB4NLB14CDR1
   GFTLDYYAIG
SEQ ID NO: 37, amino acid sequence of anti-LILRB4NLB 14CDR2
   CVSSSDGSTY
SEQ ID NO: 38, amino acid sequence of anti-LILRB4NLB14CDR3
   DQYSSTWTIRLTRCHFGS
SEQ ID NO: 39, amino acid sequence of NLB4 STAR 
MLLLVTSLLLCELPHPAFLLIPDVQLQESGGGLVQSGGSLRLSCLASGTSGNVKAVGWY
GWYRQAPGKQREVVATITRGGIPNYADSVQGRFTISRDNAQDTVFLQMNSLKPADTAV
YYCYARILTDDWHDLWGQGTQVTVSSEDLRNVTPPKVSLFEPSKAEIANKQKATLVCL
ARGFFPDHVELSWWVNGKEVHSGVCTDPQAYKESNYSYCLSSRLRVSATFWHNPRNH
FRCQVQFHGLSEEDKWPEGSPKPVTQNISAEAWGRADCGITSASYQQGVLSATILYEILL
GKATLYAVLVSTLVVMAMVKRKNSRRKRSGSGATNFSLLKQAGDVEENPGPMLLLVT
SLLLCELPHPAFLLIPDIQNPEPAVYQLKDPRSQDSTLCLFTDFDSQINVPKTMESGTFITD
KCVLDMKAMDSKSNGAIAWSNQTSFTCQDIFKETNATYPSSDVPCDATLTEKSFETDM
NLNFQNLLVIVLRILLLKVAGFNLLMTLRLWGGGGSGGGGSGGGGSRRDQRLPPDAHK
PPGGGSFRTPIQEEQADAHSTLAKI*
SEQ ID NO: 40, amino acid sequence of NLB14 STAR 
MLLLVTSLLLCELPHPAFLLIPDVQLQESGGGLVQPGGSLRLSCAASGFTLDYYAIGWFR
QAPGKEREGVSCVSSSDGSTYYADSVKGRFTISRDNAKNTVYLQMNSLKPEDTAVYYC
AADQYSSTWTIRLTRCHFGSWGQGTQVTVSSEDLRNVTPPKVSLFEPSKAEIANKQKAT
LVCLARGFFPDHVELSWWVNGKEVHSGVCTDPQAYKESNYSYCLSSRLRVSATFWHN
PRNHFRCQVQFHGLSEEDKWPEGSPKPVTQNISAEAWGRADCGITSASYQQGVLSATIL
YEILLGKATLYAVLVSTLVVMAMVKRKNSRRKRSGSGATNFSLLKQAGDVEENPGPM
LLLVTSLLLCELPHPAFLLIPDIQNPEPAVYQLKDPRSQDSTLCLFTDFDSQINVPKTMES
 
GTFITDKCVLDMKAMDSKSNGAIAWSNQTSFTCQDIFKETNATYPSSDVPCDATLTEKS
FETDMNLNFQNLLVIVLRILLLKVAGFNLLMTLRLWGGGGSGGGGSGGGGSRRDQRLP
PDAHKPPGGGSFRTPIQEEQADAHSTLAKI*
SEQ ID NO: 41, NLB4/NLB14 STAR 
MLLLVTSLLLCELPHPAFLLIPDVQLQESGGGLVQSGGSLRLSCLASGTSGNVKAVGWY
GWYRQAPGKQREVVATITRGGIPNYADSVQGRFTISRDNAQDTVFLQMNSLKPADTAV
YYCYARILTDDWHDLWGQGTQVTVSSEDLRNVTPPKVSLFEPSKAEIANKQKATLVCL
ARGFFPDHVELSWWVNGKEVHSGVCTDPQAYKESNYSYCLSSRLRVSATFWHNPRNH
FRCQVQFHGLSEEDKWPEGSPKPVTQNISAEAWGRADCGITSASYQQGVLSATILYEILL
GKATLYAVLVSTLVVMAMVKRKNSRRKRSGSGATNFSLLKQAGDVEENPGPMLLLVT
SLLLCELPHPAFLLIPDVQLQESGGGLVQPGGSLRLSCAASGFTLDYYAIGWFRQAPGKE
REGVSCVSSSDGSTYYADSVKGRFTISRDNAKNTVYLQMNSLKPEDTAVYYCAADQYS
STWTIRLTRCHFGSWGQGTQVTVSSDIQNPEPAVYQLKDPRSQDSTLCLFTDFDSQINVP
KTMESGTFITDKCVLDMKAMDSKSNGAIAWSNQTSFTCQDIFKETNATYPSSDVPCDA
TLTEKSFETDMNLNFQNLLVIVLRILLLKVAGFNLLMTLRLWGGGGSGGGGSGGGGSR
RDQRLPPDAHKPPGGGSFRTPIQEEQADAHSTLAKI*
SEQ ID NO: 42, NLB4/(myc)NLB14 STAR 
MLLLVTSLLLCELPHPAFLLIPDVQLQESGGGLVQSGGSLRLSCLASGTSGNVKAVGWY
GWYRQAPGKQREVVATITRGGIPNYADSVQGRFTISRDNAQDTVFLQMNSLKPADTAV
YYCYARILTDDWHDLWGQGTQVTVSSEDLRNVTPPKVSLFEPSKAEIANKQKATLVCL
ARGFFPDHVELSWWVNGKEVHSGVCTDPQAYKESNYSYCLSSRLRVSATFWHNPRNH
FRCQVQFHGLSEEDKWPEGSPKPVTQNISAEAWGRADCGITSASYQQGVLSATILYEILL
GKATLYAVLVSTLVVMAMVKRKNSRRKRSGSGATNFSLLKQAGDVEENPGPMLLLVT
SLLLCELPHPAFLLIPEQKLISEEDLDVQLQESGGGLVQPGGSLRLSCAASGFTLDYYAIG
WFRQAPGKEREGVSCVSSSDGSTYYADSVKGRFTISRDNAKNTVYLQMNSLKPEDTAV
YYCAADQYSSTWTIRLTRCHFGSWGQGTQVTVSSDIQNPEPAVYQLKDPRSQDSTLCLF
TDFDSQINVPKTMESGTFITDKCVLDMKAMDSKSNGAIAWSNQTSFTCQDIFKETNATY
PSSDVPCDATLTEKSFETDMNLNFQNLLVIVLRILLLKVAGFNLLMTLRLWGGGGSGGG
GSGGGGSRRDQRLPPDAHKPPGGGSFRTPIQEEQADAHSTLAKI*
SEQ ID NO: 43, mouse T cell receptor α chain constant region comprising intracellular region deletion, N-terminal modification, cysteine substitution, and hydrophobic engineering in transmembrane region 
DIQNPDPAVYQLRDDSTLCLFTDFDSQINVPKTMESGTFITDKCVLDMKAMDSKSNGAI
AWSNQTSFTCQDIFKETNATYPSSDVPCDATLTEKSFETDMNLNFQNLLVIVLRILLLKV
AGFNLLMTLRLW
SEQ ID NO: 44, mouse T cell receptor β chain constant region comprising intracellular region deletion, N-terminal modification, and cysteine substitution 
DLKNVFPPEVAVFEPSAEIATLVCLARGFFPDHVELSWWVNGKEVHSGVCTDPQAYKE
SNYSYCLSSRLRVSATFWHNPRNHFRCQVQFHGLSEEDKWPEGSPKPVTQNISAEAWG
RADCGITSASYQQGVLSATILYEILLGKATLYAVLVSTLVVMAM
SEQ ID NO: 45, amino acid sequence of wild-type human TCRγ chain constant region: 
DKQLDADVSPKPTIFLPSIAETKLQKAGTYLCLLEKFFPDVIKIHWQEKKSNTILGSQEG
NTMKTNDTYMKFSWLTVPEKSLDKEHRCIVRHENNKNGVDQEIIFPPIKTDVITMDPKD
NCSKDANDTLLLQLTNTSAYYMYLLLLLKSVVYFAIITCCLLRRTAFCCNGEKS
SEQ ID NO: 46, amino acid sequence of wild-type murine TCRγ chain constant region: 
XKRLDADISPKPTIFLPSVAETNLHKTGTYLCLLEKFFPDVIRVYWKEKDGNTILDSQEG
DTLKTNDTYMKFSWLTVPERAMGKEHRCIVKHENNKGGADQEIFFPSIKKVAVSTKPT
TCWQDKNDVLQLQFTITSAYYTYLLLLLKSVIYLAIISFSLLRRTSVCGNEKKS
SEQ ID NO: 47, amino acid sequence of wild-type human TCRδ chain constant region: 
XSQPHTKPSVFVMKNGTNVACLVKEFYPKDIRINLVSSKKITEFDPAIVISPSGKYNAVK
LGKYEDSNSVTCSVQHDNKTVHSTDFEVKTDSTDHVKPKETENTKQPSKSCHKPKAIV
HTEKVNMMSLTVLGLRMLFAKTVAVNFLLTAKLFFL*
SEQ ID NO: 48, amino acid sequence of wild-type murine TCRδ chain constant region: 
XSQPPAKPSVFIMKNGTNVACLVKDFYPKEVTISLRSSKKIVEFDPAIVISPSGKYSAVKL
GQYGDSNSVTCSVQHNSETVHSTDFEPYANSFNNEKLPEPENDTQISEPCYGPRVTVHT
EKVNMMSLTVLGLRLLFAKTIAINFLLTVKLFF*
SEQ ID NO: 49, amino acid sequence of EAAAK linker
   EAAAK
SEQ ID NO: 50, amino acid sequence of (EAAAK)2 linker
   EAAAKEAAAK
SEQ ID NO: 51, amino acid sequence of (EAAAK)3 linker
   EAAAKEAAAKEAAAK
SEQ ID NO: 52, amino acid sequence of EAAK linker
   EAAK
SEQ ID NO: 53, amino acid sequence of (EAAK)2 linker
   EAAKEAAK
SEQ ID NO: 54, amino acid sequence of (EAAK)3 linker
   EAAAKEAAKEAAK
SEQ ID NO: 55, amino acid sequence of A2(EAAAK)2A linker
   AAEAAAKEAAAKA
SEQ ID NO: 56, amino acid sequence of A3(EAAAK)3A linker
   AAAEAAAKEAAAKEAAAKA
SEQ ID NO: 57, amino acid sequence of A4(EAAAK)4A linker
   AAAAEAAAKEAAAKEAAAKEAAAKEAAAKA
SEQ ID NO: 58, amino acid sequence of A5(EAAAK)5A linker
   amino acid sequence of AAAAAEAAAKEAAAKEAAAKEAAAKEAAAKEAAAKA linker
SEQ ID NO: 59, amino acid sequence of A(EAAAK)4ALEA(EAAAK)4A linker
   AEAAAKEAAAKEAAAKEAAAKALEAEAAAKEAAAKEAAAKEAAAKA
SEQ ID NO: 60, amino acid sequence of G4S2 linker
   GGSGGS
SEQ ID NO: 61, amino acid sequence of (G4S2)2 linker
   GGSGGSGGSGGS
SEQ ID NO: 62, amino acid sequence of (G4S2)3 linker
   GGSGGSGGSGGSGGSGGS
SEQ ID NO: 63, amino acid sequence of (G4S2)4 linker
   GGSGGSGGSGGSGGSGGSGGSGGS
SEQ ID NO: 64, amino acid sequence of (G4S2)5 linker
   GGSGGSGGSGGSGGSGGSGGSGGSGGSGGS
SEQ ID NO: 65, amino acid sequence of (G4S2)6 linker
   GGSGGSGGSGGSGGSGGSGGSGGSGGSGGSGGSGGS
SEQ ID NO: 66, amino acid sequence of (G4S2)7 linker
   GGSGGSGGSGGSGGSGGSGGSGGSGGSGGSGGSGGSGGSGGS
SEQ ID NO: 67, amino acid sequence of (G4S2)8 linker
   GGSGGSGGSGGSGGSGGSGGSGGSGGSGGSGGSGGSGGSGGSGGSGGS
SEQ ID NO: 68, amino acid sequence of (G4S2)9 linker
   GGSGGSGGSGGSGGSGGSGGSGGSGGSGGSGGSGGSGGSGGSGGSGGSGGSGGS
SEQ ID NO: 69, amino acid sequence of (G4S2)10 linker 
GGSGGSGGSGGSGGSGGSGGSGGSGGSGGSGGSGGSGGSGGSGGSGGSG
GS
SEQ ID NO: 70, amino acid sequence of S7 linker
   SSSSSSS
SEQ ID NO: 71, amino acid sequence of G3S linker
   GGGS
SEQ ID NO: 72, amino acid sequence of (G3S)2 linker
   GGGSGGGS
SEQ ID NO: 73, amino acid sequence of (G3S)3 linker
   GGGSGGGSGGGS
SEQ ID NO: 74, amino acid sequence of (G3S)4 linker
   GGGSGGGSGGGSGGGS
SEQ ID NO: 75, amino acid sequence of (G3S)5 linker
   GGGSGGGSGGGSGGGSGGGS
SEQ ID NO: 76, amino acid sequence of (G3S)6 linker
   GGGSGGGSGGGSGGGSGGGSGGGS
SEQ ID NO: 77, amino acid sequence of (G3S)7 linker
   GGGSGGGSGGGSGGGSGGGSGGGSGGGS
SEQ ID NO: 78, amino acid sequence of (G3S)8 linker
   GGGSGGGSGGGSGGGSGGGSGGGSGGGSGGGS
SEQ ID NO: 79, amino acid sequence of (G3S)9 linker
   GGGSGGGSGGGSGGGSGGGSGGGSGGGSGGGSGGGS
SEQ ID NO: 80, amino acid sequence of (G3S)10 linker
   GGGSGGGSGGGSGGGSGGGSGGGSGGGSGGGSGGGSGGGS
SEQ ID NO: 81, amino acid sequence of G4S linker
   GGGGS
SEQ ID NO: 82, amino acid sequence of (G4S)2 linker
   GGGGSGGGGS
SEQ ID NO: 83, amino acid sequence of (G4S)3 linker
   GGGGSGGGGSGGGGS
SEQ ID NO: 84, amino acid sequence of (G4S)4 linker
   GGGGSGGGGSGGGGSGGGGS
SEQ ID NO: 85, amino acid sequence of (G4S)5 linker
   GGGGSGGGGSGGGGSGGGGSGGGGS
SEQ ID NO: 86, amino acid sequence of (G4S)6 linker
   GGGGSGGGGSGGGGSGGGGSGGGGSGGGGS
SEQ ID NO: 87, amino acid sequence of (G4S)7 linker
   GGGGSGGGGSGGGGSGGGGSGGGGSGGGGSGGGGS
SEQ ID NO: 88, amino acid sequence of (G4S)8 linker
   GGGGSGGGGSGGGGSGGGGSGGGGSGGGGSGGGGSGGGGS
SEQ ID NO: 89, amino acid sequence of (G4S)9 linker
   GGGGSGGGGSGGGGSGGGGSGGGGSGGGGSGGGGSGGGGSGGGGS
SEQ ID NO: 90, amino acid sequence of (G4S)10 linker
   GGGGSGGGGSGGGGSGGGGSGGGGSGGGGSGGGGSGGGGSGGGGSGGGGS
SEQ ID NO: 91, amino acid sequence of G2S linker
   GGS
SEQ ID NO: 92, amino acid sequence of (G2S)2 linker
   GGSGGS
SEQ ID NO: 93, amino acid sequence of (G2S)3 linker
   GGSGGSGGS
SEQ ID NO: 94, amino acid sequence of (G2S)4 linker
   GGSGGSGGSGGS
SEQ ID NO: 95, amino acid sequence of (G2S)5 linker
   GGSGGSGGSGGSGGS
SEQ ID NO: 96, amino acid sequence of (G2S)6 linker
   GGSGGSGGSGGSGGSGGS
SEQ ID NO: 97, amino acid sequence of (G2S)7 linker
   GGSGGSGGSGGSGGSGGSGGS
SEQ ID NO: 98, amino acid sequence of (G2S)8 linker
   GGSGGSGGSGGSGGSGGSGGSGGS
SEQ ID NO: 99, amino acid sequence of (G2S)9 linker
   GGSGGSGGSGGSGGSGGSGGSGGSGGS
SEQ ID NO: 100, amino acid sequence of (G2S)10 linker
   GGSGGSGGSGGSGGSGGSGGSGGSGGSGGS
SEQ ID NO: 101, amino acid sequence of GS linker
   GS
SEQ ID NO: 102, amino acid sequence of (GS)2 linker
   GSGS
SEQ ID NO: 103, amino acid sequence of (GS)3 linker
   GSGSGS
SEQ ID NO: 104, amino acid sequence of (GS)4 linker
   GSGSGSGS
SEQ ID NO: 105, amino acid sequence of (GS)5 linker
   GSGSGSGSGS
SEQ ID NO: 106, amino acid sequence of (GS)6 linker
   GSGSGSGSGSGS
SEQ ID NO: 107, amino acid sequence of (GS)7 linker
   GSGSGSGSGSGSGS
SEQ ID NO: 108, amino acid sequence of (GS)8 linker
   GSGSGSGSGSGSGSGS
SEQ ID NO: 109, amino acid sequence of (GS)9 linker
   GSGSGSGSGSGSGSGSGS
SEQ ID NO: 110, amino acid sequence of (GS)10 linker
   GSGSGSGSGSGSGSGSGSGS
SEQ ID NO: 111, amino acid sequence of (G)5 linker
   GGGGG
SEQ ID NO: 112, amino acid sequence of (A)11 linker
   AAAAAAAAAAA
SEQ ID NO: 113, amino acid sequence of F2A cleavable linker
   VKQTLNFDLLKLAGCVESNPG
SEQ ID NO: 114, amino acid sequence of P2A cleavable linker
   GSGATNFSLLKQAGDVEENPGP
SEQ ID NO: 115, amino acid sequence of T2A cleavable linker
   EGRGSLLTCGDVEENPG
SEQ ID NO: 116, amino acid sequence of E2A cleavable linker
   QCTNYALLKLAGDVESNPG
SEQ ID NO: 117, amino acid sequence of disulfide bond-type cleavable linker
   LEAGCKNFFPRSFTSCGSLE
SEQ ID NO: 118, NLB4 VHH nucleotide sequence 
GATGTGCAGCTGCAGGAGAGCGGCGGCGGACTGGTTCAATCTGGAGGCAGCCTGA
GGCTGAGCTGTCTGGCCAGCGGAACCTCCGGCAACGTGAAGGCCGTGGGCTGGTAC
GGCTGGTATAGACAGGCCCCTGGCAAGCAGCGGGAAGTGGTGGCCACAATCACCA
GAGGCGGCATCCCCAATTACGCCGATAGCGTGCAAGGCAGATTCACCATCAGCCGG
GACAACGCCCAGGACACAGTGTTCCTGCAGATGAACAGCCTGAAACCTGCTGATAC
 
CGCCGTGTACTACTGCTACGCCAGAATCCTGACAGACGACTGGCACGACCTGTGGG
GCCAGGGCACCCAGGTGACCGTGTCCAGC
SEQ ID NO: 119, NLB14 VHH nucleotide sequence 
GACGTCCAGCTGCAGGAGAGCGGCGGAGGACTGGTTCAACCTGGAGGCAGCCTGA
GACTGAGCTGTGCTGCTTCTGGCTTTACCCTGGACTACTACGCCATCGGCTGGTTCA
GGCAGGCCCCTGGCAAGGAACGGGAAGGCGTGTCCTGCGTGTCCAGCAGTGATGGC
TCTACCTACTACGCCGATAGCGTGAAGGGCAGATTCACCATCAGCAGAGACAACGC
CAAGAATACCGTGTATCTGCAGATGAACAGCCTGAAACCTGAGGACACCGCCGTGT
ACTACTGCGCCGCCGACCAGTACAGCTCTACATGGACCATCCGGCTGACAAGATGC
CACTTCGGCAGCTGGGGCCAGGGCACACAGGTGACCGTGTCTAGC
SEQ ID NO: 120, NLB4 STAR nucleotide sequence 
atgcttctcctggtgacaagccttctgctctgtgagttaccacacccagcattcctcctgatcccaGATGTGCAGCTGCAGGA
GAGCGGCGGCGGACTGGTTCAATCTGGAGGCAGCCTGAGGCTGAGCTGTCTGGCCA
GCGGAACCTCCGGCAACGTGAAGGCCGTGGGCTGGTACGGCTGGTATAGACAGGCC
CCTGGCAAGCAGCGGGAAGTGGTGGCCACAATCACCAGAGGCGGCATCCCCAATTA
CGCCGATAGCGTGCAAGGCAGATTCACCATCAGCCGGGACAACGCCCAGGACACA
GTGTTCCTGCAGATGAACAGCCTGAAACCTGCTGATACCGCCGTGTACTACTGCTAC
GCCAGAATCCTGACAGACGACTGGCACGACCTGTGGGGCCAGGGCACCCAGGTGA
CCGTGTCCAGCGAGGACCTGAGAAACGTGACCCCCCCCAAGGTGAGCCTGTTCGAG
CCCAGCAAGGCCGAGATCGCCAACAAGCAGAAGGCCACCCTGGTGTGCCTGGCCA
GAGGCTTCTTCCCCGACCACGTGGAGCTGAGCTGGTGGGTGAACGGCAAGGAGGTG
CACAGCGGCGTGTGCACCGACCCCCAGGCCTACAAGGAGAGCAACTACAGCTACTG
CCTGAGCAGCAGACTGAGAGTGAGCGCCACCTTCTGGCACAACCCCAGAAACCACT
TCAGATGCCAGGTGCAGTTCCACGGCCTGAGCGAGGAGGACAAGTGGCCCGAGGG
CAGCCCCAAGCCCGTGACCCAGAACATCAGCGCCGAGGCCTGGGGCAGAGCCGAC
TGCGGCATCACCAGCGCCAGCTACCAGCAGGGCGTGCTGAGCGCCACCATCCTGTA
CGAGATCCTGCTGGGCAAGGCCACCCTGTACGCCGTGCTGGTGAGCACCCTGGTGG
TGATGGCCATGGTGAAGAGAAAGAACAGCAGAAAGAGAAGCGGCAGCGGCG
CCACCAACTTCAGCCTGCTGAAGCAGGCCGGCGACGTGGAGGAGAACCCCGGCCCC
ATGCTGCTGCTGGTGACCAGCCTGCTGCTGTGCGAGCTGCCCCACCCCGCCTTCCTG
CTGATCCCCGACATCCAGAACCCCGAGCCCGCCGTGTACCAGCTGAAGGACCCCAG
AAGCCAGGACAGCACCCTGTGCCTGTTCACCGACTTCGACAGCCAGATCAACGTGC
CCAAGACCATGGAGAGCGGCACCTTCATCACCGACAAGTGCGTGCTGGACATGAAG
GCCATGGACAGCAAGAGCAACGGCGCCATCGCCTGGAGCAACCAGACCAGCTTCA
 
CCTGCCAGGACATCTTCAAGGAGACCAACGCCACCTACCCCAGCAGCGACGTGCCC
TGCGACGCCACCCTGACCGAGAAGAGCTTCGAGACCGACATGAACCTGAACTTCCA
GAACCTGCTGGTGATCGTGCTGAGAATCCTGCTGAAGGTGGCCGGCTTCAACC
TGCTGATGACCCTGAGACTGTGGGGTGGCGGTGGCAGCGGCGGTGGTGGTTCCGGA
GGCGGCGGTTCTAGAAGAGACCAGAGACTGCCCCCCGACGCCCACAAGCCCCCCGG
CGGCGGCAGCTTCAGAACCCCCATCCAGGAGGAGCAGGCCGACGCCCACAGCACC
CTGGCCAAGATCTGA
SEQ ID NO: 121, NLB14 STAR nucleotide sequence 
atgcttctcctggtgacaagccttctgctctgtgagttaccacacccagcattcctcctgatcccaGACGTCCAGCTGCAGGA
GAGCGGCGGAGGACTGGTTCAACCTGGAGGCAGCCTGAGACTGAGCTGTGCTGCTT
CTGGCTTTACCCTGGACTACTACGCCATCGGCTGGTTCAGGCAGGCCCCTGGCAAG
GAACGGGAAGGCGTGTCCTGCGTGTCCAGCAGTGATGGCTCTACCTACTACGCCGA
TAGCGTGAAGGGCAGATTCACCATCAGCAGAGACAACGCCAAGAATACCGTGTATC
TGCAGATGAACAGCCTGAAACCTGAGGACACCGCCGTGTACTACTGCGCCGCCGAC
CAGTACAGCTCTACATGGACCATCCGGCTGACAAGATGCCACTTCGGCAGCTGGGG
CCAGGGCACACAGGTGACCGTGTCTAGCGAGGACCTGAGAAACGTGACCCCCCCCA
AGGTGAGCCTGTTCGAGCCCAGCAAGGCCGAGATCGCCAACAAGCAGAAGGCCAC
CCTGGTGTGCCTGGCCAGAGGCTTCTTCCCCGACCACGTGGAGCTGAGCTGGTGGG
TGAACGGCAAGGAGGTGCACAGCGGCGTGTGCACCGACCCCCAGGCCTACAAGGA
GAGCAACTACAGCTACTGCCTGAGCAGCAGACTGAGAGTGAGCGCCACCTTCTGGC
ACAACCCCAGAAACCACTTCAGATGCCAGGTGCAGTTCCACGGCCTGAGCGAGGAG
GACAAGTGGCCCGAGGGCAGCCCCAAGCCCGTGACCCAGAACATCAGCGCCGAGG
CCTGGGGCAGAGCCGACTGCGGCATCACCAGCGCCAGCTACCAGCAGGGCGTGCTG
AGCGCCACCATCCTGTACGAGATCCTGCTGGGCAAGGCCACCCTGTACGCCGTGCT
GGTGAGCACCCTGGTGGTGATGGCCATGGTGAAGAGAAAGAACAGCAGAAGAAAG
AGAAGCGGCAGCGGCGCCACCAACTTCAGCCTGCTGAAGCAGGCCGGCGACGTGG
AGGAGAACCCCGGCCCCATGCTGCTGCTGGTGACCAGCCTGCTGCTGTGCGAGCTG
CCCCACCCCGCCTTCCTGCTGATCCCCGACATCCAGAACCCCGAGCCCGCCGTGTAC
CAGCTGAAGGACCCCAGAAGCCAGGACAGCACCCTGTGCCTGTTCACCGACTTCGA
CAGCCAGATCAACGTGCCCAAGACCATGGAGAGCGGCACCTTCATCACCGACAAGT
GCGTGCTGGACATGAAGGCCATGGACAGCAAGAGCAACGGCGCCATCGCCTGGAG
CAACCAGACCAGCTTCACCTGCCAGGACATCTTCAAGGAGACCAACGCCACCTACC
CCAGCAGCGACGTGCCCTGCGACGCCACCCTGACCGAGAAGAGCTTCGAGACCGAC
ATGAACCTGAACTTCCAGAACCTGCTGGTGATCGTGCTGAGAATCCTGCTGCTGAA
 
GGTGGCCGGCTTCAACCTGCTGATGACCCTGAGACTGTGGGTGGCGGTGGCAGCG
GCGGTGGTGGTTCCGGAGGCGGCGGTTCTAGAAGAGACCAGAGACTGCCCCCGAC
GCCCACAAGCCCCCGGCGGCAGCTTCAGAACCCCCATCCAGGAGGAGCAGG
CCGACGCCCACAGCACCCTGGCCAAGATCTGA
SEQ ID NO: 122, NLB4/NLB 14 STAR nucleotide sequence 
atgcttctcctggtgacaagccttctgctctgtgagttaccacacccagcattcctcctgatcccaGATGTGCAGCTGCAGGA
GAGCGGCGGCGGACTGGTTCAATCTGGAGGCAGCCTGAGGCTGAGCTGTCTGGCCA
GCGGAACCTCCGGCAACGTGAAGGCCGTGGGCTGGTACGGCTGGTATAGACAGGCC
CCTGGCAAGCAGCGGGAAGTGGTGGCCACAATCACCAGAGGCGGCATCCCCAATTA
CGCCGATAGCGTGCAAGGCAGATTCACCATCAGCCGGGACAACGCCCAGGACACA
GTGTTCCTGCAGATGAACAGCCTGAAACCTGCTGATACCGCCGTGTACTACTGCTAC
GCCAGAATCCTGACAGACGACTGGCACGACCTGTGGGGCCAGGGCACCCAGGTGA
CCGTGTCCAGCGAGGACCTGAGAAACGTGACCCCCCCCAAGGTGAGCCTGTTCGAG
CCCAGCAAGGCCGAGATCGCCAACAAGCAGAAGGCCACCCTGGTGTGCCTGGCCA
GAGGCTTCTTCCCCGACCACGTGGAGCTGAGCTGGTGGGTGAACGGCAAGGAGGTG
CACAGCGGCGTGTGCACCGACCCCCAGGCCTACAAGGAGAGCAACTACAGCTACTG
CCTGAGCAGCAGACTGAGAGTGAGCGCCACCTTCTGGCACAACCCCAGAAACCACT
TCAGATGCCAGGTGCAGTTCCACGGCCTGAGCGAGGAGGACAAGTGGCCCGAGGG
CAGCCCCAAGCCCGTGACCCAGAACATCAGCGCCGAGGCCTGGGGCAGAGCCGAC
TGCGGCATCACCAGCGCCAGCTACCAGCAGGGCGTGCTGAGCGCCACCATCCTGTA
CGAGATCCTGCTGGGCAAGGCCACCCTGTACGCCGTGCTGGTGAGCACCCTGGTGG
TGATGGCCATGGTGAAGAGAAAGAACAGCAGAAAGAGAAGCGGCAGCGGCG
CCACCAACTTCAGCCTGCTGAAGCAGGCCGGCGACGTGGAGGAGAACCCCGGCCCC
ATGCTGCTGCTGGTGACCAGCCTGCTGCTGTGCGAGCTGCCCCACCCCGCCTTCCTG
CTGATCCCCGACGTCCAGCTGCAGGAGAGCGGCGGAGGACTGGTTCAACCTGGAGG
CAGCCTGAGACTGAGCTGTGCTGCTTCTGGCTTTACCCTGGACTACTACGCCATCGG
CTGGTTCAGGCAGGCCCCTGGCAAGGAACGGGAAGGCGTGTCCTGCGTGTCCAGCA
GTGATGGCTCTACCTACTACGCCGATAGCGTGAAGGGCAGATTCACCATCAGCAGA
GACAACGCCAAGAATACCGTGTATCTGCAGATGAACAGCCTGAAACCTGAGGACAC
CGCCGTGTACTACTGCGCCGCCGACCAGTACAGCTCTACATGGACCATCCGGCTGA
CAAGATGCCACTTCGGCAGCTGGGGCCAGGGCACACAGGTGACCGTGTCTAGCGAC
ATCCAGAACCCCGAGCCCGCCGTGTACCAGCTGAAGGACCCCAGAAGCCAGGACA
GCACCCTGTGCCTGTTCACCGACTTCGACAGCCAGATCAACGTGCCCAAGACCATG
GAGAGCGGCACCTTCATCACCGACAAGTGCGTGCTGGACATGAAGGCCATGGACAG
 
CAAGAGCAACGGCGCCATCGCCTGGAGCAACCAGACCAGCTTCACCTGCCAGGACA
TCTTCAAGGAGACCAACGCCACCTACCCCAGCAGCGACGTGCCCTGCGACGCCACC
CTGACCGAGAAGAGCTTCGAGACCGACATGAACCTGAACTTCCAGAACCTGCTGGT
GATCGTGCTGAGAATCCTGCTGCTGAAGGTGGCCGGCTTCAACCTGCTGATGACCCT
GAGACTGTGGGGTGGCGGTGGCAGCGGCGGTGGTGGTTCCGGAGGCGGCGGTTCTA
GAAGAGACCAGAGACTGCCCCCCGACGCCCACAAGCCCCCCGGCGGCGGCAGCTTC
AGAACCCCCATCCAGGAGGAGCAGGCCGACGCCCACAGCACCCTGGCCAAGATCT
GA
SEQ ID NO: 123, NLB4/(myc)NLB14 STAR nucleotide sequence

atgcttctcctggtgacaagccttctgctctgtgagttaccacacccagcattcctcctgatcccaGATGTGCAGCTGCAGGA
GAGCGGCGGCGGACTGGTTCAATCTGGAGGCAGCCTGAGGCTGAGCTGTCTGGCCA
GCGGAACCTCCGGCAACGTGAAGGCCGTGGGCTGGTACGGCTGGTATAGACAGGCC
CCTGGCAAGCAGCGGGAAGTGGTGGCCACAATCACCAGAGGCGGCATCCCCAATTA
CGCCGATAGCGTGCAAGGCAGATTCACCATCAGCCGGGACAACGCCCAGGACACA
GTGTTCCTGCAGATGAACAGCCTGAAACCTGCTGATACCGCCGTGTACTACTGCTAC
GCCAGAATCCTGACAGACGACTGGCACGACCTGTGGGGCCAGGGCACCCAGGTGA
CCGTGTCCAGCGAGGACCTGAGAAACGTGACCCCCCCCAAGGTGAGCCTGTTCGAG
CCCAGCAAGGCCGAGATCGCCAACAAGCAGAAGGCCACCCTGGTGTGCCTGGCCA
GAGGCTTCTTCCCCGACCACGTGGAGCTGAGCTGGTGGGTGAACGGCAAGGAGGTG
CACAGCGGCGTGTGCACCGACCCCCAGGCCTACAAGGAGAGCAACTACAGCTACTG
CCTGAGCAGCAGACTGAGAGTGAGCGCCACCTTCTGGCACAACCCCAGAAACCACT
TCAGATGCCAGGTGCAGTTCCACGGCCTGAGCGAGGAGGACAAGTGGCCCGAGGG
CAGCCCCAAGCCCGTGACCCAGAACATCAGCGCCGAGGCCTGGGGCAGAGCCGAC
TGCGGCATCACCAGCGCCAGCTACCAGCAGGGCGTGCTGAGCGCCACCATCCTGTA
CGAGATCCTGCTGGGCAAGGCCACCCTGTACGCCGTGCTGGTGAGCACCCTGGTGG
TGATGGCCATGGTGAAGAGAAAGAACAGCAGAAGAAAGAGAAGCGGCAGCGGCG
CCACCAACTTCAGCCTGCTGAAGCAGGCCGGCGACGTGGAGGAGAACCCCGGCCCC
ATGCTGCTGCTGGTGACCAGCCTGCTGCTGTGCGAGCTGCCCCACCCCGCCTTCCTG
CTGATCCCCGAGCAGAAACTCATCTCTGAAGAGGATCTGGACGTCCAGCTGCAGGA
GAGCGGCGGAGGACTGGTTCAACCTGGAGGCAGCCTGAGACTGAGCTGTGCTGCTT
CTGGCTTTACCCTGGACTACTACGCCATCGGCTGGTTCAGGCAGGCCCCTGGCAAG
GAACGGGAAGGCGTGTCCTGCGTGTCCAGCAGTGATGGCTCTACCTACTACGCCGA
TAGCGTGAAGGGCAGATTCACCATCAGCAGAGACAACGCCAAGAATACCGTGTATC
TGCAGATGAACAGCCTGAAACCTGAGGACACCGCCGTGTACTACTGCGCCGCCGAC

CAGTACAGCTCTACATGGACCATCCGGCTGACAAGATGCCACTTCGGCAGCTGGGG
CCAGGGCACACAGGTGACCGTGTCTAGCGACATCCAGAACCCCGAGCCCGCCGTGT
ACCAGCTGAAGGACCCCAGAAGCCAGGACAGCACCCTGTGCCTGTTCACCGACTTC
GACAGCCAGATCAACGTGCCCAAGACCATGGAGAGCGGCACCTTCATCACCGACAA
GTGCGTGCTGGACATGAAGGCCATGGACAGCAAGAGCAACGGCGCCATCGCCTGG
AGCAACCAGACCAGCTTCACCTGCCAGGACATCTTCAAGGAGACCAACGCCACCTA
CCCCAGCAGCGACGTGCCCTGCGACGCCACCCTGACCGAGAAGAGCTTCGAGACCG
ACATGAACCTGAACTTCCAGAACCTGCTGGTGATCGTGCTGAGAATCCTGCTGCTG
AAGGTGGCCGGCTTCAACCTGCTGATGACCCTGAGACTGTGGGGTGGCGGTGGCAG
CGGCGGTGGTGGTTCCGGAGGCGGCGGTTCTAGAAGAGACCAGAGACTGCCCCCCG
ACGCCCACAAGCCCCCCGGCGGCGGCAGCTTCAGAACCCCCATCCAGGAGGAGCA
GGCCGACGCCCACAGCACCCTGGCCAAGATCTGA.

## Claims

1. A synthetic T cell receptor and antigen receptor, wherein
the synthetic T cell receptor and antigen receptor comprises an α chain and a β chain, wherein the α chain comprises a first target-binding region and a first constant region, and the β chain comprises a second target-binding region and a second constant region; or the α chain comprises a first target-binding region, and the β chain comprises a second target-binding region and a second constant region; or
ii) the synthetic T cell receptor and antigen receptor comprises a γ chain and a δ chain, wherein the γ chain comprises a first target-binding region and a first constant region, and the δ chain comprises a second target-binding region and a second constant region; or the γ chain comprises a first target-binding region and a first constant region, and the δ chain comprises a second target-binding region and a second constant region;
wherein the first target-binding region and/or the second target-binding region comprises one or a plurality of antigen-binding regions, the plurality of antigen-binding regions are the same or different, and the plurality of antigen-binding regions are linked directly or via a linker;
the antigen-binding region in the first target-binding region comprises an antibody or an antibody fragment specifically binding to LILRB4, and the antigen-binding region in the second target-binding region comprises an antibody or an antibody fragment specifically binding to LILRB4.

2. The synthetic T cell receptor and antigen receptor according to claim 1, wherein the antigen-binding region in the first target-binding region comprises a single chain antibody or a single domain antibody specifically binding to LILRB4; and/or the antigen-binding region in the second target-binding region comprises a single chain antibody or a single domain antibody specifically binding to LILRB4;
preferably, the single chain antibody comprises a heavy chain variable region and a light chain variable region which are linked directly or via a linker;
preferably, the plurality of antigen-binding regions in the first target-binding region and/or in the second target-binding region bind to different regions of LILRB4, e.g., different epitopes.

3. The synthetic T cell receptor and antigen receptor according to claim 1 or 2, wherein the antigen-binding region in the first target-binding region comprises one or a plurality of single domain antibodies, and/or the antigen-binding region in the second target-binding region comprises one or a plurality of single domain antibodies;
preferably, the plurality of single domain antibodies comprised in the antigen-binding region in the first target-binding region are the same or different;
preferably, the plurality of single domain antibodies comprised in the antigen-binding region in the second target-binding region are the same or different;
further preferably, the plurality of single domain antibodies are linked directly or via a linker.

4. The synthetic T cell receptor and antigen receptor according to claim 3, wherein the single domain antibody comprises a heavy chain variable region, and the heavy chain variable region comprises CDR1-3, wherein
i) the CDR1 comprises the amino acid sequence set forth in SEQ ID NO: 33, the CDR2 comprises the amino acid sequence set forth in SEQ ID NO: 34, and the CDR3 comprises the amino acid sequence set forth in SEQ ID NO: 35;
or,
ii) the CDR1 comprises the amino acid sequence set forth in SEQ ID NO: 36, the CDR2 comprises the amino acid sequence set forth in SEQ ID NO: 37, and the CDR3 comprises the amino acid sequence set forth in SEQ ID NO: 38.

5. The synthetic T cell receptor and antigen receptor according to claim 3, wherein the single domain antibody comprises the amino acid sequence set forth in SEQ ID NO: 28 or 29.

6. The synthetic T cell receptor and antigen receptor according to claim 1, wherein
i) the α chain and/or the β chain is linked at its C-terminus to at least one functional domain, the at least one functional domain being linked, directly or via a linker, to the C-terminus of the α chain and/or the β chain;
or,
ii) the γ chain and/or the δ chain is linked at its C-terminus to at least one functional domain, the at least one functional domain being linked, directly or via a linker, to the C-terminus of the γ chain and/or the δ chain.

7. The synthetic T cell receptor and antigen receptor according to claim 1, wherein
i) an intracellular region of the α chain and/or the β chain in the synthetic T cell receptor and antigen receptor is deleted;
or,
ii) an intracellular region of the γ chain and/or the δ chain in the synthetic T cell receptor and antigen receptor is deleted.

8. The synthetic T cell receptor and antigen receptor according to claim 7, wherein
i) the α chain and/or the β chain is linked at its C-terminus to at least one functional domain, the at least one functional domain being linked, directly or via a linker, to the C-terminus of the α chain and/or the β chain;
or,
ii) the γ chain and/or the δ chain is linked at its C-terminus to at least one functional domain, the at least one functional domain being linked, directly or via a linker, to the C-terminus of the γ chain and/or the δ chain.

9. The synthetic T cell receptor and antigen receptor according to any one of claims 6-8, wherein
i) the C-terminus of the α chain in the synthetic T cell receptor and antigen receptor is linked to 1, 2, 3, 4, 5, 6, 7, 8, 9, 10, or more functional domains, and/or the C-terminus of the β chain in the synthetic T cell receptor and antigen receptor is linked to 1, 2, 3, 4, 5, 6, 7, 8, 9, 10, or more functional domains;
or,
ii) the C-terminus of the γ chain in the synthetic T cell receptor and antigen receptor is linked to 1, 2, 3, 4, 5, 6, 7, 8, 9, 10, or more functional domains, and/or the C-terminus of the δ chain in the synthetic T cell receptor and antigen receptor is linked to 1, 2, 3, 4, 5, 6, 7, 8, 9, 10, or more functional domains;
wherein the functional domains are the same or different.

10. The synthetic T cell receptor and antigen receptor according to any one of claims 6-8, wherein the functional domain is a co-stimulatory molecule or a fragment thereof, a co-inhibitory molecule or a fragment thereof, a cytokine receptor or a fragment thereof, or an intracellular protein or a fragment thereof;
preferably, the co-stimulatory molecule is selected from CD40, OX40, ICOS, CD28, 4-1BB, or CD27;
preferably, the co-inhibitory molecule is selected from TIM3, PD1, CTLA4, or LAG3; preferably, the cytokine receptor is selected from an interleukin receptor, an interferon receptor, a tumor necrosis factor superfamily receptor, a colony-stimulating factor receptor, a chemokine receptor, a growth factor receptor, or other membrane proteins;
preferably, the intracellular protein is a domain of a T-cell regulatory factor, e.g., a domain of NIK.

11. The synthetic T cell receptor and antigen receptor according to any one of claims 1-10, wherein the linker is selected from a rigid linker, a flexible linker, a cleavable linker, or a non-essential amino acid;
preferably, the amino acid sequence of the rigid linker is selected from one or a combination of two or more of SEQ ID NOs: 49-59;
preferably, the flexible linker is selected from a peptide fragment rich in glycine and/or serine;
preferably, the flexible linker is selected from one or a combination of two or more of SEQ ID NOs: 60-112;
preferably, the cleavable linker is selected from one or a combination of two or more of SEQ ID NOs: 113-117.

12. The synthetic T cell receptor and antigen receptor according to any one of claims 1-11, wherein the first constant region is a TCRα chain constant region or a TCRγ chain constant region, preferably a modified TCRα chain constant region or a modified TCRγ chain constant region;
preferably, the TCRα chain constant region is selected from a human TCRα chain constant region or a murine (preferably mouse) TCRα chain constant region;
preferably, the TCRγ chain constant region is selected from a human TCRγ chain constant region or a murine (preferably mouse) TCRγ chain constant region.

13. The synthetic T cell receptor and antigen receptor according to claim 12, wherein the modified TCRα chain constant region is derived from a murine (preferably mouse) TCRα chain constant region comprising one or more modifications at position 6, 13, 15-18, 48, 112, 114, or 115 relative to a wild-type murine (preferably mouse) TCRα chain constant region, the modification being a mutation or a deletion; or
the modified TCRα chain constant region is derived from a murine (preferably mouse) TCRα chain constant region comprising one or more modifications at position 13, 36, 47, 53, 58, 78, 98, or 122 relative to the wild-type murine (preferably mouse) TCRα chain constant region, the modification being a mutation or a deletion.

14. The synthetic T cell receptor and antigen receptor according to claim 12, wherein the modified TCRα chain constant region is derived from a murine (preferably mouse) TCRα chain constant region comprising a mutation of the amino acid at position 48, e.g., threonine T, to cysteine C, relative to a wild-type murine (preferably mouse) TCRα chain constant region;
the modified TCRα chain constant region is derived from a murine (preferably mouse) TCRα chain constant region comprising a mutation of the amino acid at position 112, e.g., serine S, to leucine L, a mutation of the amino acid at position 114, e.g., methionine M, to isoleucine I, and/or a mutation of the amino acid at position 115, e.g., glycine G, to valine V, relative to the wild-type murine (preferably mouse) TCRα chain constant region;
the modified TCRα chain constant region is derived from a murine (preferably mouse) TCRα chain constant region comprising a substitution of the amino acid at position 6, e.g., E, with D, a substitution of K at position 13 with R, and deletions of the amino acids at positions 15-18, relative to the wild-type murine (preferably mouse) TCRα chain constant region;
the modified TCRα chain constant region is derived from a murine (preferably mouse) TCRα chain constant region comprising a substitution of the amino acid K at position 122 with R relative to the wild-type murine (preferably mouse) TCRα chain constant region;
the modified TCRα chain constant region is derived from a murine (preferably mouse) TCRα chain constant region comprising a mutation of the amino acid at position 48, e.g., threonine T, to cysteine C, a mutation of the amino acid at position 112, e.g., serine S, to leucine L, a mutation of the amino acid at position 114, e.g., methionine M, to isoleucine I, and a mutation of the amino acid at position 115, e.g., glycine G, to valine V, relative to the wild-type murine (preferably mouse) TCRα chain constant region;
the modified TCRα chain constant region is derived from a murine (preferably mouse) TCRα chain constant region comprising a mutation of the amino acid at position 48, e.g., threonine T, to cysteine C, and a substitution of the amino acid K at position 122 with R, relative to the wild-type murine (preferably mouse) TCRα chain constant region;
the modified TCRα chain constant region is derived from a murine (preferably mouse) TCRα chain constant region comprising a substitution of the amino acid at position 6, e.g., E, with D, a substitution of K at position 13 with R, deletions of the amino acids at positions 15-18, and a mutation of the amino acid at position 48, e.g., threonine T, to cysteine C, relative to the wild-type murine (preferably mouse) TCRα chain constant region;
the modified TCRα chain constant region is derived from a murine (preferably mouse) TCRα chain constant region comprising a mutation of the amino acid at position 48, e.g., threonine T, to cysteine C, a mutation of the amino acid at position 112, e.g., serine S, to leucine L, a mutation of the amino acid at position 114, e.g., methionine M, to isoleucine I, a mutation of the amino acid at position 115, e.g., glycine G, to valine V, and a substitution of the amino acid K at position 122 with R, relative to the wild-type murine (preferably mouse) TCRα chain constant region;
the modified TCRα chain constant region is derived from a murine (preferably mouse) TCRα chain constant region comprising a substitution of the amino acid at position 6, e.g., E, with D, a substitution of K at position 13 with R, deletions of the amino acids at positions 15-18, a mutation of the amino acid at position 48, e.g., threonine T, to cysteine C, a mutation of the amino acid at position 112, e.g., serine S, to leucine L, a mutation of the amino acid at position 114, e.g., methionine M, to isoleucine I, a mutation of the amino acid at position 115, e.g., glycine G, to valine V, and a substitution of the amino acid K at position 122 with R, relative to the wild-type murine (preferably mouse) TCRα chain constant region;
the modified TCRα chain constant region is derived from a murine (preferably mouse) TCRα chain constant region comprising a substitution of the amino acid at position 6, e.g., E, with D, a substitution of K at position 13 with R, deletions of the amino acids at positions 15-18, a mutation of the amino acid at position 48, e.g., threonine T, to cysteine C, a mutation of the amino acid at position 112, e.g., serine S, to leucine L, a mutation of the amino acid at position 114, e.g., methionine M, to isoleucine I, and a mutation of the amino acid at position 115, e.g., glycine G, to valine V, relative to the wild-type murine (preferably mouse) TCRα chain constant region;
the modified TCRα chain constant region is derived from a murine (preferably mouse) TCRα chain constant region comprising a substitution of the amino acid at position 6, e.g., E, with D, a substitution of K at position 13 with R, deletions of the amino acids at positions 15-18, a mutation of the amino acid at position 48, e.g., threonine T, to cysteine C, and a substitution of the amino acid K at position 122 with R, relative to the wild-type murine (preferably mouse) TCRα chain constant region;
the modified TCRα chain constant region is derived from a murine (preferably mouse) TCRα chain constant region comprising a substitution of the amino acid at position 6, e.g., E, with D, a substitution of K at position 13 with R, deletions of the amino acids at positions 15-18, a mutation of the amino acid at position 112, e.g., serine S, to leucine L, a mutation of the amino acid at position 114, e.g., methionine M, to isoleucine I, and a mutation of the amino acid at position 115, e.g., glycine G, to valine V, relative to the wild-type murine (preferably mouse) TCRα chain constant region;
the modified TCRα chain constant region is derived from a murine (preferably mouse) TCRα chain constant region comprising a substitution of the amino acid at position 6, e.g., E, with D, a substitution of K at position 13 with R, deletions of the amino acids at positions 15-18, and a substitution of the amino acid K at position 122 with R, relative to the wild-type murine (preferably mouse) TCRα chain constant region; or
the modified TCRα chain constant region is derived from a murine (preferably mouse) TCRα chain constant region comprising a substitution of the amino acid at position 6, e.g., E, with D, a substitution of K at position 13 with R, deletions of the amino acids at positions 15-18, a mutation of the amino acid at position 112, e.g., serine S, to leucine L, a mutation of the amino acid at position 114, e.g., methionine M, to isoleucine I, a mutation of the amino acid at position 115, e.g., glycine G, to valine V, and a substitution of the amino acid K at position 122 with R, relative to the wild-type murine (preferably mouse) TCRα chain constant region.

15. The synthetic T cell receptor and antigen receptor according to claim 12, wherein the first constant region comprises the amino acid sequence set forth in any one of SEQ ID NOs: 1, 3, 5, 7, 8, 16, 30, 31, or 43.

16. The synthetic T cell receptor and antigen receptor according to any one of claims 1-15, wherein the second constant region is a TCRβ chain constant region or a TCRδ chain constant region, preferably a modified TCRβ chain constant region or a modified TCRδ chain constant region;
preferably, the TCRβ chain constant region is selected from a human TCRβ chain constant region or a murine (preferably mouse) TCRβ chain constant region;
preferably, the TCRδ chain constant region is selected from a human TCRδ chain constant region or a murine (preferably mouse) TCRδ chain constant region.

17. The synthetic T cell receptor and antigen receptor according to claim 16, wherein the modified TCRβ chain constant region is derived from a murine (preferably mouse) TCRβ chain constant region comprising one or more modifications at position 3, 6, 9, 11, 12, 17, 21-25, 56, 150, 168, or 170 relative to a wild-type murine (preferably mouse) TCRβ chain constant region, the modification being a mutation or a deletion; or
the modified TCRβ chain constant region is derived from a murine (preferably mouse) TCRβ chain constant region comprising one or more modifications at position 9, 17, 23, 25, 49, 63, 103, 110, 150, 168, or 170 relative to the wild-type murine (preferably mouse) TCRβ chain constant region, the modification being a mutation or a deletion.

18. The synthetic T cell receptor and antigen receptor according to claim 16, wherein the modified TCRβ chain constant region is derived from a murine (preferably mouse) TCRβ chain constant region comprising a mutation of the amino acid at position 56, e.g., serine S, to cysteine C, relative to a wild-type murine (preferably mouse) TCRβ chain constant region;
the modified TCRβ chain constant region is derived from a murine (preferably mouse) TCRβ chain constant region comprising a substitution of lysine at position 150, 168, or 170 with arginine;
the modified TCRβ chain constant region is derived from a murine (preferably mouse) TCRβ chain constant region comprising a substitution of the amino acid at position 3, e.g., R, with K, a substitution of the amino acid at position 6, e.g., T, with F, a substitution of K at position 9 with E, a substitution of S at position 11 with A, a substitution of L at position 12 with V, and deletions of the amino acids at positions 17 and 21-25, relative to the wild-type murine (preferably mouse) TCRβ chain constant region;
the modified TCRβ chain constant region is derived from a murine (preferably mouse) TCRβ chain constant region comprising a mutation of the amino acid at position 56, e.g., serine S, to cysteine C, and a substitution of lysine at position 150, 168, or 170 with arginine, relative to the wild-type murine (preferably mouse) TCRβ chain constant region;
the modified TCRβ chain constant region is derived from a murine (preferably mouse) TCRβ chain constant region comprising a substitution of the amino acid at position 3, e.g., R, with K, a substitution of the amino acid at position 6, e.g., T, with F, a substitution of K at position 9 with E, a substitution of S at position 11 with A, a substitution of L at position 12 with V, deletions of the amino acids at positions 17 and 21-25, and a mutation of the amino acid at position 56, e.g., serine S, to cysteine C, relative to the wild-type murine (preferably mouse) TCRβ chain constant region;
the modified TCRβ chain constant region is derived from a murine (preferably mouse) TCRβ chain constant region comprising a substitution of the amino acid at position 3, e.g., R, with K, a substitution of the amino acid at position 6, e.g., T, with F, a substitution of K at position 9 with E, a substitution of S at position 11 with A, a substitution of L at position 12 with V, deletions of the amino acids at positions 17 and 21-25, a mutation of the amino acid at position 56, e.g., serine S, to cysteine C, and a substitution of lysine at position 150, 168, or 170 with arginine, relative to the wild-type murine (preferably mouse) TCRβ chain constant region;
the modified TCRβ chain constant region is derived from a murine (preferably mouse) TCRβ chain constant region comprising a substitution of the amino acid at position 3, e.g., R, with K, a substitution of the amino acid at position 6, e.g., T, with F, a substitution of K at position 9 with E, a substitution of S at position 11 with A, a substitution of L at position 12 with V, deletions of the amino acids at positions 17 and 21-25, and a substitution of lysine at position 150, 168, or 170 with arginine, relative to the wild-type murine (preferably mouse) TCRβ chain constant region.

19. The synthetic T cell receptor and antigen receptor according to claim 16, wherein the second constant region comprises the amino acid sequence set forth in any one of SEQ ID NOs: 2, 4, 6, 9, 17, 32, or 44.

20. The synthetic T cell receptor and antigen receptor according to claim 1 or 2, wherein the first target-binding region is linked, directly or via a linker, to the first constant region, and/or the second target-binding region is linked, directly or via a linker, to the second constant region.

21. The synthetic T cell receptor and antigen receptor according to claim 20, wherein the linker is selected from a rigid linker, a flexible linker, a cleavable linker, or a non-essential amino acid; preferably, the amino acid sequence of the rigid linker is selected from one or a combination of two or more of SEQ ID NOs: 49-59;
preferably, the flexible linker is selected from a peptide fragment rich in glycine and/or serine; preferably, the flexible linker is selected from one or a combination of two or more of SEQ ID NOs: 60-112;
preferably, the cleavable linker is selected from one or a combination of two or more of SEQ ID NOs: 113-117.

22. A synthetic T cell receptor and antigen receptor complex, wherein the complex comprises the synthetic T cell receptor and antigen receptor according to any one of claims 1-21, as well as CD3ε, CD3γ, CD3δ, and CD3ζ.

23. The synthetic T cell receptor and antigen receptor complex according to claim 22, wherein the CD3ε, CD3γ, CD3δ, and/or CD3ζ is of human origin;
preferably, the CD3ε comprises the amino acid sequence set forth in SEQ ID NO: 20;
preferably, the CD3γ comprises the amino acid sequence set forth in SEQ ID NO: 18;
preferably, the CD3δ comprises the amino acid sequence set forth in SEQ ID NO: 19;
preferably, the CD3ζ comprises the amino acid sequence set forth in SEQ ID NO: 21.

24. An antibody or an antigen-binding fragment, comprising a heavy chain variable region, wherein the heavy chain variable region comprises CDR1-3, wherein
i) the CDR1 comprises the amino acid sequence set forth in SEQ ID NO: 33, the CDR2 comprises the amino acid sequence set forth in SEQ ID NO: 34, and the CDR3 comprises the amino acid sequence set forth in SEQ ID NO: 35;
or,
ii) the CDR1 comprises the amino acid sequence set forth in SEQ ID NO: 36, the CDR2 comprises the amino acid sequence set forth in SEQ ID NO: 37, and the CDR3 comprises the amino acid sequence set forth in SEQ ID NO: 38.

25. The antibody or the antigen-binding fragment according to claim 24, wherein the antibody or the antigen-binding fragment is a single chain antibody or a single domain antibody.

26. The antibody or the antigen-binding fragment according to claim 24, wherein the antibody or the antigen-binding fragment comprises the amino acid sequence set forth in SEQ ID NO: 28 or 29.

27. An antigen receptor, comprising a transmembrane region, an intracellular region, and one or more, the same or different, extracellular binding domains, wherein the extracellular binding domain is an extracellular antigen-binding domain;
the extracellular antigen-binding domain comprises CDR1-3, wherein
i) the CDR1 comprises the amino acid sequence set forth in SEQ ID NO: 33, the CDR2 comprises the amino acid sequence set forth in SEQ ID NO: 34, and the CDR3 comprises the amino acid sequence set forth in SEQ ID NO: 35;
or,
ii) the CDR1 comprises the amino acid sequence set forth in SEQ ID NO: 36, the CDR2 comprises the amino acid sequence set forth in SEQ ID NO: 37, and the CDR3 comprises the amino acid sequence set forth in SEQ ID NO: 38.

28. The antigen receptor according to claim 27, wherein the extracellular antigen-binding domain comprises the antibody or the antigen-binding fragment according to any one of claims 24-26.

29. The antigen receptor according to claim 27, wherein the antigen receptor is a STAR, a TCR, or a CAR;
preferably, the transmembrane region is derived from human CD8;
preferably, the intracellular region is derived from 4-1BB, CD28, or CD3ζ.

30. The antigen receptor according to claim 27, wherein the transmembrane region is linked, directly or via a linker, to one or more extracellular antigen-binding domains.

31. The antigen receptor according to claim 30, wherein the linker is selected from a rigid linker, a flexible linker, a cleavable linker, and a non-essential amino acid;
preferably, the amino acid sequence of the rigid linker is selected from one or a combination of two or more of SEQ ID NOs: 49-59;
preferably, the flexible linker is selected from a peptide fragment rich in glycine and/or serine;
preferably, the flexible linker is selected from one or a combination of two or more of SEQ ID NOs: 60-112;
preferably, the cleavable linker is selected from one or a combination of two or more of SEQ ID NOs: 113-117.

32. A nucleic acid encoding the synthetic T cell receptor and antigen receptor according to any one of claims 1-21, the synthetic T cell receptor and antigen receptor complex according to any one of claims 22-23, the antibody or the antigen-binding fragment according to any one of claims 24-26, or the antigen receptor according to any one of claims 27-31.

33. A vector, comprising the nucleic acid according to claim 32.

34. A host cell, comprising the nucleic acid according to claim 32 or the vector according to claim 33.

35. An immune cell expressing the synthetic T cell receptor and antigen receptor according to any one of claims 1-21, the synthetic T cell receptor and antigen receptor complex according to any one of claims 22-23, the antibody or the antigen-binding fragment according to any one of claims 24-26, or the antigen receptor according to any one of claims 27-31.

36. The immune cell according to claim 35, wherein the immune cell comprises one or more of the nucleic acids according to claim 32.

37. The immune cell according to claim 35 or 36, wherein the immune cell is selected from a T cell, a Treg cell, a macrophage, an NK cell, an NKT cell, a peripheral blood mononuclear cell, a TIL cell, or a dendritic cell (DC).

38. The immune cell according to claim 35 or 36, wherein the immune cell is isolated from a T cell derived from a subject.

39. A preparation method for an immune cell, comprising transfecting an immune cell with the nucleic acid sequence according to claim 32 for expression.

40. A preparation method for a recombinant T cell, comprising the following steps:
1) obtaining the nucleic acid according to claim 32 from a positive T cell clone;
2) isolating and culturing a primary T cell; and
3) delivering the nucleic acid obtained in step 1) to the primary T cell in step 2) to obtain a recombinant T cell expressing the synthetic T cell receptor and antigen receptor according to any one of claims 1-21.

41. A preparation method for a synthetic T cell receptor and antigen receptor, comprising the following steps:
(1) obtaining the nucleic acid according to claim 32 from a positive T cell clone;
(2) ligating the nucleic acid obtained in step (1) to a vector backbone to obtain an expression vector;
(3) transforming the expression vector obtained in step (2) into a host cell, and subsequently, inducing the expression thereof; and
(4) obtaining a synthetic T cell receptor and antigen receptor.

42. Use of the synthetic T cell receptor and antigen receptor according to any one of claims 1-21, the synthetic T cell receptor and antigen receptor complex according to any one of claims 22-23, the antibody or the antigen-binding fragment according to any one of claims 24-26, the antigen receptor according to any one of claims 27-31, the nucleic acid according to claim 32, or the immune cell according to any one of claims 35-38 in preparing a product for the diagnosis or treatment of a tumor.

43. The use according to claim 42, wherein the tumor comprises lymphoma, non-small cell lung cancer, leukemia, ovarian cancer, nasopharyngeal cancer, breast cancer, endometrial cancer, colon cancer, rectal cancer, stomach cancer, bladder cancer, lung cancer, bronchial cancer, bone cancer, prostate cancer, pancreatic cancer, liver and bile duct cancer, esophageal cancer, kidney cancer, thyroid cancer, head and neck cancer, testicular cancer, glioblastoma, astrocytoma, melanoma, myelodysplastic syndrome, and sarcoma;
preferably, the leukemia is selected from acute lymphocytic (lymphoblastic) leukemia, acute myeloid leukemia, myeloid leukemia, chronic lymphocytic leukemia, multiple myeloma, plasma cell leukemia, and chronic myeloid leukemia;
preferably, the lymphoma is selected from Hodgkin's lymphoma and non-Hodgkin's lymphoma, comprising B-cell lymphoma, diffuse large B-cell lymphoma, follicular lymphoma, mantle cell lymphoma, marginal zone B-cell lymphoma, T-cell lymphoma, and Waldenström macroglobulinemia;
preferably, the sarcoma is selected from osteosarcoma, Ewing's sarcoma, leiomyosarcoma, synovial sarcoma, soft tissue sarcoma, angiosarcoma, liposarcoma, fibrosarcoma, rhabdomyosarcoma, and chondrosarcoma;
preferably, the acute myeloid leukemia is M4 or M5 acute myeloid leukemia;
preferably, the chronic myeloid leukemia is chronic myelomonocytic leukemia.

44. A pharmaceutical composition, comprising the synthetic T cell receptor and antigen receptor according to any one of claims 1-21, the synthetic T cell receptor and antigen receptor complex according to any one of claims 22-23, the antibody or the antigen-binding fragment according to any one of claims 24-26, the antigen receptor according to any one of claims 27-31, the nucleic acid according to claim 32, or the immune cell according to any one of claims 35-38.

45. A kit, comprising the synthetic T cell receptor and antigen receptor according to any one of claims 1-21, the synthetic T cell receptor and antigen receptor complex according to any one of claims 22-23, the antibody or the antigen-binding fragment according to any one of claims 24-26, the antigen receptor according to any one of claims 27-31, the nucleic acid according to claim 32, or the immune cell according to any one of claims 35-38.

46. A method for treating a tumor, comprising administering to a subject an effective amount of the antibody or the antigen-binding fragment according to any one of claims 24-26, the immune cell according to any one of claims 35-38, or the pharmaceutical composition according to claim 44.
